# FASCICULE DE BREVET EUROPEEN

(11) **EP 2 807 159 B1**
(45) Date de publication et mention de la délivrance du brevet: **23.11.2016**
(21) Numéro de dépôt: 13704189.3
(22) Date de dépôt: 23.01.2013
(51) Int. Cl.: C07D 471/04, C07D 487/04, C07D 491/052, A61K 31/4353, A61K 31/436, A61K 31/407, A61P 35/00, A61P 37/00

(54) **NOUVEAUX DERIVES D'INDOLIZINE, LEUR PROCEDE DE PREPARATION ET LES COMPOSITIONS PHARMACEUTIQUES QUI LES CONTIENNENT POUR LE TRAITEMENT DU CANCER**
NEUE INDOLIZINDERIVATE, HERSTELLUNGSVERFAHREN DAFÜR UND PHARMAZEUTISCHE ZUSAMMENSETZUNGEN ZUR KREBSBEHANDLUNG
NEW INDOLIZINE DERIVATIVES, METHOD FOR PREPARING SAME AND PHARMACEUTICAL COMPOSITIONS CONTAINING THEM FOR THE TREATMENT OF CANCER

(30) Priorité: 24.01.2012 FR 1200193
(43) Date de publication de la demande: 03.12.2014
(73) Titulaire: Les Laboratoires Servier, 92284 Suresnes Cedex (FR); Vernalis (R&D) Ltd., Berkshire RG41 5RD (GB)
(72) Inventeur: LE DIGUARHER, Thierry, F-45550 saint denis l' Hotel (FR); CASARA, Patrick, F-67100 Strasbourg (FR); STARCK, Jérôme-Benoît, F-92500 Rueil-malmaison (FR); HENLIN, Jean-Michel, F-92150 Suresnes (FR); DAVIDSON, James Edward Paul, Cambridge Cambridgeshire CB22 5DJ (GB); MURRAY, James Brooke, Linton Cambridgeshire CB21 4YL (GB); GRAHAM, Christopher John, Newmarket Suffolk CB8 0LE (GB); CHEN, I-Jen, Cambridge Cambridgeshire CB1 3NY (GB); GENESTE, Olivier, F-92500 Rueil Malmaison (FR); HICKMAN, John, F-75017 Paris (FR); DEPIL, Stéphane, F-92130 Issy Les Moulineaux (FR); LE TIRAN, Arnaud, F-78290 Croissy Sur Seine (FR); NYERGES, Miklos, H-2016 Leanyfalu (HU); DE NANTEUIL, Guillaume, F-92150 Suresnes (FR)
(86) Numéro de dépôt international: PCT/FR2013/050136
(87) Numéro de publication internationale: WO 2013/110890

(56) Documents cités:
- PORTER J ET AL: "Tetrahydroisoquinoline amide substituted phenyl pyrazoles as selective Bcl-2 inhibitors", BIOORGANIC & MEDICINAL CHEMISTRY LETTERS, PERGAMON, ELSEVIER SCIENCE, GB, vol. 19, no. 1, 1 janvier 2009 (2009-01-01), pages 230-233, XP025816913, ISSN: 0960-894X, DOI: 10.1016/J.BMCL.2008.10.113 [extrait le 2008-10-31]
- PORTER J ET AL: "Atropisomeric small molecule Bcl-2 ligands: Determination of bioactive conformation", BIOORGANIC & MEDICINAL CHEMISTRY LETTERS, PERGAMON, ELSEVIER SCIENCE, GB, vol. 19, no. 6, 15 mars 2009 (2009-03-15), pages 1767-1772, XP026005876, ISSN: 0960-894X, DOI: 10.1016/J.BMCL.2009.01.071 [extrait le 2009-01-27]

## Description

La présente invention concerne de nouveaux dérivés d'indolizine, leur procédé de préparation et les compositions pharmaceutiques qui les contiennent.

Les composés de la présente invention sont nouveaux et présentent des caractéristiques pharmacologiques très intéressantes dans le domaine de l'apoptose et de la cancérologie.

L'apoptose, ou mort cellulaire programmée, est un processus physiologique crucial pour le développement embryonnaire et le maintien de l'homéostasie tissulaire.
La mort cellulaire de type apoptotique fait intervenir des changements morphologiques, tels que la condensation du noyau, la fragmentation de l'ADN, ainsi que des phénomènes biochimiques, tels que l'activation des caspases qui vont dégrader des composants structuraux clés de la cellule pour induire son désassemblage et sa mort. La régulation du processus d'apoptose est complexe et implique l'activation ou la répression de plusieurs voies de signalisation intracellulaire (Cory S. et al., Nature Review Cancer, 2002, 2, 647-656).
Des dérégulations de l'apoptose sont impliquées dans certaines pathologies. Une apoptose accrue est liée aux maladies neurodégénératives telles que la maladie de Parkinson, la maladie d'Alzheimer et l'ischémie. Inversement, des déficiences dans l'exécution de l'apoptose jouent un rôle important dans le développement des cancers et leur chimiorésistance, des maladies auto-immunes, des maladies inflammatoires et des infections virales. Ainsi, l'échappement à l'apoptose fait partie des signatures phénotypiques du cancer (Hanahan D. et al., Cell 2000, 100, 57-70).

Les protéines anti-apoptotiques de la famille Bcl-2 sont associées à de nombreuses pathologies. L'implication des protéines de la famille Bcl-2 est décrite dans de nombreux types de cancer, tel que le cancer colorectal, le cancer du sein, le cancer du poumon à petites cellules, le cancer du poumon non à petites cellules, le cancer de la vessie, le cancer de l'ovaire, le cancer de la prostate, la leucémie lymphoïde chronique, le lymphome folliculaire, le myélome, le cancer de la prostate... La surexpression des protéines anti-apoptotiques de la famille Bcl-2 est impliquée dans la tumorogenèse, dans la résistance à la chimiothérapie et dans le pronostique clinique des patients atteints de cancer. Il existe donc un besoin thérapeutique de composés inhibant l'activité anti-apoptotique des protéines de la famille Bcl-2.

J. Porter et al. décrivent des dérivés de tetrahydroisoquinoline en tant qu'inhibiteurs de Bcl-2 (Bio. Med. Chem. Lett. 19 (2009) 230-233 and 1767-1772). Les composés de la présente invention outre leur nouveauté, présentent des propriétés pro-apoptotiques permettant de les utiliser dans les pathologies impliquant un défaut d'apoptose, comme par exemple dans le traitement du cancer, des maladies immunitaires et auto-immunes.

La présente invention concerne plus particulièrement les composés de formule (I) : 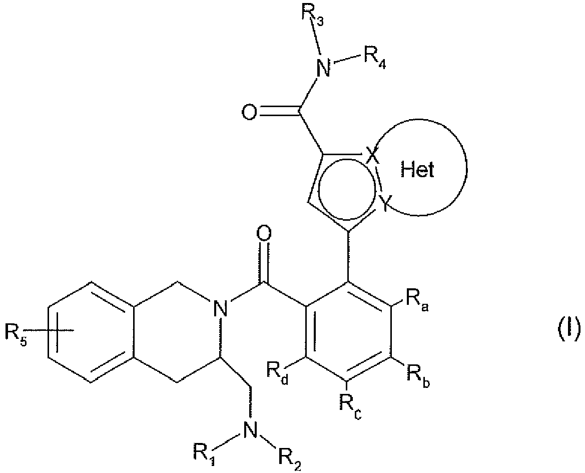 dans laquelle :
◆ X et Y représentent un atome de carbone ou un atome d'azote, étant entendu qu'ils ne peuvent représenter simultanément deux atomes de carbone ou deux atomes d'azote,
◆ la partie Het du groupe 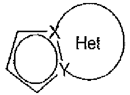 représente un cycle éventuellement substitué, aromatique ou non, constitué de 5, 6 ou 7 chaînons, et pouvant contenir, en plus de l'azote représenté par X ou par Y, un à 3 hétéroatomes choisis indépendamment parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié,
◆ R₁ et R₂ représentent indépendamment l'un de l'autre un atome d'hydrogène ou un groupement alkyle (C₁-C₆) linéaire ou ramifié, ou bien R₁ et R₂ forment avec l'atome d'azote qui les porte, un hétérocycloalkyle constitué de 4 à 7 chaînons et pouvant contenir, en plus de l'atome d'azote, un autre hétéroatome choisi parmi oxygène, soufre, SO₂ et NR dans lequel R représente un atome d'hydrogène, un groupement alkyle (C₁-C₆) linéaire ou ramifié, un groupement (C₁-C₆)alkylsulfonyl, un groupement polyhalogénoalkyle (C₁-C₆) linéaire ou ramifié, ou un groupement -C(O)-O-Alk dans lequel Alk est un groupement alkyle (C₁-C₆) linéaire ou ramifié,
◆ R₃ représente un groupement alkyle (C₁-C₆) linéaire ou ramifié, alkényle (C₂-C₆), alkynyle (C₂-C₆), cycloalkyle, cycloalkyl(C₄-C₁₀)alkyle (C₁-C₆) linéaire ou ramifié, aryle ou hétéroaryle,
◆ R₄ représente un groupement aryle, hétéroaryle, cycloalkyle ou alkyle (C₁-C₆) linéaire ou ramifié,
◆ R₅ représente un atome d'hydrogène ou d'halogène,
◆ Rₐ, R_{b}, R_{c} et R_{d} représentent indépendamment les uns des autres un atome d'hydrogène, un atome d'halogène, un groupement alkyle (C₁-C₆) linéaire ou ramifié, un groupement 1alkoxy (C₁-C₆) linéaire ou ramifié, un groupe hydroxy, un groupement polyhalogénoalkyle (C₁-C₆) linéaire ou ramifié, ou un groupe trifluorométhoxy, ou bien les substituants de l'un des couples (Rₐ,R_{b}), (R_{b},R_{c}) ou (R_{c},R_{d}) forment ensemble avec les atomes de carbone qui les portent un cycle constitué de 5 à 7 chaînons, pouvant contenir de un à 3 hétéroatomes choisis parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié, étant aussi entendu qu'un ou plusieurs atomes de carbone du cycle précédemment défini peut(vent) être deutéré(s),
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 3 hétéroatomes choisis parmi oxygène, soufre ou azote,
- par "cycloalkyle", on entend tout groupement carbocyclique non aromatique, mono ou bi-cyclique, contenant 4 à 10 chaînons
les groupements alkyle, aryle, hétéroaryle, cycloalkyle et hétérocycloalkyle ainsi définis pouvant être substitués par 1 à 3 groupements choisis parmi alkyle (C₁-C₆) linéaire ou ramifié éventuellement substitué, spiro (C₃-C₆), alkoxy (C₁-C₆) linéaire ou ramifié, (C₁-C₆)alkyl-S-, hydroxy, oxo (ou *N*-oxide le cas échéant), nitro, cyano, -COOR', NR'R", polyhalogénoalkyle (C₁-C₆) linéaire ou ramifié, trifluorométhoxy, (C₁-C₆)alkylsulfonyl ou halogène, étant entendu que R' et R" représentent, indépendamment l'un de l'autre, un atome d'hydrogène ou un groupement alkyle (C₁-C₆) linéaire ou ramifié,
la partie Het du groupe 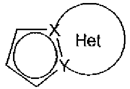 défini ci-dessus pouvant être substituée par un groupement choisi parmi alkyle (C₁-C₆) linéaire ou ramifié, hydroxy, NR₁'R₁", ou halogène, étant entendu que R₁' et R₁" ont les mêmes définitions que les groupes R' et R" mentionnés précédemment,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

De manière avantageuse, le groupe : 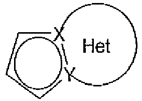 représente l'un des groupements suivants : 5,6,7,8-tétrahydroindolizine éventuellement substitué par un hydroxy, indolizine, 1,2,3,4-tétrahydropyrrolo[1,2-*a*]pyrazine, 3,4-dihydropyrrolo[1,2-*a*]pyrazine-2(1*H*)-carboxylate de *tert*-butyle, 3,4-dihydro-1*H-*pyrrolo[2,1-*c*][1,4]oxazine, 2,3-dihydro-1*H-*pyrrolizine éventuellement substitué par un hydroxy, 6,7,8,9-tétrahydro-5*H-*pyrrolo[1,2-*a*]azépine ou pyrrolo[1,2-*a*]pyrazine.

Dans les composés préférés de l'invention, R₁ et R₂ représentent chacun un groupement alkyle éventuellement substitué par un méthoxy, ou bien R₁ et R₂ forment avec l'atome d'azote qui les porte un hétérocycloalkyle choisi parmi les groupes suivants : morpholine éventuellement substituée par un ou plusieurs alkyle(s) (C₁-C₆) linéaire(s) ou ramifiée), oxidomorpholine, thiomorpholine 1,1-dioxide, 1,4-oxazépane, 3-méthoxypyrrolidine, 3,3-difluoropyrrolidine, 3-méthoxyazétidine, 3-fluoroazétidine, oxopipérazine ou pipérazine, les deux derniers groupes étant substitués par un groupement alkyle (C₁-C₆) linéaire ou ramifié, polyhalogénoalkyle (C₁-C₆) linéaire ou ramifié ou méthylsulfonyl.

De manière préférentielle, Rₐ et R_{d} représentent chacun un atome d'hydrogène et (R_{b},R_{c}) forment ensemble avec les atomes de carbone qui les portent un groupe 1,3-dioxolane dans lequel un des atomes de carbone est optionnellement deutéré, un groupe 1,4-dioxane, un groupe 1,4-dioxépane, ou bien Rₐ, R_{c} et R_{d} représentent chacun un atome d'hydrogène et R_{b} représente un halogène, un méthyle, un méthoxy, un éthoxy, un trifluorométhyle ou un trifluorométhoxy.

Le groupement R₄ préféré est un 4-hydroxyphényle, 3-fluoro-4-hydroxyphényle ou 5-hydroxypyrimidine.

Dans les composés préférés, R₃ représente un groupement choisi parmi phényle, indole, indoline, 1,2,3,4-tétrahydroquinoline, 3,4-dihydro-2H-1,4-benzoxazine, indane, 1*H-*indazole, 1*H-*pyrrolo[2,3-*b*]pyridine, pyrimidine, cyclobutylméthyle, cyclopropylméthyle, 1*H-*pyrazole, pyridine, pyridazine, ces groupements comportant éventuellement un ou plusieurs substituants choisis parmi halogène, alkyle (C₁-C₆) linéaire ou ramifié, cyano ou alkoxy (C₁-C₆) linéaire ou ramifié.

Les composés préférés de l'invention sont listés ci-dessous :
- *N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-5,6,7,8-tétrahydro-1-indolizine carboxamide,
- 3-{5-chloro-2-[((3S)-3-(4-morpholinylméthyl)-3,4-dihydro-2(1*H*)-isoquinolinyl) carbanyl]phényl}-*N*-(4-hydroxyphényl)-*N-*(1-méthyl-1*H-*indol-5-yl)-5,6,7,8-tétrahydro-1-indolizine carboxamide,
- *N*(4-hydroxyphényl)-*N-*{1-méthyl-1*H-*indazol-5-yl)-3-{2,2-dideutério-6-[((3,*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide,
- N-(4-hydroxyphényl)-*N-*(1-méthyl-1*H*-indazol-5-yl)-3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizine-1-carboxamide,
- *N*-(4-hydroxyphényl)-3-{7-[((3*S*)-3-(4-morpholinylméthyl)-3,4-dihydro-2(*1H*)-isoquinolinyl)carbonyl]-2,3-dihydro-1,4-benzodioxin-6-yl}-*N*-phény1-5,6,7,8-tétrahydro-1-indolizine carboxamide,
- *N-*{4-hydroxyphényl)-*N-*(1-méthyl-1*H-*indol-5-yl)-3-{6-[((3*S*)-3-[(4-méthyl-1-pipérazinyl)méthyl]-3,4-dihydro-2(1*H*)-isoquinolinyl)carbonyl]-1,3-benzodioxol-5-yl}-1-indolizine carboxamide,
- *N*-[4-(hydroxy)phényl]-*N*-(1-méthyl-1*H*-indol-5-yl)-3-{6-[((3*S*)-3-(4-morpholinyl méthyl)-3,4-dihydro-2(1*H*)-isoquinolinyl)carbonyl]-1,3-benzodioxol-5-yl}-5,6,7,8-tétrahydro-1-indolizine carboxamide,
- *N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-1-indolizine carboxamide,
- 3-{5-chloro-2-[((3*S*)-3-(4-morpholinylméthyl)-3,4-dihydro-2(1*H*)-isoquinolinyl)carbonyl]phényl}-*N*-(4-hydroxyphényl)-*N*-(1-méthyl-1*H*-indol-5-yl)-1-indolizine carboxamide,
- 6-{5-chloro-2-[((3*S*)-3-(4-morpholinylméthyl)-3,4-dihydro-2(1*H*)-isoquinolinyl) carbonyl]phényl}-*N-*(3-fluoro-4-méthylphényl)-*N*-(4-hydroxyphényl)-3,4-dihydro-1*H-*pyrrolo[2,1-*c*][1,4]oxazine-8-carboxamide,
- 3-(5-chloro-2-{[(3*S*)-3-(morpholin-4-ylméthyl)-3,4-dihydroisoquinolin-2(1H)-yl]carbonyl}phényl)-*N*-(4-hydroxyphényl)-*N*-(1-méthyl-1*H-*pyrazol-4-yl)-5,6,7,8-tétrahydroindolizine-1-carboxamide,
- *N*-(3-fluoro-4-méthylphényl)-*N*-(4-hydroxyphényl)-3-{6-[((3*S*)-3-(4-morpholinylméthyl)-3,4-dihydro-2(1*H-*isoquinolinyl)carbonyl]-1,3-benzodioxol-5-yl}-5,6,7,8-tétrahydro-1-indolizine carboxamide,
- *N*-[4-(hydroxy)phényl]-*N*-(1-méthyl-2,3-dihydro-1*H-*indol-5-yl)-3-{6-[((3*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide,
- 3-{5-chloro-2-[((3*S*)-3-(4-morpholinylméthyl)-3,4-dihydro-2(1*H*)-isoquinolinyl)carbonyl]phényl}-*N*-(4-hydroxyphényl)-*N*-(1-méthyl-2,3-dihydro-1*H*-indol-5-yl)-5,6,7,8-tétrahydro-1-indolizine carboxamide,
- *N*-(4-hydroxyphényl)-N-(1-méthyl-1*H*-indol-5-yl)-3-{6-[((35)-3-(4-morpholinylméthyl)-3,4-dihydro-2(1*H*)-isoquinolinyl)carbonyl]-1,3-benzodioxol-5-yl}-1-indolizine carboxamide,
- 3-{5-chloro-2-[((3*S*)-3-(4-morpholinylméthyl)-3,4-dihydro-2(1*H*)-isoquinolinyl) carbonyl]phényl}-*N*-{4-hydroxyphényl)-*N*-(1-méthyl-2,3-dihydro-1*H* indol-5-yl)-1-indolizine carboxamide,
- 6-{5-chloro-2-[((3*S*)-3-(4-morpholinylméthyl)-3,4-dihydro-2(1*H*)-isoquinolinyl) carbonyl]phényl}-*N-*(4-hydroxyphényl)-*N*-phényl-3,4-dihydro-1*H-*pyrrolo[2,1-c][1,4]oxazine-8-carboxamide,
- *N*-(3-fluorophényl)-*N*-(4-hydroxyphényl)-3-(6-{[(3S)-3-(morpholin-4-ylméthyl)-3,4-dihydroisoquinolin-2(1*H*)-yl]carbonyl}-1,3-benzodioxol-5-yl)-5,6,7,8-tétrahydr oindolizine-1-carboxamide,
leurs énantiomères et diastéréoisomères, ainsi que leurs sels d'addition à un acide ou à une base pharmaceutiquement acceptable.

L'invention s'étend également au procédé de préparation des composés de formule (I) caractérisé en ce que l'on utilise comme produit de départ le composé de formule (II) : 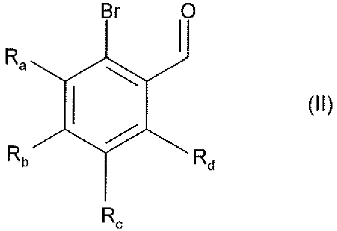 dans laquelle Rₐ, R_{b}, R_{c} et R_{d} sont tels que définis dans la formule (I),
composé de formule (II) qui est soumis à une réaction de Heck, en milieu aqueux ou organique, en présence d'un catalyseur au palladium, d'une base, d'une phosphine et du composé de formule (III) : 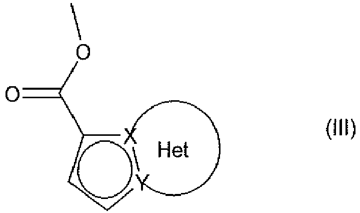 dans laquelle les groupements X, Y et Het sont tels que définis dans la formule (I),
pour obtenir le composé de formule (IV) : 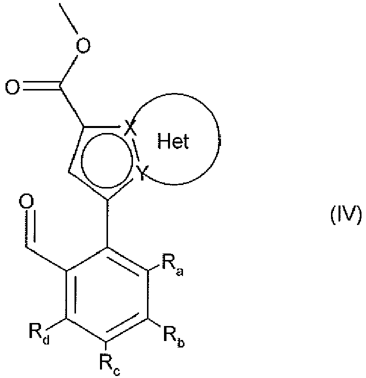 dans laquelle Rₐ, R_{b}, R_{c}, R_{d}, X, Y et Het sont tels que définis dans la formule (I),
composé de formule (IV) dont la fonction aldéhyde est oxydée en acide carboxylique pour former le composé de formule (V) : 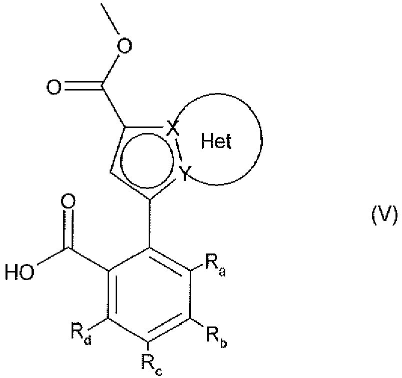 dans laquelle Rₐ, R_{b}, R_{c}, R_{d}, X, Y et Het sont tels que définis dans la formule (I),
composé de formule (V) qui subit ensuite un couplage peptidique avec un composé de formule (VI) : 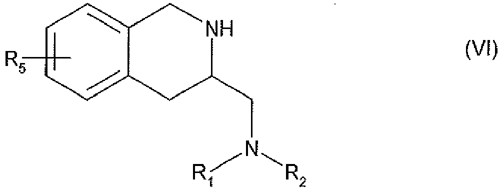 dans laquelle R₁, R₂, et R₅ sont tels que définis dans la formule (I),
pour conduire au composé de formule (VII) : 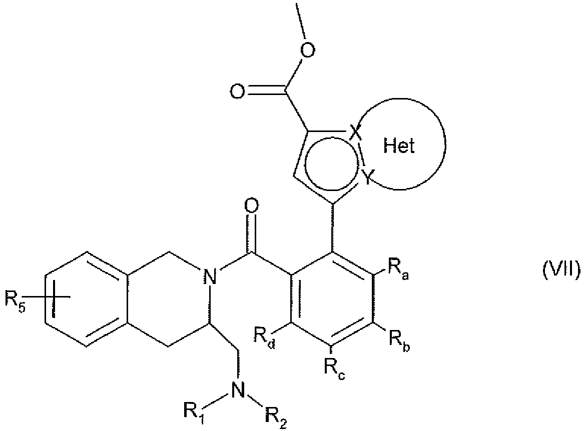 dans laquelle Rₐ, R_{b}, R_{c}, R_{d}, R₁, R₂, R₅, X, Y et Het sont tels que définis dans la formule (I), composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

Plus particulièrement, lorsque l'un des groupements R₃ ou R₄ de l'amine NHR₃R₄ est substitué par une fonction hydroxy, cette dernière peut être préalablement soumise à une réaction de protection avant tout couplage avec l'acide carboxylique du composé de formule (VII), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

Les composés de formules (II), (III), (VI), ainsi que l'amine NHR₃R₄, sont soit commerciaux, soit accessibles à l'homme du métier par des réactions chimiques classiques et décrites dans la littérature.

L'étude pharmacologique des dérivés de l'invention a montré qu'ils possédaient des propriétés pro-apoptotiques. La capacité à réactiver le processus apoptotique dans les cellules cancéreuses représente un intérêt thérapeutique majeur dans le traitement des cancers, des maladies immunitaires et auto-immunes.

Plus particulièrement, les composés selon l'invention seront utiles dans le traitement des cancers chimio ou radiorésistants, ainsi que dans les hémopathies malignes et le cancer du poumon à petites cellules.
Parmi les traitements des cancers envisagés on peut citer, sans s'y limiter, des cancers de la vessie, du cerveau, du sein, de l'utérus, des leucémies lymphoïdes chroniques, du cancer colorectal, des cancers de l'oesophage, du foie, des leucémies lymphoblastiques, des lymphomes folliculaires, des mélanomes, des hémopathies malignes, des myélomes, du cancer de l'ovaire, du cancer du poumon non à petites cellules, du cancer de la prostate et du cancer du poumon à petites cellules.

La présente invention a également pour objet les compositions pharmaceutiques contenant au moins un composé de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Par ailleurs, la présente invention concerne également l'association d'un composé de formule (I) avec un agent anticancéreux choisi parmi les agents génotoxiques, les poisons mitotiques, les anti-métabolites, les inhibiteurs du protéasome, les inhibiteurs de kinase, ou les anticorps, ainsi que les compositions pharmaceutiques contenant ce type d'association et leur utilisation pour la fabrication de médicaments utiles dans le traitement du cancer.

Les composés de l'invention peuvent également être utilisés en association avec une radiothérapie dans le traitement du cancer.

Les Préparations et Exemples suivants illustrent l'invention et ne la limitent en aucune façon.

### Préparation 1 : Acide 6-[1-(méthoxycarbonyl)-5,6,7,8-tétrahydro-3-indolizinyl]-1,3-benzodioxole-5-carboxylique

### Stade A : Acide 1-formyl-2-pipéridine carboxylique

A une solution de 40 g d'un mélange racémique d'acide 2-pipéridine carboxylique (0,310 mmol) dans 300 mL d'acide formique placée à 0°C, on ajoute goutte à goutte 200 mL (2,15 mmol) d'anhydride acétique. L'ensemble est ensuite agité à température ambiante durant une nuit. Puis, le milieu réactionnel est refroidi à 0°C, hydrolysé par l'addition de 250 mL d'eau, et agité pendant une demi-heure à 0°C avant d'être concentré à sec. L'huile ainsi obtenue est reprise dans 200 mL de méthanol puis concentrée à sec. Le produit du titre est obtenu sous la forme d'une huile avec un rendement de 98%. Il est utilisé directement, sans autre purification, pour l'étape suivante.

**RMN ¹H :** δ (400 MHz; dmso-d6; 300°K) : 13.0 (m, 1H OH); 8.0-8.05 (2s, 1H aldéhyde) ; 4.9-4.5 (2d, 1H α de N et COOH) ; 4.1-2.6 (m, 2H en α du N); 2.2-1.2 (m, 6H pipéridine).
**IR : ν** : -OH : 2000-3000 cm⁻¹ acide ; ν : >C=O 1703 cm⁻¹ bande large.

### Stade B : 5,6,7,8-Tétralrydro-1-indolizine carboxylate de méthyle

A une solution de 10 g d'acide carboxylique obtenu au Stade A (63,6 mmol) dans 65 mL de dichloroéthane sont ajoutés successivement 13,4 g de chlorure de tosyle (70,4 mmol), 11,5 mL de 2-chloroacrylate de méthyle (113,5 mmol), puis, au goutte à goutte, 17,8 mL de *N,N,N-*triéthylamine (127,2 mmol). Le milieu réactionnel est ensuite porté au reflux pendant 1h30. On se place ensuite à température ambiante, puis on ajoute 5 mL de 2-chloroacrylate de méthyle (48,9 mmol) et, au goutte à goutte, 9 m de *N,N,N* triéthylamine (64 mmol). L'ensemble est chauffé au reflux pendant une nuit.
Le milieu réactionnel est ensuite dilué avec du chlorure de méthylène, lavé successivement avec une solution HCl 1N, une solution saturée de NaHCO₃, puis une solution saturée de NaCl jusqu'à obtenir un pH neutre. La phase organique est alors séchée sur MgSO₄, filtrée, concentrée à sec, et purifiée par chromatographie sur gel de silice (gradient heptane AcOEt). On obtient le produit du titre sous la forme d'une huile.

**RMN ¹H.** δ (400 MHz ; CDCl₃ ; 300°K) : 6.55-6.40 (d, 2H, tétrahydroindolizine) ; 3.91 (t, 3H méthyl ester); 3.78 (s, 3H tétrahydroindolizine); 3.08 (t, 2H, tétrahydroindolizine); 1.95-1.85 (m, 4H, tétrahydroindolizine)
**IR : ν** :>C=O 1692 cm⁻¹ ester

### Stade C: 3-(6-Formyl-1,3-benzodioxol-5-yl)-5,6,7,8-tétrahydro-1-indolizine carboxylate de méthyle

A une solution de 6,4 g de l'ester obtenu au Stade B (35,7 mmol) dans 12 mL de *N,N-*diméthylacétamide, on ajoute successivement 12,3g de 6-bromo-1,3-benzodioxole-5-carbaldéhyde (53,6 mmol), 7 g d'acétate de potassium (71,4 mmol), puis l'ensemble est agité sous argon pendant 20 minutes. On ajoute alors 1,3 g de catalyseur au palladium PdCl₂(PPh₃)₂ (1,8 mol). Le milieu réactionnel est ensuite chauffé à 130°C pendant une heure avant d'y ajouter 139 µL d'H₂O. Le chauffage est maintenu à cette même température pendant la nuit. On laisse le milieu revenir à température ambiante, puis on le dilue avec de l'AcOEt. On ajoute du noir animal (2 g par g de produit) et l'ensemble est agité à température ambiante pendant 1 heure, puis filtré. La phase organique est alors lavée avec de l'eau, séchée sur sulfate de magnésium et concentrée à sec. Le produit brut ainsi obtenu est purifié sur gel de silice (gradient heptane/ ACOEt). Le produit du titre est obtenu sous la forme d'une huile.

**RMN¹H:** δ :(400 MHz ; dmso-d6 ; 353°K) : 9.65 (s, 1H, H aldéhyde) ; 7.3-7.15 (2s, 2H, H aromatiques) ; 6.45 (s, 1H tétrahydroindolizine) ; 6.20 (s, 2H méthylènedioxy); 3.70 (s, 3H méthyl ester) ; 3.5-4.0 (m, 2H tétrahydroindolizine) ; 3.05 (m, 2H tétrahydroindolizine) ; 1.85 (m, 4H tétrahydroindolizine)
**IR** : ν : >C=O 1695 cm⁻¹ ester; v : >C=O 1674 cm⁻¹

### Stade D : Acide 6-[1-(méthoxycarbonyl)-5,6,7,8-tétrahydro-3-indolizinyl]-1,3-benzodioxole-5-carboxylique

On prépare une solution contenant 3,37 g du composé obtenu au Stade C (10,3 mmol) dans 9,3 mL d'acétone et 8,8 mL (80,24 mmol) de 2-méthyl-2-butène, laquelle est placée à 0°C. On ajoute, goutte à goutte, 9,3 mL d'une solution aqueuse contenant un mélange de 3,3 g de NaClO₂ (36,05 mmol) et de 3,6 g de Na₂PO₄ (25,75 mmol). L'ensemble est ensuite agité à température ambiante durant 7 heures. Puis, le milieu réactionnel est concentré pour éliminer l'acétone. Le solide alors obtenu est filtré, lavé à l'eau puis séché sous vide à 40°C pendant une nuit. Le produit du titre est obtenu sous la forme d'un solide, qui est utilisé pour la suite sans autre purification.

**RMN ¹H** : δ (400 MHz ; dmso-d6 ; 300°K) : 12.10 (m, 1H, H acide carboxylique) ; 7.40-6.88 (2s, 2H, H aromatiques); 6.20 (s, 1H, H tétrahydroindolizine); 6.18 (s, 2H, H méthylènedioxy); 3.70 (s, 3H, méthyl ester) ; 3.55 (t, 2H tétrahydroindolizine) ; 3.00 (t, 2H tétrahydroindolizine) ; 1.80 (m, 4H, H tétrahydroindolizine)
**IR :** ν : -OH : 3000-2000 cm⁻¹ acide ; ν : >C=O 1686-1676 cm⁻¹ ester+acide ; ν : >C=C< 1608 cm⁻¹

### Préparation 2: Acide 4-bromo-2-[1-(méthoxycarbonyl)-5,6,7,8-tétrahydro-3-indolizinyl]benzoïque

On procède selon le procédé de la Préparation 1 en remplaçant le 6-bromo-1,3-benzodioxole-5-carbaldéhyde utilisé au Stade C par le 2,4-dibromobenzaldéhyde. On obtient un mélange de deux régioisomères qui sont séparés par chromatographie.

### Préparation 3 : Acide 4-chloro-2-[1-(méthoxycarbonyl)-5,6,7,8-tétrahydro-3-indolizinyl] benzoïque

On procède selon le procédé de la Préparation 1 en remplaçant le 6-bromo-1,3-benzodioxole-5-carbaldéhyde utilisé au Stade C par le 2-bromo-4-chlorobenzaldéhyde.

### Préparation 4 : Acide 4-fluoro-2-[1-(méthoxycarbonyl)-5,6,7,8-tétrabydro-3-indolizinyl]benzoïque

On procède selon le procédé de la Préparation 1 en remplaçant le 6-bromo-1,3-benzodioxole-5-carbaldéhyde utilisé au Stade C par le 2-bromo-4-fluorobenzaldéhyde.

### Préparation 5: Acide 8-[1-(méthoxycarbonyl)-5,6,7,8-tétrahydro-3-indolizinyl]-3,4-dihydro-2H-1,5-benzodioxépine-7-carboxylique

On procède selon le procédé de la Préparation 1 en remplaçant le 6-bromo-1,3-benzodioxole-5-carbaldéhyde utilisé au Stade C par le 8-bromo-3,4-dihydro-2*H*-1,5-benzodioxépine-7-carbaldéhyde.

### Préparation 6: Acide 4-méthoxy-2-[1-(méthoxycarbonyl)-5,6,7,8-tétrabydro-3-indolizinyl] benzoïque

On procède selon le procédé de la Préparation 1 en remplaçant le 6-bromo-1,3-benzodioxole-5-carbaldéhyde utilisé au Stade C par le 2-bromo-4-méthoxybenzaldéhyde.

### Préparation 7: Acide 7-[1-(méthoxycarbonyl)-5,6,7,8-tétrahydro-3-indolizinyl]-2,3-dihydro-1,4-benzodioxine-6-carboxylique

On procède selon le procédé de la Préparation 1 en remplaçant le 6-bromo-1,3-benzodioxole-5-carbaldéhyde utilisé au Stade C par le 7-bromo-2,3-dihydro-1,4-benzodioxine-6-carbaldéhyde.

### Préparation 8 : Acide 4-éthoxy-2-[1-(méthoxycarbonyl)-5,6,7,8-tétrahydro-3-indolizinyl]benzoïque

On procède selon le procédé de la Préparation 1 en remplaçant le 6-bromo-1,3-benzodioxole-5-carbaldéhyde utilisé au Stade C par le 2-bromo-4-éthoxybenzaldéhyde.

### Préparation 9: Acide 2,2-dideutério-6-[1-(méthoxycarbonyl)-5,6,7,8-tétrahydro-3-indolizinyl]-1,3-benzodioxole-5-carboxylique

### Stade A : 2,2-Dideutério-1,3-benzodioxole-5-carbaldéhyde

A une solution de 30 g de 3,4-dihydroxybenzaldéhyde (217 mmol) dans 110 mL de DMF anhydre, on ajoute successivement 114 g de carbonate de césium (347 mmol) et 22 mL de chlorure de méthylène dideutéré (347 mmol). Le milieu réactionnel est ensuite agité à température ambiante pendant une nuit. Après filtration sur célite de l'insoluble, le filtrat résiduel est ensuite hydrolysé par addition de 200 mL d'eau, puis extrait avec de l'acétate d'éthyle. Les phases organiques sont alors regroupées, lavées avec une solution saturée de LiCl, puis séchées sur MgSO₄. Après concentration à sec, le résidu est purifié par chromatographie sur gel de silice (gradient éther de pétrole / AcOEt). Le produit du titre est obtenu sous la forme d'un solide.

**RMN¹H:** δ : (400 MHz ; dmso-d6 ; 300°K) : 9.8 (s, 1H, H aldéhyde) ; 7.4-6.95 (m, 3H, H aromatiques)
**IR :** ν : C=O aldéhyde 1670 cm⁻¹

### Stade B : 6-Bromo-2,2-dideutério-1,3-benzodioxole-5-carbaldéhyde

A une solution de 10 g du composé obtenu au Stade A (65,7 mmol) dans 100 mL de méthanol refroidie à 0°C, on ajoute au goutte à goutte 3,7 mL d'une solution de brome (1.1 équivalent molaire) dans 10 mL de méthanol. L'ensemble est ensuite agité à température ambiante durant une nuit. Le milieu réactionnel est concentré à sec, puis repris avec de l'eau. Après agitation, le solide résultant est alors filtré puis séché.

**RMN¹H :** δ (400 MHz ; dmso-d6 ; 300°K) : 10.2 (s, 1H, H aldéhyde) ; 7.4 (s, 1H, H aromatique); 7.05 (s, 1H, H aromatique)
**IR :** ν : C=O aldéhyde : 1670 cm⁻¹ ; C=C aromatiques :1611 cm⁻¹

### Stade C: Acide 2,2-dideutério-6-[1-(méthoxycarbonyl)-5,6,7,8-tétrahydro-3-indoliziny/]-1,3-benzodioxole-5-canboxylique

On procède selon le protocole décrit aux Stades C et D de la Préparation 1 en remplaçant le 6-bromo-1,3-benzodioxole-5-carbaldéhyde utilisé au Stade C par le 6-bromo-2,2-dideutério-1,3-benzodioxole-5-carbaldéhyde.

### Préparation 10 : Acide 2-[1-(méthoxycarbonyl)-5,6,7,8-tétrahydro-3-indolizinyl]-4-(trifluorométhyl)benzoïque

On procède selon le protocole décrit à la Préparation 1 en remplaçant 6-bromo-1,3-benzodioxole-5-carbaldéhyde utilisé au Stade C par le 2-bromo-4-(trifluorométhyl)benzaldéhyde.

### Préparation 11 : Acide 2-[1-(méthoxycarbonyl)-5,6,7,8-tétrahydro-3-indolizinyl]-4-(trifluorométhoxy)benzoïque

On procède selon le protocole décrit à la Préparation 1 en remplaçant 6-bromo-1,3-benzodioxole-5-carbaldéhyde utilisé au Stade C par le 2-bromo-4-(trifluorométhoxy)benzaldéhyde.

### Préparation 12 : Acide 2-[1-(méthoxycarbonyl)-5,6,7,8-tétrahydro-3-indolizinyl]benzoïque

On procède selon le protocole décrit à la Préparation 1 en remplaçant 6-bromo-1,3-benzodioxole-5-carbaldéhyde utilisé au Stade C par le 2-bromobenzaldéhyde.

### Préparation 13: Acide 6-[1-(méthoxycarbonyl)-3-indolizinyl]-1,3-benzodioxole-5-carboxylique

### Stade A : Bromure de 1-(carboxymthéthyl)-1,2-dihydropyridinium

A une solution de 16,2 mL de pyridine (200 mmol) dans 120 mL d'acétate d'éthyle, on ajoute par portions 27,8 g (200 mmoles) d'acide bromoacétique. L'ensemble est ensuite agité à température ambiante pendant une nuit. Le précipité ainsi obtenu est filtré, puis lavé avec de l'actétate d'éthyle froid. Après séchage, on obtient le produit du titre sous la forme d'une poudre qui est utilisée directement pour l'étape suivante.

**RMN ¹H:** δ (400 MHz ; dmso-d6 ; 300°K) : 9.15 (d, 2H, H aromatiques pyridine)) ; 8.7 (t, 1H, H aromatique) ; 8.25 (t, 2H, H aromatique); 5.65 (s, 2H, H CH₂COOH)
**IR :** ν : C=O : 1732 cm⁻¹ ; -OH acide : 2800 cm⁻¹

### Stade B ; 1-Indolizinecarboxylate de méthyle

A une suspension de 6,55 g du sel de pyridinium obtenu au Stade A (30 mmol) dans 240 mL de toluène, on ajoute successivement 16,7 mL d'acrylate de méthyle (150 mmol), 4,2 mL de triéthylamine (30 mmol), puis par portions 20,9g de MnO₂ (240 mmol). L'ensemble est ensuite chauffé à 90°C durant 3 h. Après refroisissement, le milieu réactionnel est filtré sur un gâteau de célite et concentré à sec. Le produit du titre est alors isolé par purification sur gel de silice (gradient heptane /AcOEt: 0-10%) sous la forme d'une huile qui cristallise à froid.

**RMN ¹H: δ** (300 MHz ; dmso-d6 ; 300°K) : 8.5 (d, 1H, H indolizine) ; 8.05 (d, 1H, H indolizine); 7.6 (s, 1H, H indolizine); 7.15 (m, 2H, H indolizine); 6.85 (m, 1H, H indolizine); 4.25 (q, 2H, -C(O)CH₂CH₃); 1.35 (t, 3H, -C(O)CH₂CH₃)
**IR :** ν : C=O ester : 1675 cm⁻¹ , C=C aromatiques : 1634 cm⁻¹

### Stade C : Acide 6-[1-(méthoxycarbonyl)-3-indolizinyl]-1,3-benzodioxole-5-carboxylique

On procède selon le protocole décrit aux Stades C et D de la Préparation 1.

### Préparation 14 : Acide 4-chloro-2-[1-(méthoxycarbonyl)-3-indolizinyl]benzoïque

On forme la 1-indolizinecarboxylate de méthyle selon le procédé décrit aux Stades A et B de la Préparation 13. Le produit du titre est ensuite obtenu suivant le protocole décrit aux Stades C et D de la Préparation 1 en remplaçant le 6-bromo-1,3-benzodioxole-5-carbaldéhyde utilisé au Stade C par le 2-bromo-4-chlorobenzaldéhyde.

### Préparation 15 : Acide 7-[1-(méthoxycarbonyl)-3-indolizinyl]-2,3-dihydro-1,4-benzodioxine-6-carboxylique

On forme la 1-indolizinecarboxylate de méthyle selon le procédé décrit aux Stades A et B de la Préparation 13. Le produit du titre est ensuite obtenu suivant le protocole décrit aux Stades C et D de la Préparation 1 en remplaçant le 6-bromo-1,3-benzodioxole-5-carbaldéhyde utilisé au Stade C par le 7-bromo-2,3-dihydro-1,4-benzodioxine-6-carbaldéhyde.

### Préparation 16 : Acide 4-méthoxy-2-[1-(méthoxycarbonyl)-3-indolizinyl]benzoïque

On forme la 1-indolizinecarboxylate de méthyle selon le procédé décrit aux Stades A et B de la Préparation 13. Le produit du titre est ensuite obtenu suivant le protocole décrit aux Stades C et D de la Préparation 1 en remplaçant le 6-bromo-1,3-benzodioxole-5-carbaldéhyde utilisé au Stade C par le 2-bromo-4-méthoxybenzaldéhyde.

### Préparation 17 : Acide 6-[2-(tert-butoxycarbonyl)-8-(méthoxycarbonyl)-1,2,3,4-tétrahydropyrrolo[1,2-a]pyrazin-6-yl]-1,3-benzodioxoIe-5-carboxylique

### Stade A : 4-Formyl-1,3-pipérazinedicarboxylate de 1-tert-butyle et de 3-méthyle

A une solution de pentafluorophénol dans 520 mL d'éther anhydre placée à 0°C, on ajoute successivement 49 g d'1-éthyl-3-(3'-diméthylaminopropyl)-carbodiimide (286 mmol) par portions et 12 mL d'acide formique (312 mmol). L'ensemble est agité à température ambiante pendant 2h. On ajoute ensuite un mélange de 32 g de 1,3-pipérazinedicarboxylate de 1-*tert*-butyle et de 3-méthyle (130 mmol) et de 18 mL de triéthylamine (130 mmol) en solution dans 520 mL de CH₂Cl₂. L'ensemble est agité pendant une nuit à température ambiante. Le milieu réactionnel est hydrolysé avec une solution aqueuse de HCl, 1N et extrait avec du CH₂Cl₂. Les phases organiques sont alors regroupées, puis lavées avec une solution aqueuse de NaHCO₃ saturée, puis avec une solution aqueuse de NaCl saturée jusqu'à neutralité. Après séchage sur MgSO₄, filtration et concentration à sec, on isole le produit par chromatographie sur gel de silice (gradient éther de pétrole / AcOEt : 0-30%). On obtient le produit du titre sous la forme d'une huile.

**IR** : ν : C=O : 1674-1745 cm⁻¹
**m/z** (C₁₂H₂₀N₂O₅): 272.1(M+); 295.121 (M+Na)⁺; 567.253 (2M+Na)⁺

### Stade B : 4-(tert-Butoxycanbonyl)-1-formyl-2 pipérazinecarboxylate de lithium

A une solution de 28 g du composé obtenu au Stade A (103 mmol) dans 515 mL de dioxane, on ajoute 4,8 g de LiOH (113 mmol) en solution dans 100 mL d'H₂O. L'ensemble est agité à température ambiante durant 4 h. Le milieu réactionnel est ensuite concentré à sec, puis co-évaporé plusieurs fois avec de l'acétate d'éthyle. Le produit du titre est obtenu sous la forme d'un solide et est utilisé directement pour l'étape suivante de cyclisation.

**RMN¹³C:** δ (500 MHz; ; dmso-d6 ; 300°K) : 46 (s, C pipérazine) ; 42-38 (m, C pipérazine) ; 58-53 (s, C pipérazine) ; 28.5 (s, C ^{t}Bu).
**IR** : ν : C=O : 1650 cm⁻¹ ; 2800 cm⁻¹

### Stade C : 3,4-Dihydropyrrolo[1,2-a]pyrazine-2,8(1H)-dicarboxylate de 2-tert-butyle et de 8-méthyle,

A une suspension de 29 g du composé obtenu au Stade B (103 mmoles) dans 800 mL de dichloroéthane, on ajoute successivement 24 g de chlorure de tosyle (124 mol), 12.6 mL de 2-chloroacrylate de méthyle (124 mmol), puis 35 mL de triéthylamine (247 mmoles). L'ensemble est agité au reflux durant 2h. Après refroidissement, le mileu réactionnel est dilué avec de l'acétate d'éthyle et la phase organique est lavée avec une solution de NaCl saturée jusqu'à neutralité. Après séchage sur MgSO₄, filtration et concentration à sec, le produit du titre est isolé par chromatographie sur gel de silice (gradient éther de pétrole / AcOEt : 0-20%) sous la forme d'un solide.

**RMN ¹H:** δ (400 MHz ; dmso-d6 ; 300°K) : 6.8-6.43 (m, 2H, H pyrrole); 4.75-3.75 (m, 6H, H pipérazine)) ; 3.73 (s, 3H, H COOCH3) ; 1.48 (s, 9H, H ^{t}Bu).
**IR** : ν : C=O (ester conjugué) : 1712 cm⁻¹ , C=O (carbamate) : 1677cm⁻¹

### Stade D : Acide 6-[2-(tert-butoxycarboxyl)-8-(méthoxycarbonyl)-1,2,3,4-tétrahydroprrolo[1,2-a]pyryrazin-6-yl]-1,3-benzodioxole-5-carboxylique

On procède selon le protocole décrit aux Stades C et D de la Préparation 1.

### Préparation 18 : Acide 6-[7-(méthoxycarbonyl)-2,3-dihydro-1H-pyrrolizin-5-yl]-1,3-benzodioxole-5-carboxylique

On procède selon le protocole décrit à la Préparation 1 en remplaçant l'acide 2-pipéridine carboxylique utilisé au Stade A par la proline.

### Préparation 19 : Acide 4-chloro-2-[7-(méthoxycarbonyl)-2,3-dihydi-o-1H-pyrrolizin-5-yl]benzoïque

On procède selon le protocole décrit à la Préparation 1 en remplaçant l'acide 2-pipéridine carboxylique utilisé au Stade A par la proline, alors que le 6-bromo-1,3-benzodioxole-5-carbaldéhyde utilisé au Stade C est remplacé par le 2-bromo-4-chlorobenzaldéhyde.

### Préparation 20 : Acide 4-chloro-2-[8-(méthoxycarbonyl)-3,4-dihydro-1H-pyrrolo[2,1-c][1,4]oxazin-6-yl]benzoïque

On procède selon le protocole décrit à la Préparation 1 en remplaçant l'acide 2-pipéridine carboxylique par l'acide 3-morpholinecarboxylique, alors que le 6-bromo-1,3-benzodioxole-5-carbaldéhyde utilisé au Stade C est remplacé par le 2-bromo-4-chlorobenzaldéhyde.

### Préparation 21 : Acide 6-[8-(méthoxycarbonyl)-3,4-dihydro-1H-pyrrolo[2,1-c][1,4]oxazin-6-yl]-1,3-benzodioxole-5-carboxylique

On procède selon le protocole décrit à la Préparation 1 en remplaçant l'acide 2-pipéridine carboxylique par l'acide 3-morpholinecarboxylique.

### Préparation 22: Acide 4-chloro-2-[1-(méthoxycarbonyl)-6,7,8,9-tétrahydro-5H-pyrrolo[1,2-a]azépin-3-yl]benzoïque

On procède selon le protocole décrit à la Préparation 1 en remplaçant l'acide 2-pipéridine carboxylique par l'acide 2-azépanecarboxylique, alors que le 6-bromo-1,3-benzodioxole-5-carbaldéhyde utilisé au Stade C est remplacé par le 2-bromo-4-chlorobenzaldéhyde.

### Préparation 23 : Acide 6-[1-(méthoxycarbonyl)-6,7,8,9-tétrahydro-5H-pyrrolo[1,2-a] azépin-3-yl]-1,3-benzodioxole-5-carboxylique

On procède selon le protocole décrit à la Préparation 1 en remplaçant l'acide 2-pipéridine carboxylique par l'acide 2-azépanecarboxylique.

### Préparation 24 : Acide 4-chloro-2-[3-(méthoxycarbonyl)-5,6,7,8-tétrahydro-1-indolizinyl]benzoïque

### Stade A : 1-(4-bromobutyl)-1H-pyrrole-2-carboxylate de méthyle

A une suspension de 6,7 g (241.7 mmol) de NaH (60%) dans 400 mL de THF anhydre placé à 0°C, on ajoute 20 g (161.13 mmol) de 1*H-*pyrrole-2-carboxylate de méthyle (voir Tétrahedron 2008, 64, 7745). L'ensemble est agité à température ambiante durant une heure. Puis, on ajoute 95 mL de 1,4-dibromobutane. Après addition, la réaction est chauffée à reflux pendant 12 h. Le précipité obtenu est filtré puis lavé avec du THF. Le filtrat est ensuite concentré à sec. Le composé est alors isolé par chromatographie sur gel de silice (gradient cyclohexane / acétate d'éthyle : 0 à 20%) sous la forme d'une huile.

| ***Microanalyse élémentaire:*** | | | | |
|---|---|---|---|---|
| | %C | *%H* | *%N* | *%Br* |
| *Calculé* | *46.17* | *5.42* | *5.38* | *30.72* |
| *Trouvé* | *46.76* | *5.56* | *5*.*29* | *30.77* |

**IR** : ν : -C=O: 1700 cm⁻¹ v : C-O-C : 1238 cm⁻¹

### Stade B : 5,6,7,8-Tétrahydro-3-indolizinecarboxylate de méthyle

Une solution de 8 g (30,8 mmol) du dérivé bromé obtenu au Stade A dans 700 mL d'acétonitrile est portée à reflux. On y ajoute une solution d'un mélange de 25 g d'azobisisobutyronitrile (151 mmol) et de 30 g de Bu₃SnH (100 mmol) dans 500 mL de toluène. L'ensemble est porté à reflux pendant 120 h. Le milieu réactionnel est ensuite concentré à sec. Le composé est alors isolé par chromatographie sur gel de silice (gradient de cyclohexane / acétate d'éthyle 5 à 10%) sous la forme d'une huile.

**RMN ¹H :** δ (400 MHz; CDCl₃ ; 300°K) : 6.90 (d, 1H, H pyrrole); 5.85 (d, 1H, H pyrrole); 4.30 (t, 2H, CH₂ indolizine); 3.80 (s 3H, Me); 2.80 (t, 2H, CH₂ indolizine); 1.95 (m, 2H, CH₂ indolizine); 1.80 (m, 2H, CH₂ indolizine)
**IR:** ν : -C=O : 1695 cm⁻¹

### Stade C : Acide 4-chloro-2-[3-(méthoxycarbonyl)-5,6,7,8-tétrahydro-1-indolizinyl] benzoïque

On procède selon le protocole décrit aux Stades C et D de la Préparation 1.

### Préparation 25 : Acide 2-[1-(méthoxycarbonyl)-5,6,7,8-tétrahydroindolizin-3-yl]-4-méthylbenzoïque

On procède selon le procédé de la Préparation 1 en remplaçant le 6-bromo-1,3-benzodioxole-5-carbaldéhyde utilisé au Stade C par le 2-bromo-4-méthylbenzaldéhyde.

### Préparation 26 : Acide 4-fluoro-2-[8-(méthoxycarbonyl)-3,4-dihydro-1H-pyrrolo[2,1-c][1,4]oxazin-6-yl]benzoïque

On procède selon le protocole décrit à la Préparation 1 en remplaçant l'acide 2-pipéridine carboxylique par l'acide 3-morpholinecarboxylique, alors que le 6-brome-1,3-benzodioxole-5-carbaldéhyde utilisé au Stade C est remplacé par le 2-bromo-4-fluorobenzaldéhyde.

### Préparation 27 : Acide 4-fluoro-2-[1'-(méthoxycarbonyl)-5',6-dihydro-8'H-spiro[1,3-dioxolane-2,7'-indolizin]-3'-yl]benzoïque

### Stade A : 8-Formyl-1,4-dioxa-8-azaspiro[4.5]dlécarte-7-carboxylate de méthyle

24 g de 1,4-dioxa-8-azaspiro[4.5]décane-9-carboxylate de méthyle (111 mmol) sont solubilisés dans 80 mL d'acétate d'éthyle et 80 mL de dichloromethane. On ajoute 26 g de (4-nitrophényl)formate (155 mmol) et l'ensemble est agité à température ambiante pendant 1 h. Le milieu réactionnel est évaporé à sec et repris dans l'acétate d'éthyle. La phase organique est ensuite lavée successivement avec une solution de NaOH 1 N, de l'eau, puis avec une solution de NH₄Cl saturée jusqu'à atteindre un pH neutre. Elle est ensuite séchée sur sulfate de magnésium, filtrée et concentrée à sec. L'huile ainsi obtenue est purifiée par chromatographie flash (gradient heptane/acétate d'éthyle). On obtient le produit du titre sous la forme d'une huile.

**RMN¹H:** δ (400 MHz; dmso-d6; 300°K) : 8.15 (s, 1H, CHO); 5.0-4.75 (m, 1H, H tertiaire) ; 4.3-3.7 (m, 5H, 4H éthylènedioxy + 1H aliphatique pipéridine) ; 3.70 (s, 3H, Me) ; 3.4-2.9 (2m, 1H, H aliphatique pipéridine) ; 2.3-1.75 (m, 2H, H aliphatique pipéridine) ; 1.7-1.5 (m, 2H, H aliphatique pipéridine)

### Stade B : Acide 8-formyl-1,4-dioxa-8-azaspiro[4.5]décane-carboxylique

15,25 g du composé obtenu au Stade A (62,7 mmol) est mis en solution dans 160 ml de dioxane, Une solution de 125 mL de KOH 1M est additionnée goutte à goutte et l'ensemble est agité à température ambiante pendant 1 h. On ajoute ensuite 125 mL de HCl 1M et on extrait le composé avec du dichlorométhane. La phase organique est séchée sur MgSO₄, filtrée et concentré à sec. On obtient le produit du titre sous la forme d'une poudre.

**RMN¹H:** δ (400 MHz; dmso-d6 ; 300°K) 13.5-12 (m, 1H, OH) ; 8.1 + 8.0 (2s, 1H, CHO) ; 4.9 + 4.6 (2m, 1H, H tertiaire) ; 4.0-3.8 (m, 4H, éthylènedioxy) ; 4.2 +3.7 (2ms, 1H, H aliphatique pipéridine) ; 3.4 + 2.9 (2m, 1H, H aliphatique pipéridine) ; 2.4-1.5 (m, 4H, H aliphatique pipéridine)
**IR:** ν : OH: 3500-2000 cm⁻¹ -C=O (acide + aldéhyde) :1731 + 1655 cm⁻¹

### Stade C: 5',6'-Dihydro-8'H-spiro[1,3-dioxolane-2,7'-indolizine]-1'-carboxylate de méthyle

A une solution de 13,5 g (62,7 mmol) de l'acide obtenu au Stade B dans 380 mL de dichloroéthane, on ajoute successivement 39,5 mL (238,4 mmol) de triéthylamine, puis par pelleté 12,5 g (65,6 mmol) de chlorure de *para*-toluène sulfonyle et 23,7 mL (238,4 mmol) de chloroacrylate de méthyle. L'ensemble est agité à 80°C pendant 18h. Le milieu réactionnel est ensuite filtré sur célite. Le filtrat est ensuite lavé avec une solution saturée de NaHCO₃ puis avec une solution saturée de NH₄Cl. La phase organique est séchée sur MgSO₄, filtrée et concentré à sec. L'huile ainsi obtenue est purifiée par chromatographie flash (gradient heptane/acétate d'éthyle). Le produit est obtenu sous la forme d'un solide.

**RMN** 1**H**: δ (400 MHz ; dmso-d6 ; 300°K) 6.70 (d, 1H, pyrrol) ; 6.40 (d, 1H, pyrrole) ; 4.05 (t, 2H, H aliphatique, pipéridine) ; 4.00 (m, 4H, éthylènedioxy) ; 3.70 (s, 3H, méthyle); 3.15 (s, 2H, H aliphatique pipéridine) ; 2.05 (t, 2H, H aliphatique pipéridine) **IR:** ν :-C=O (ester): 1689 cm⁻¹

### Stade D : 3'-(5-Fluroro-2 formylphéhyl)-5',6'-dihydro-8'H-spiro[1,3-dioxolane-2,7'-indolizine]-1'-carboxylate de méthyle

On procède selon le procédé du Stade C de la Préparation 1 en remplaçant le 6-bromo-1.3-benzodioxole-5-carbaldéhyde par le 2-bromo-4-fluorobenzaldéhyde.

### Stade E : Acide 4-fluoro-2-[1'-(méthoxycarbonyl)-5',6'-dihydro-8'H-spiro[1,3-dioxolante-2,7'-indolizin]-3'-yl]benzoïque

On procède selon le procédé du Stade D de la Préparation 1.

### Préparation 28 : Acide 4-fluoro-2-[(2R)-2-hydroxy-7-(méthoxycarbonyl)-2,3-dihydro-1H-pyrrolizin-5-yl]benzoïque

### Stade A : (4R)-4-{[tert Butyl(diméthyl)silyl]oxy}-L-prolinate de méthyle

La protection de la fonction alcool du (4*R*)-4-hydroxy-*L*-prolinate de méthyle par un groupement *ter*-butyldiméthylsilyl est réalisée selon le protocole décrit dans le document WO 2012040242.

### Stade B : (4R)-4-{[tert-Butyl(diméthyl)silyl]oxy}-1-formyl-L-prolinate de méthyle

13,7 g (52,8 mmol) de composé du Stade A sont solubilisés dans 100 mL d'acétonitrile. On y ajoute 13,2 g (79,3 mmol) de (4-nitrophényl)formate et 39 mL (238 mmol) de diisopropyléthylamine. L'ensemble est agité à température ambiante pendant 6 h. Le milieu réactionnel est évaporé à sec et repris dans l'acétate d'éthyle. La phase organique est ensuite lavée successivement avec une solution 1 N de NaOH, avec de l'eau, puis avec une solution de NH₄Cl saturée jusqu'à neutralité. Elle est ensuite séchée sur sulfate de magnésium, filtrée et concentrée à sec. L'huile ainsi obtenue est purifiée par chromatographie flash (gradient : dichlorométhane /méthanol ammoniacal). On obtient le produit du titre sous la forme d'une huile.

**RMN ¹H:** δ (400 MHz ; dmso-d6 ; 300°K) : 8.15 et 8.12 (s, 1H, formyl); 4.62 et 4.25 (t, 1H, H alpha ester) ; 4.40 (m, 1H, SiOCH,) ; 3.65 et 3.6 (s, 3H, OMe) ; 3.5 et 3.3 (m, 2H, 2H proline) ; 2.12 et 1.95 (m, 2H, 2H proline) ; 0.8 (s, 9H, Si^{t}Bu) ; 0.05 (s, 6H, SiMe₂). **IR:** ν : C=O ester : 1749 cm⁻¹ ; C=O formyl : 1659 cm⁻¹

### Stade C : (2S,4R)-4{[tert-Butyl(diméthyl)silyl]oxy}-1-formylpyrrolidine-2-carboxylate de lithium

Le composé du Stade B (26,2 g ; 91,1 mmol) est solubilisé dans 450 mL de dioxane. Une solution de lithine (4,2 g ; 100 mmol) dans l'eau (90 mL) est ajoutée. L'ensemble est agité à température ambiante pendant 7 h. Le milieu réactionnel est évaporé à sec et utilisé tel quel pour l'étape suivante.

### Stade D : (2R)-2-{[tert-Butyl(diméthyl}silyl]oxy}-2,3-dihydro-1H-pyrrolizine-7-carboxylate de méthyle

A une solution de 22,4 g (80.2 mmol) du carboxylate de lithium obtenu au Stade D dans 640 mL de dichloroéthane, on ajoute successivement 27 mL (192 mmol) de triéthylamine, puis, par portion, 18 g (96 mmol) de chlorure de *para*-toluène sulfonique et 9,7 mL (96,2 mmol) de chloroacrylate de méthyle. Le milieu réactionnel est chauffé au reflux pendant 18 h, puis refroidi et dilué dans l'acétate d'éthyle. La phase organique est lavée avec de l'eau, de la saumure, avant d'être séchée sur MgSO₄. Après filtration et concentration du milieu, l'huile obtenue est purifiée par chromatographie flash (gradient : heptane/acétate d'éthyle). On obtient le produit du titre sous la forme d'une huile.

**RMN ¹H:** δ (400 MHz ; dmso-d6 ; 300°K) : 6.7 (d, 1H, H pyrrole); 6.4 (d, 1H, H pyrrole) ; 5.0 (m, 1H, SiOCH,) ; 4.2-3.75 (ABx, 2H, 2H dihydropyrrolizine) ; 3.3+2.8 (ABx, 2H, 2H dihydropyrrolizine) ; 3.70 (s, 3H, CO₂CH₃) ; 0.9 (s, 9H, ^{t}Bu) ; 0.10 (ABx, 6H, SiMe₂)
**IR:** ν : -C=O (ester) : 1701 cm⁻¹; SiCH₃ : 1249 cm⁻¹; Si-O : 1118-1090 cm⁻¹; Si-C : 835-777 cm⁻¹

### Stade E : (2R)-2-{[tert-butyl(diméthyl)silyl]oxy}-5(5-fluoro-2-formylphényl)-2,3-dihydro-1H-pyrrolizine-7-carboxylate de méthyle

On procède selon le procédé du Stade C de la Préparation 1 en remplaçant le 6-bromo-1.3-benzodioxole-5-carbaldéhyde par le 2-bromo-4-fluorobenzaldéhyde.

### Stade F: Acide 2-[(2R)-2-{[tert-butyl(diméthyl)silyl]oxy}-7(méthoxycarbonyl)-2,3-dihydro-1H-pyrrolizin-5-yl]-4-fluorobenzoïque

On procède selon le procédé du Stade D de la Préparation 1.

### Préparation 29 : Acide 4-fluoro-2-[(2S)-2-hydroxy-7-(méthoxycarbonyl)-2,3-dihydro-1H-pyrrolizin-5-yl]benzoïque

On procède selon le procédé de la Préparation 28 en remplaçant le (4*R*)-4-hydroxy-*L-*prolinate de méthyle utilisé au Stade A par le (4*S*)-4-hydroxy-*L*-prolinate de méthyle.

### Préparation 30: Acide 4-fluoro-2-[7-hydroxy-1-(méthoxycarbonyl)-5,6,7,8-tétrahydroindolizin-3-yl]benzoïque

On procède selon le procédé de la Préparation 27 en remplaçant le 2-bromo-4-fluorobenzaldéhyde utilisé au Stade D par le 6-bromo-1.3-benzodioxole-5-carbaldéhyde.

### Préparation 31 : Acide 6-[8-(méthoxycarbonyl)pyrrolo[1,2-a]pyrazin-6-yl]-1,3-benzodioxole-5-carboxylique

### Stade A : Pyrrolo[1,2-a]pyrazine-8-carboxylate de méthyle

A une solution de 10 g (65,7 mmol) de pyrazin-2-ylacetate de méthyle dans 263 mL d'acétone anhydre (4 mL/mmol), on ajoute successivement 5.7 g de bromure de lithium (65.7 mmol), 22,1 g de bicarbonate de sodium (263 mmol), puis 9,4 mL de chloroacétaldéhyde (solution à 50% dans l'eau) (72.6 mmoles). L'ensemble est ensuite chauffé à reflux toute une nuit. Le milieu réactionnel est ensuite concentré à sec, puis repris dans l'acétate d'éthyle. La phase organique est lavée avec une solution saturée de chlorure de sodium, séchée sur sulfate de magnésium, filtrée puis concentrée à sec. On obtient une huile qui est purifiée par chromatographie sur gel de silice (gradient CH₂Cl₂/ acétate d'éthyle). Après concentration, on obtient le produit du titre sous la forme d'un solide.

**RMN ¹H:** δ (400 MHz ; dmso-d6 ; 300°K) : 9.36 (m, 1H, H pyrazine); 8.50 (dd, 1H, H pyrazine)) ; 7.8 (m, 2H, 1H pyrazine, 1H pyrrole) ; 7.31 (m, 1H, H pyrrole). 3.88 (s, 3H, OCH₃)
**IR :** ν : -C=O (ester conjugué) : 1686 cm⁻¹

### Stade B: Acide 6-[8-(méthoxycarbonyl)pyrrolo[1,2-a]pyrazin-6-yl]-1,3-benzodioxole-5-carboxylique

On procède selon les procédés des Stades C et D de la Préparation 1.

### Préparation 32 : Acide 4-fluoro-2-[1-(méthoxycarbonyl)-3-indolizinyl]benzoïque

On forme la 1-indolizinecarboxylate de méthyle selon le procédé décrit aux Stades A et B de la Préparation 13. Le produit du titre est ensuite obtenu suivant le protocole décrit aux Stades C et D de la Préparation 1 en remplaçant le 6-bromo-1,3-benzodioxole-5-carbaldéhyde utilisé au Stade C par le 2-bromo-4-fluorobenzaldéhyde.

### Préparation 1' : (3S)-3-(4-Morpholinylméthyl)-1,2,3,4-tétrahydroisoquinoline

### Stade A : (3S)-3-(4-Morpholinylcarbonyl)-3,4-dihydro-2(1H)-isoquinoline carboxylate de benzyle

A une solution de 5 g d'acide (3*S*)-2-[(benzyloxy)carbonyl]-1,2,3,4-tétrahydro-3-isoquinolinecarboxylique (16 mmol) dans 160 mL de dichlorométhane sont ajoutés 1,5 mL de morpholine (17,6 mmol), puis 9 mL de *N,N,N-*triéthylamine (64 mmol), 3,3 g de 1-éthyl-3-(3'-diméthylaminopropyl)-carbodiimide (EDC) (19,2 mmol), et 2,6 g d'hydroxybenzotriazole (HOBT) (19,2 mmol). Le milieu réactionnel est agité à température ambiante pendant une nuit, puis il est versé sur une solution de chlorure d'ammonium et extrait avec de l'acétate d'éthyle. La phase organique est ensuite séchée sur sulfate de magnésium, puis filtrée et évaporée à sec. Le produit brut ainsi obtenu est alors purifié par chromatographie sur gel de silice (gradient dichlorométhane/ méthanol). Le produit est obtenu sous la forme d'une mousse.

**RMN ¹H :** δ (400 MHz; dmso-d6; 353°K): 7.30 (m, 5H benzyl); 7.15 (m, 4H aromatiques) ; 5.2-5.0 (m, 3H, 2H benzyl, 1H dihydroisoquinoline); 4.75-4.5 (2d, 2H dihydroisoquinoline); 3.55-3.3 (m, 8H morpholine); 3.15-2.9 (2dd, 2H dihydroisoquinoline)
**IR :** ν : >C=O : 1694 ;1650 cm⁻¹

### Stade B : (3S)-3-(4-Morpholinyiméthyl)-3,4-dihydro-2(1H)-isoquinoline carboxylate de benzyle

A une solution de 5,3 g du produit obtenu au Stade A (13,9 mmol) dans 278 mL de tétrahydrofurane sont ajoutés 14 mL de BH₃Me₂S (27,8 mmol) à température ambiante. Le tout est chauffé pendant 4 heures à 80°C. On laisse revenir à température ambiante, puis on ajoute 7 mL (14 mmol) de BH₃Me₂S. Le milieu réactionnel est de nouveau chauffé à 80°C pendant 2 heures. On évapore ensuite le tétrahydrofurane, puis on ajoute lentement du méthanol, puis 5,6 mL d'acide chlorhydrique 5N (27,8 mmol). Le mélange est agité à température ambiante pendant une nuit, puis à 80°C pendant 1h. On ajoute ensuite une solution de NaHCO₃ saturée sur le milieu réactionnel placé à 0°C jusqu'à atteindre un pH=8, puis on extrait avec de l'acétate d'éthyle. La phase organique est ensuite séchée sur sulfate de magnésium, puis filtrée et évaporée à sec. Le produit du titre est obtenu sous la forme d'une huile.

**RMN¹H:** δ (400 MHz ; dmso-d6 ; 353°K) : 7.43-7.30 (massif, 5H benzyl) ; 7.19 (m, 4H aromatiques) ; 5.16 (m, 2H, 2H benzyl) ; 4.79-4.29 (d, 2H dihydroisoquinoline) ; 4.58 (m, 1H dihydroisoquinoline) ; 3.50 (m, 4H morpholine) ; 3.02-2.80 (dd, 2H dihydroisoquinoline) ; 2.42-2.28 (massif, 5H, 4H morpholine, 1H morpholine) ; 2.15 (dd, 1H morpholine)
**IR :** ν : >CH : 2810 cm⁻¹ ; ν : >C=O : 1694 cm⁻¹ ; ν : >C-O-C< : 1114 cm⁻¹ ; ν : >CH-Ar : 751 ; 697 cm⁻¹

### Stade C : (3S)-3-(4-Morpholinylméthyl)-1,2,3,4-tétrahydroisoquinoline

A une solution de 4,9 g du composé du Stade B (13,4 mmol) dans 67 mL d'éthanol est ajouté 0,980 g de dihydroxide de palladium (20% massique) à température ambiante. Le milieu réactionnel est placé sous 1,2 bar de d'hydrogène à température ambiante pendant 4 heures. Il est ensuite passé sur une filtre Wattman, puis le palladium est rincé plusieurs fois avec de l'éthanol. Le filtrat est évaporé à sec. Le produit du titre est obtenu sous la forme d'une huile.

**RMN ¹H :** δ (400 MHz ; dmso-d6 ; 300°K) : 7.12-7.0 (massif, 4H aromatiques) ; 3.92 (s, 2H tétrahydroisoquinoline) ; 3.60 (t, 4H morpholine) ; 2.98 (m, 1H tétrahydroisoquinoline) ; 2.68 (dd, 1H tétrahydroisoquinoline) ; 2.5-2.3 (massif, 8H, 1H tétrahydroisoquinoline, 6H morpholine, 1H NH)
**IR :** ν : >NH : 3322 cm⁻¹ ; ν : >C-O-C< : 1115 cm⁻¹; ν : >CH-Ar : 742 cm⁻¹

### Préparation 2' : (3R)-3-(4-Morpholinylméthyl)-1,2,3,4-tétrahydroisoquinoline

On procède selon le procédé de la Préparation 1' en remplaçant l'acide (3*S*)*-*2-[(benzyloxy)carbonyl]-1,2,3,4-tétrahydro-3-isoquinolinecarboxylique utilisé au Stade A par l'acide (3*R*)-2-[(benzyloxy)carbonyl]-1,2,3,4-tétrahydro-3-isoquinolinecarboxylique.

### Préparation 3' : (3S)-3-[(4-Méthyl-1-pipérazinyl)méthyl]-1,2,3,4-tétrahydroisoquinoline

On procède selon le procédé de la Préparation 1' en remplaçant la morpholine utilisée au Stade A par la 1-méthyl-pipérazine.

### Préparation 4': :(3S)-3-(1,4-Oxazépan-4-ylméthyl)-1,2,3,4-tétrahydroisoquinoline

On procède selon le procédé de la Préparation 1' en remplaçant la morpholine utilisée au Stade A par le 1,4-oxazépane.

### Réparation (3S)-3-{[(3R)-3-Méthylmorpholinyl]méthyl}-1,2,3,4-tétrahydroisoquinoline

On procède selon le procédé de la Préparation 1' en remplaçant la morpholine utilisée au Stade A par la (3*R*)-3-méthylmorpholine.

### Préparation 6' : (3S)-3-{[(3S)-3-Méthylmorpholinyl]méthyl}-1,2,3,4-tétrahydroisoquinoline

On procède selon le procédé de la Préparation 1' en remplaçant la morpholine utilisée au Stade A par la (3S)-3-méthylmorpholine.

### Préparation 7' : (3S)-3-{[(3S,5S)-3,5-Diméthylmorpholinyl]méthyl}-1,2,3,4-tétrahydroisoquinoline

On procède selon le procédé de la Préparation l'en remplaçant la morpholine utilisée au Stade A par la (3S,5S)-3,5-diméthylmorpholine.

### Préparation 8' : N,N-Diméthyl[(3S)-1,2,3,4-tétrahydro-3-isoquinolinyl]methanamine

On procède selon le procédé de la Préparation 1' en remplaçant la morpholine utilisée au Stade A par la *N,N*-diméthylamine.

### Préparation 9' : (3S)-3-{[4-(2-Méthoxyéthyl)piperazin-1-yl]méthyl}-1,2,3,4-tétrahydroisoquinoline

On procède selon le procédé de la Préparation 1' en remplaçant la morpholine utilisée au Stade A par la 1-(2-méthoxyéthyl)pipérazine.

### Préparation 10' : 1-Méthyl-4-[(3S)-1,2,3,4-tétrahydroisoquinolin-3-ylméthyl]piperazin-2-one

On procède selon le procédé de la Préparation 1' en remplaçant la morpholine utilisée au Stade A par la 1-méthylpiperazin-2-one.

### Préparation 11': 2-Méthoxy-N-méthyl-N-[(3S)-1,2,3,4-tétrahydroisoquinolin-3-ylméthyl]ethanamine

On procède selon le procédé de la Préparation 1' en remplaçant la morpholine utilisée au Stade A par la 2-méthoxy-*N*-méthylethanamine.

### Préparation 12 : -N-Éthyl-2-méthoxy-N-[(3S)-1,2,3,4-tétrahydroisoquinolin-3-ylméthyl]ethanamine

On procède selon le procédé de la Préparation 1' en remplaçant la morpholine utilisée au Stade A par la *N*-éthyl-2-méthoxyethanamine.

### Préparation 13' : (3S)-3-{[4-(Méthylsulfonyl)piperazin-1-yl]méthyl}-1,2,3,4-tétrahydroisoquinoline

On procède selon le procédé de la Préparation 1' en remplaçant la morpholine utilisée au Stade A par la 1-(méthylsulfonyl)pipérazine.

### Préparation 14' : (3S)-3-{[4-(2,2,2-Trifluoroéthyl)piperazin-1-yl]méthyl}-1,2,3,4-tétrahydroisoquinoline

On procède selon le procédé de la Préparation 1' en remplaçant la morpholine utilisée au Stade A par la 1-(2,2,2-trifluoroéthyl)pipérazine.

### Préparation 15' : (3S)-3-[(1,1-Dioxidothiomorpholin-4-yl)méthyl]-1,2,3,4-tétrahydroisoquinoline

On procède selon le procédé de la Préparation 1' en remplaçant la morpholine utilisée au Stade A par la thiomorpholine 1,1-dioxide.

### Préparation 16' : (3S)-3-[(3-Méthoxypyrrolidin-1-yl)méthyl]-1,2,3,4-tétrahydroisoquinoline

On procède selon le procédé de la Préparation 1' en remplaçant la morpholine utilisée au Stade A par la 3-méthoxypyrrolidine.

### Préparation_17' : (3S)-3-[(3,3-Difluoropyrrolidin-1-yl)methyl]-1,2,3,4-tétrahydroisoquinoline

On procède selon le procédé de la Préparation 1' en remplaçant la morpholine utilisée au Stade A par la 3,3-difluoropyrrolidine.

### Préparation 18' : (3S)-3-[(3-méthoxyazétidin-1-yl)méthyl]-1,2,3,4-tétrahydroisoquinoline

On procède selon le procédé de la Préparation 1' en remplaçant la morpholine utilisée au Stade A par la 3-méthoxyazétidine.

### Préparation 19' : (3S)-3-[(3-fluoroazétidin-1-yl)méthyl]-1,2,3,4-tétrahydroisoquinoline

On procède selon le procédé de la Préparation 1' en remplaçant la morpholine utilisée au Stade A par la 3-fluoroazétidine.

### Préparation 1" : 4-{[tert-Butyl(diméthyl)silyl]oxy}-N-phénylaniline

A une solution de 12 g de 4-anilinophénol (64,7 mmol) dans 200 mL d'acétonitrile sont ajoutés à température ambiante 6,7 g d'imidazole (97,05 mmol) et 11,7 g de tert-butyl(chloro)diméthylsilane (77,64 mmol). L'ensemble est mis sous agitation à 70°C pendant 4 heures. Puis, le milieu réactionnel est versé sur de l'eau et extrait avec de l'éther. La phase organique est ensuite séchée sur sulfate de magnésium, puis filtrée et évaporée à sec. Le produit brut ainsi obtenu est alors purifié par chromatographie sur gel de silice (gradient éther de pétrole / dichlorométhane). Le produit du titre est obtenu sous la forme d'une poudre.

**RMN¹H:** δ (400 MHz ; dmso-d6 ; 300°K) : 7.84 (s, 1H NH) ; 7.17 (t, 2H aniline) ; 6.98 (d, 2H phénoxy) ; 6.94 (d, 2H aniline) ; 6.76 (d, 2H phénoxy) ; 6.72(t, 1H aniline) ; 0.95 (s, 9H *tert*-butyl) 0.15 (s, 6H diméthyl)
**IR :** v : >NH : 3403 cm⁻¹ ;>Ar : 1597 cm⁻¹

Les amines NHR₃R₄ dans lesquelles R₃ et R₄ représentent indépendamment l'une de l'autre un groupement aryle ou hétéroaryle sont obtenues selon les procédés décrits dans la littérature (Surry D.S et al., Chemical Science, 2011, 2, 27-50, Charles M.D. et al., Organic Letters, 2005, 7, 3965-3968). La réaction de protection de la fonction hydroxy du 4-anilinophénol décrite à la Préparation 1" peut être appliquée à diverses amines secondaires NHR₃R₄ (telles que définies précédemment), comportant une ou plusieurs fonctions hydroxy, lorsque celles-ci sont disponibles commercialement. Alternativement, les amines secondaires comportant au moins un substituant hydroxy peuvent être synthétisées directement sous une forme protégée, i.e. à partir de réactifs dont la fonction hydroxy a été préalablement protégée. Parmi les groupements protecteurs, le *tert*-butylsilyloxy et le benzyloxy sont particulièrement préférés.
Parmi les amines NHR₃R₄ comportant un substituant hydroxy qui sont utilisées pour synthétiser les composés de l'invention, on peut citer : le 4-(4-toluidino)phénol, le 4-(4-chloroanilino)phénol, le 4-(3-fluoro-4-méthylanilino)phénol, le 4-[4-(trifluorométhoxy)anilino]phénol, le 4-[4-hydroxyanilino]phénol, le {4-[(1-méthyl-1*H-*indol-6-yl)amino]phényl}méthanol, le 4-(2,3-dihydro-1*H*-indol-6-ylamino)phénol, le 4-[(1-méthyl-2,3-dihydro-1*H*-indol-6-yl)amino]phénol, 4-[(1-méthyl-1*H*-indol-6-yl)amino]phénol, le 4-[(1-méthyl-1*H*-indol-6-yl)amino]cyclohexanol, le 4-[(1-méthyl-1,2,3,4-tétrahydro-6-quinolinyl)amino]phénol, le 4-[(4-méthyl-3,4-dihydro-2*H*-1,4-benzoxazin-7-yl)amino]phénol, le 4-[4-(diéthylamino)anilino]phénol, le 4-(2,3-dihydro-1*H*-indén-5-ylamino)phénol, le 4-[(1-méthyl-1*H*-indazol-5-yl)amino]phénol, le 4-((1'-méthyl-1',2'-dihydrospiro[cyclopropane-1,3'-indol]-5'-yl)amino]phénol, le 4-[(1,3,3-triméthyl-2,3-dihydro-1*H*-indol-5-yl) amino] phénol, le 4-[4-méthoxy-3-(trifluorométhyl)anilino]phénol, le 4-[4-(méthylsulfanyl)-3-(trifluorométhyl)anilino]phénol, le 2-fluoro-4-[(1-méthyl-1H-indol-5-yl)amino]phénol, le 4-[(1-éthyl-1*H*-indol-5-yl)amino]phénol, le 4-[(1-éthyl-2,3-dihydro-1*H*-indol-5-yl)amino]phénol, le 4-[(1-isopropyl-2,3-dihydro-1*H*-indol-5-yl)amino]phénol, le 4-(butylamino)phénol, le 3-[(1-méthyl-1*H*-indol-5-yl)amino]-1-propanol, le 4-[(1-méthyl-1*H*-indol-5-yl)amino]-1-butanol, le 4-[(3-fluoro-4-méthylphényl)amino]phénol, 4-[(3-chloro-4-méthylphényl)amino]phénol, 4-[(4-fluorophényl)amino]phénol, 4-[(1-méthyl-1*H-*pyrrolo[2,3-b]pyridin-5-yl)amino]phénol, 4-[(4-fluorophényl)amino]phénol, 4-[(2-fluorophényl)amino]phénol, 4-[(3-fluorophényl)amino]phénol, 4-[(2,4-difluorophényl) amino]phénol, 4-[(3,4-difluorophényl)amino]phénol, 3-[(4-hydroxyphényl)amino] benzonitrile, 4-[(3-méthoxyphényl)amino]phénol, 4-[(3,5-difluorophényl)amino]phénol, 4-[(3-méthylphényl)amino]phénol, 4-[(4-hydroxyphényl)amino]benzonitrile, 4-[(3-chlorophényl)amino]phénol, 4-(pyrimidin-2-ylamino)phénol, 4-[(cyclobutylméthyl) amino]phénol, 2-[(4-hydroxyphényl)amino]benzonitrile, 4-{[(1-méthyl-1H-pyrazol-4-yl)méthyl]amino}phénol, 4-[(cyclopropylméthyl)amino]phénol, 4-{[(1-méthyl-1*H-*pyrazol-3-yl)méthyl]amino}phénol, 4-(but-2-yn-1-ylamino)phénol, 4-(pyrazin-2-ylamino) phénol, 4-(pyridin-2-ylamino)phénol, 4-(pyridazin-3-ylamino)phénol, 4-(pyrimidin-5-ylamino)phénol, 4-(pyridin-3-ylamino)phénol, 4-[(3,5-difluoro-4-méthoxyphényl) amino]phénol, 4-(pyridin-4-ylamino)phénol, 4-[(3-fluoro-4-méthoxyphényl)amino] phénol, 2-(phénylamino)pyrimidin-5-ol, 5-[(4-hydroxyphényl)amino]-2-méthoxy benzonitrile, 4-{[3-(trifluorométhyl)phényl]amino}phénol.
La ou les fonction(s) hydroxy des amines secondaires listées ci-dessus est (sont) préalablement protégée(s) par un groupement protecteur adapté avant tout couplage à un dérivé d'acide du composé de formule (VII) tel que défini dans le procédé général précédent.

### Exemple 1 : Chlorhydrate de N-(4-hydroxyphényl)-3-{6-[((3S)-3-(4-morpholinyl méthyl)-3,4-dihydro-2(1H-isoquinolinyl)carbonyl]-1,3-benzodioxol-5-yl}-N-phényl-5,6,7,8-tétrahydro-1-indolizine carboxamide

### Stade A : 3-{6-[((3S)-3-(4-Morpholinylméthyl)-3,4-dihydro-2(1H)-isoquinolinyl) carbonyl]-1,3-benzodioxol-5-yl}-5,6,7,8-tétrahydro-1-indolizine carboxylate de méthyle,

A une solution de 2 g du composé de la Préparation 1 dans 20 mL de dichlorométhane, on ajoute à température ambiante 5,5 mL de *N,N,N*-triéthylamine (6,96 mmol), 2,12 g du composé de la Préparation 1' (6,96 mmol), puis 0,94 g d'hydroxybenzotriazole (HOBT) et 1,34 g d'1-éthyl-3-(3'-diméthylaminopropyl)-carbodiimide (EDC) (6,96 mmol). Le milieu réactionnel est ensuite agité à température ambiante pendant 1 nuit, puis il est versé sur une solution de chlorure d'ammonium et extrait avec de l'acétate d'éthyle. La phase organique est ensuite séchée sur sulfate de magnésium, puis filtrée et évaporée à sec. Le produit brut ainsi obtenu est alors purifié par chromatographie sur gel de silice (gradient heptane/ AcOEt). Le produit du titre est obtenu sous la forme d'une huile.

**RMN¹H :** δ (500 MHz ; dmso-d6 ; 300°K) : 7.2-6.9 (m, 4H, H aromatiques) ; 7.04-7.03-7.00 (m, 1H, H aromatique); 6.85 (m, 1H, H aromatique); 6.35-6.26-6.06 (m, 1H, H tétrahydroindolizine); 6.15-6.12 (m, 2H, H méthylènedioxy) ; 5.06-4.84 (m, 1H, H dihydroisoquinoline) ; 4.86-4.17 (m, 2H, H dihydroisoquinoline) ; 3.65-3.6-3.55 (m, 3H, H méthyl ester) ; 3.43-4.26 (m, 2H, H tétrahydroindolizine) ; 3.58-3.5 (m, 4H, H morpholine); 2.37-3.05 (m, 4H, 2H dihydroisoquinoline, 2H tétrahydroindolizine) ; 1.68-2.56 (m, 4H, H morpholine); 1.4-2.0 (m, 4H, H tétrahydroindolizine)
**IR :** v : >C=O 1695 cm⁻¹ ester ; v : >C=O 1625 cm⁻¹ amide ; v : >C-O-C< 1214-1176-1115 cm⁻¹ ; >CH-Ar 772-744 cm⁻¹

### Stade B: 3-[6-[(3S)-3-(Morpholinométhyl)-3,4-dihydro-1H-isoquinoline-2-carbonyl]-1,3-benzodioxol-5-yl]-5,6,7,8-tétrahydro-1-indolizine carboxylate de lithium

A une solution de 4,6 g du composé du Stade A (8,26 mmol) dans 24 mL de dioxane est ajoutée une solution d'hydroxyde de lithium (675 mg, 16,1 mmol). L'ensemble est placé dans un four à micro-ondes à 140W, 100°C pour une durée de 2h30. Le milieu réactionnel est ensuite filtré et évaporé. Le solide ainsi obtenu est séché à 40°C dans une étuve en présence de P₂O₅.

**RMN¹H :** δ (400 MHz ; dmso-d6 ; 353°K) : 6.7-7.15 (massif, 6H, H aromatiques); 6.21 (s, 1H, H aromatique) ; 6.03 (s, 2H,H méthylènedioxy); 4.0-5.0 (massif, 3H dihydroisoquinoline); 3.4-3.6 (massif, 3H tétrahydroindolizine, 3H morpholine); 2.5-3.1 (massif, 4H, 2H tétrahydroindolizine, 2H morpholine) ; 1.5-2.4 ( massif, 10H morpholine)
**IR :** v :>C=O 1567 large cm⁻¹ acétate ; v : 1236 cm⁻¹

### Stade C: N-(4-{[tert-Butyl(diméthyl)silyl]oxy}phényl)-3-{6-[((3S)-3-(4-morpholinylméthyl)-3,4-dihydro-2(1H)-isoquinolinyl)carbonyl]-1,3-benzodioxo-5-yl} -N-phényl-5,6,7,8-tétrahydro-1-indolizine carboxamide

A une solution de 2,6 g du composé du Stade B (4,73 mmol) dans 47 mL de dichlorométhane sont ajoutés, au goutte à goutte, 1,2 mL de chlorure d'oxalyle à 0°C. Le milieu réactionnel est agité à température ambiante pendant 11 heures, puis co-évaporé le plusieurs fois avec du dichlorométhane. Le produit ainsi obtenu est mis en suspension dans 37 mL de dichlorométhane, puis est additionné sur une solution de 2,1 g du composé obtenu à la Préparation 1" (7,1 mmol) dans 10 mL de dichlorométhane en présence de 0,6 mL de pyridine (7,1 mmol). L'ensemble est agité à température ambiante pendant une nuit. Le milieu réactionnel est concentré, purifié par chromatographie sur gel de silice (gradient dichlorométhane/méthanol). Le produit du titre est obtenu sous forme d'une mousse.

**RMN¹H:** δ (500MHz; dmso-d6 ; 300°K) : 6.9-7.3 (9H aromatiques);6.88 (2H aromatiques); 6.72-6.87 (2H aromatiques); 6.64 (2H aromatiques); 6.13 (2H méthylènedioxy) ; 5.05-4.74(1H dihydroisoquinoline) ; 4.25-4.13 (2H dihydroisoquinoline); 3.44-3.7 (4H morpholine); 3.62-3.52 (2H tétrahydroindolizine) ; 3.0-2.6 (4H, 2H tétrahydroindolizine, 2H dihydroisoquinoline); 2.54-1.94 (6H morpholine); 1.91-1.53 (4H tétrahydroindolizine); 0.92 (9H *tert*-butyl); 0.17 (6H diméthyl)
**IR :** v :>C=O : 1632 cm⁻¹ ; v : >C-O-C< : 1237 cm⁻¹ ; v : -Si-O-C- : 1035 cm⁻¹ ; -Si-C- : 910 cm⁻¹, >CH-Ar : 806 cm⁻¹

### Stade D: Chlorhydrate de N-(4-hydroxyphényl)-3-{6[((3S)-3-(4-morpholinylméthyl)-3,4-dihydro-2(1H)-isoquinolinyl)carbonyl]-1,3-benzodioxol-5-yl}-N-phénylyl-5,6,7,8-tétrahydro-1-indolizine carboxamide

A une solution de 1,9 g du composé obtenu au Stade C (2,3 mmol) dans 4 mL de méthanol, on ajoute 0,646 g (11,5 mmol) d'hydroxyde de potassium solubilisé dans 8 mL de méthanol. L'ensemble est agité à température ambiante pendant 30 min. Le milieu réactionnel est ensuite dilué dans le dichlorométhane et lavé successivement avec une solution HCl 1N, une solution de NaHCO₃ saturée, puis une solution de NaCl saturée jusqu'à atteindre un pH neutre. La phase organique est ensuite séchée sur sulfate de magnésium, filtrée et évaporée. Le produit brut ainsi obtenu est purifié sur gel de silice (gradient dichlorométhane/méthanol). Le solide est ensuite solubilisé dans le dichlorométhane et 2 mL d'éther chlorhydrique 1N sont ajoutés. Le tout est agité pendant 1 heure, puis évaporé à sec. Le chlorhydrate ainsi obtenu est dissout dans un mélange eau / acétonitrile jusqu'à solubilisation totale, puis est lyophilisé.

| ***Microanalyse élémentaire:*** | | | | |
|---|---|---|---|---|
| | %*C* | %*H* | *%N* | *%Cl⁻* |
| *Calculé* | *69.11* | *5.8* | *7.5* | *4*.*74* |
| *Trouvé* | *68.95* | *5.46* | *7*.*51* | *4.48* |

***Pouvoir rotatoire :*** (α) _{D}²⁰ = + 50.8° (c = 9 mg/mL, MeOH)

### Exemple 2 : Chlorhydrate de N-(4-hydroxyphényl)-N-(4-méthylphényl)-3-{6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant le composé de la Préparation 1" utilisé au Stade C par le 4-{[*tert*-butyl(diméthyl)silyl]oxy}-*N*-(4-méthylphényl)aniline.

| ***Microanalyse élémeiitaire :*** | | | | | |
|---|---|---|---|---|---|
| | %C | *%H* | *%N* | *%Cl* | *%Cl⁻* |
| *Calculé* | *69.42* | *5.96* | *7.36* | *4*.*66* | *4*.*66* |
| *Trouvé* | *69.19* | *5.76* | *7.19* | *4.62* | *4.48* |

### Exemple 3 : Chlorhydrate de N-(4-chlorophényl)-N-(4-hydroxyphényl)-3-{6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant le composé de la Préparation 1" utilisé au Stade C par le 4-{[*tert*-butyl(diméthyl)silyl]oxy}-*N*-(4-chlorophényl)aniline.

| ***Microanalyse élémentaire:*** | | | | | |
|---|---|---|---|---|---|
| | %*C* | *%H* | *%N* | *%Cl* | *%Cl⁻* |
| *Calculé* | *66.07* | *5.42* | *7.17* | *9. 07* | *4.54* |
| *Trouvé* | *65.74* | *5.14* | *7.08* | *9.02* | *4.48* |

***Pourvoir rotatoire** :* (α) _{D}²⁰ = + 80.9° (c = 2.5 mg/mL, MeOH)

### Exemple 4 : Chlorhydrate de N-(3-fluoro-4-méthylphényl)-N-(4-hydroxyphényl)-3-{6-[((3S)-3-(4-morpholinylméthym)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant le composé de la Préparation 1" utilisé au Stade C par le 4-{[*tert*-butyl(diméthyl)silyl]oxy}-*N*-(3-fluoro-4-méthylphényl)aniline.

### Masse haute résolution (ESI+) :

Formule brute : C₄₄H₄₃FN₄O₆
[M+H]⁺ calculé : 743.3245
[M+H]⁺ mesuré: 743.3250

***Pouvoir rogatoire :*** (α) _{D}²⁰ = + 40.7° (c = 2.5 mg/mL, MeOH)

### Exemple 5 : Chlorhydrate de N-(4-hydroxyphényl)-3-{6-[((3S)-3-(4-morpholinyl méthyl)-3,4-dihydro-2(1H)-isoquinolinyl)carbonyl]-1,3-benzodioxol-5-yl}-N-[4-(trifluorométhoxy)phényl]-5,6,7,8-tétrahydro-1-indolizine carboxamide

On procède selon le procédé de l'Exemple 1 en remplaçant le composé de la Préparation 1" utilisé au Stade C par le 4-{[*tert*-butyl(diméthyl)silyl]oxy}-*N*-(4-trifluorométhoxyphényl)aniline.

| ***Microanalyse élémentaire :*** | | | | |
|---|---|---|---|---|
| | *%C* | *%H* | *%N* | *%Cl-* |
| *Calculé* | *63.57* | *5.09* | *6.74* | *4.26* |
| *Trouvé* | *63.38* | *4.95* | *6.88* | *4.23* |

### Exemple 6 : Chlorhydrate de N,N-bis(4-hydroxyphényl)-3-{6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant le composé de la Préparation 1" utilisé au Stade C par le *4-*{[*tert*-butyl(diméthyl)silyl]oxy}-*N*-(4-{[*tert-*butyl(diméthyl)silyl]oxy}phényl)aniline.

| ***Microallalyse élémentaire :*** | | | | |
|---|---|---|---|---|
| | *%C* | *%H* | *%N* | *%Cl-* |
| *Calculé* | *67.66* | *5.68* | *7.34* | *4.64* |
| *Trouvé* | *67.11* | *5.49* | *7.31* | *4.74* |

***Pouvoir rotatoire :*** (α)_{D}²⁰ = + 50.8° (c = 6 mg/mL, MeOH)

### Exemple 7 : Chlorhydrate de N-[4-(hydroxyméthyl)phényl]-N-(1-méthyl-1H-indol-5-yl)-3-{6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant le composé de la Préparation 1" utilisé au Stade C par le *N*-[4-({[*tert*-butyl(diméthyl)silyl]oxy}méthyl)phényl]-1-méthyl-1*H*-indol-5-amine.

| ***Microanalyse élémentaire:*** | | | | |
|---|---|---|---|---|
| | %*C* | *%H* | *%N* | *%Cl⁻* |
| *Calculé* | *69.32* | *5.94* | *8.6* | *4.35* |
| *Trouvé* | *69.63* | *5.75* | *8.59* | *4.13* |

### Exemple 8 : Bis(chlorhydrate) de N-(2,3-dihydro-1H-indol-5-yl)- N-[4-(hydroxy)phényl]-3-{6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant le composé de la Préparation 1 " utilisé au Stade C par le *N*-(4-{[*tert*-butyl(diméthyl)silyl]oxy}phényl)-5-indolinamine.

| ***Microanalyse élémentaire :*** | | | | |
|---|---|---|---|---|
| | %*C* | *%H* | *%N* | *%Cl* |
| *Calculé* | *65.53* | *5*.*74* | *8.49* | *8.60* |
| *Trouvé* | *65.47* | *5.69* | *8.43* | *7.82* |

***Pouvoir rotatoire :*** (α) _{D}²⁰ = + 40.9° (c = 3.5 mg/mL, MeOH)

### Exemple 9 : Chlorhydrate de N-[4-(hydroxy)phényl]-N-(1-méthyl-2,3-dihydro-1H-indol-5-yl)-3-{6-[{(3S)-3-(4-morpholinylméthyl)-3,4-dihydro-2(1H)-isoquinolinyl) carbonyl]-1,3-benzodioxol-5-yl}-5,6,7,8-tétrahydro-1-indolizine carboxamide

On procède selon le procédé de l'Exemple 1 en remplaçant le composé de la Préparation 1" utilisé au Stade C par le *N*-{4-{[*tert*-butyl(diméthyl)silyl]oxy}phényl)-1-méthyl-5-indolinamine.

### Masse haute résolution (ESI+) :

Formule brute : C₄₆H₄₇N₅O₆
[M+H]⁺ calculé : 766.3605
[M+H]⁺ mesuré : 766.3601

### Exemple 10 : Chlorhydrate de N-[4-(hydroxy)phényl]-N-(1-méthyl-1H-indol-5-yl)-3-{6-[((3S)-3-(4-morpholinylméthyl)-3,4-dihydro-2(1H)-isoquinolinyl)carbonyl]-1,3-benzodioxol-5-yl]-5,6,7,8-tétrahydro-1-indolizine carboxamide

On procède selon le procédé de l'Exemple 1 en remplaçant le composé de la Préparation 1" utilisé au Stade C par le *N*-(4-{[*tert*-butyl(diméthyl)silyl]oxylphényl)-1-méthyl-1*H-*indol-5-amine.

| ***Microanalyse élémentaire :*** (théorie pour 1.1 HCl) | | | | |
|---|---|---|---|---|
| | *%C* | *%H* | *%N* | *%Cl⁻* |
| *Calculé* | *68.72* | *5*.*78* | *8.71* | *4.85* |
| *Trouvé* | *68.39* | *5.55* | *8.66* | *4.33* |

***Pouvoir rotatoire:*** (α) _{D}²⁰ = + 37.6° (c = 7 mg/mL, MeOH)

### Exemple 11 : Chlorhydrate de N-(4-hydroxyphényl)-3-{6-[((3R)-3-(4-morpholinyl méthyl)-3,4-dihydro-2(1H)-isoquinolinyl)carbonyl]-1,3-benzodioxol-5-yl}-N-phényl-5,6,7,8-tétrahydro-1-indolizine carboxamide

On procéde selon le procédé de l'Exemple 1 en remplaçant le composé de la Préparation 1' utilisé au Stade A par le composé de la Préparation 2'.

| ***Microanalyse élémentaire :*** | | | | |
|---|---|---|---|---|
| | *%C* | *%H* | *%N* | *%Cl⁻* |
| *Calculé* | *69.11* | *5.80* | *7.50* | *4.74* |
| *Trouvé* | *68.89* | *5.23* | *7.41* | *4.62* |

***Pouvoir rotatoire** :* (α)_{D}²⁰ = - 45.1° (c = 9 mg/mL, MeOH)

### Exemple 12 : Chlorhydrate de N-(4-hydroxycyclohexyl)-N-(1-méthyl-1H-indol-5-yl)-3-{6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant le composé de la Préparation 1" utilisé au Stade C par le *N*-(4-{[*tert*-butyl(diméthyl)silyl]oxy}cyclohexyl)-1-méthyl-1*H*-indol-5-amine.

### Masse haute résolution (ESI+) :

Formule brute : C₄₆H₅₁N₅O₆
[M+H]⁺ calculé : 770.3918
[M+H]⁺ mesuré : 770.3928

### Exemple 13 Bis(chlorhydrate) de N-(4-hydroxyphényl)-N-(1-méthyl-1H-indol-5-yl)-3-{6-[((3S)-3-[(4-méthyl-1-pipérazinyl)méthyl]-3,4-dihydro-2(1H)-isoquinolinyl) carbonyl]-1,3-benzodioxol-5-yl}-5,6,7,8-tétrahydro-1-indolizine carboxamide

On procède selon le procédé de l'Exemple 1 en remplaçant d'une part le composé de la Préparation l' utilisé au Stade A par le composé de la Préparation 3', et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | | |
|---|---|---|---|---|---|
| | %*C* | *%H* | *%N* | *%Cl* | *%Cl⁻* |
| *Calculé* | *66.43* | *5.93* | *9.89* | *8*.*34* | *8.34* |
| *Trouvé* | *66.36* | *5.79* | *9.83* | *8*.*11* | *7. 67* |

***Pouvoir rotatoire** :* (α) _{D}²⁰ = + 60.1° (c = 6 mg/mL, MeOH)

### Exemple 14 : Chlorhydrate de N-(4-hydroxyphényl)-N-(1-méthyl-1H-indol-5-yl)-3-{6-[((3S)-3-(1,4-oxazépan-4-ylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant d'une part le composé de la Préparation l' utilisé au Stade A par le composé de la Préparation 4', et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | | |
|---|---|---|---|---|---|
| | %*C* | *%H* | %*N* | *%Cl* | *%CL* |
| *Calculé* | *69.32* | *5.94* | *8.60* | *4*.*35* | *4.35* |
| *Trouvé* | *69.24* | *5.56* | *8.52* | *4.47* | *4.44* |

### Exemple 15 : Chlorhydrate de N-(4-hydroxyphényl)-N-(1-méthyl-1H-indol-5-yl)-3-{6-[((3S)-3-{[(3R)-3-méthylmorpholinyl]méthyl}-3,4-dihydro-2(1H)-isoquinolinyl) carbonyl]-1,3-benzodioxol-5-yl}-5,6,7,8-tétrahydro-1-indolizine carboxamide

On procède selon le procédé de l'Exemple 1 en remplaçant d'une part le composé de la Préparation l' utilisé au Stade A par le composé de la Préparation 5', et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | %*C* | *%H* | *%N* | *%Cl⁻* |
| *Calculé* | *69.32* | *5.94* | *8.60* | *4.35* |
| *Trouvé* | *69.10* | *5.68* | *8.52* | *4. 29* |

### Exemple 16 : Chlorhydrate de N-(4-hydroxyphényl)-N-(1-méthyl-1H-indol-5-yl)-3-{6-[((3S)-3-{[(3S)-3-méthylmorpholinyl]méthyl}-3,4-dihydro-2(1H)-isoquinolinyl) carbonyl]-1,3-benzodioxol-5-yl}-5,6,7,8-tétrahydro-1-indolizine carboxamide

On procède selon le procédé de l'Exemple 1 en remplaçant d'une part le composé de la Préparation l' utilisé au Stade A par le composé de la Préparation 6', et d'autre part le composé de la Préparation 1" utilisé au Stade C par le *N*-(4-{[*tert-*butyl(diméthyl)silyl]oxy]phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *69.32* | *5.94* | *8.60* | *4.35* |
| *Trouvé* | *68.97* | *5.55* | *8*.*44* | *3.73* |

### Exemple 17 : Chlorhydrate de 3-{6-[((3S)-3-{[(3S,5S)-3,5-diméthylmorpholinyl] méthyl}-3,4-dihydro-2(1H)-isoquinolinyl)carbonyl]-1,3-benzodioxol-5-yl}-N-(4-hydroxyphcnyl)-N-(1-méthyl-1H-indol-5-yl)-5,6,7,8-tétrahydro-1-indolizine carboxamide

On procède selon le procédé de l'Exemple 1 en remplaçant d'une part le composé de la Préparation l'utilisé au Stade A par le composé de la Préparation 7', et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *69*.*59* | *6.08* | *8.45* | *4.28* |
| *Trouvé* | *69.29* | *5.52* | *8.46* | *4.09* |

### Exemple 18 : Chlorhydrate de 3-{5-bromo-2-[((3S)-3-(4-morpholinylméthyl)-3,4-dihydro-2(1H)-isoquinolinyl)carbonyl)phényl}-N-{4-hydroxyphényl)-N-(1-méthyl-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 2, et d'autre part le composé de la Préparation 1" utilisé au Stade C par le *N*-(4-{[*tert-*butyl(diméthyl)silyl]oxy}phényl)-1-méthyl-1*H*-indol-5-amine.

### Masse haute résolution (ESI+) :

Formule brute : C₄₅H₄₄⁷⁹BrN₅O₄
[M+H]⁺ calculé : 798.2655
[M+H]⁺ mesuré : 798.2626

### Exemple 19 : Chlorhydrate de 3-{5-bromo-2-[((3S)-3-(4-morpholinylméthyl)-3,4-dihydro-2(1H)-isoquinolinyl)carbonyl]phényl}-N-(4-hydroxyphényl)-N-(1-méthyl-2,3-dihydro-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 2, et d'autre part le composé de la Préparation 1" utilisé au Stade C par le *N*-(4-{[*tert-*butyl(diméthyl)silyl]oxy}phényl)-1-méthyl-5-indolinamine.

### Masse haute résolution (ESI+) :

Fomule brute : C₄₅H₄₆⁷⁹BrN₅O₄
[M+H]⁺ calculé: 800.2811
[M+H]⁺ mesuré : 800.2791

### Exemple 20 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-phényl-5,6,7,8-tétrahydro-1-indolizine carboxamide

On procède selon le procédé de l'Exemple 1 en remplaçant le composé de la Préparation 1 utilisé au Stade A par le composé de la Préparation 3.

| ***Microanalyse élémentaire :*** | | | | |
|---|---|---|---|---|
| | *%C* | *%H* | *%N* | *%Cl⁻* |
| *Calculé* | *68.38* | *5.74* | *7.59* | *4.81* |
| *Trouvé* | *67.89* | *5.71* | *7.72* | *4.68* |

***Pouvoir rotatoire :*** (α) _{D}²⁰ = + 53.7° (c = 6 mg/mL, MeOH)

### Exemple 21 : Bis(chlorhydrate) de 3-{5-chloro-2-[((3S)-3-(4-morpholinylméthyl)-3,4-dihydro-2(1H)-isoquinolinyl)carbonyl]phényl}-N-(2,3-dihydro-1H-indol-5-yl)-N-(4-hydroxyphényl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4*-*{[*tert-*butyl(diméthyl)silyl]oxy}phényl)-5-indolinamine.

### Masse haute résolution (ESI+) :

Formule brute : C₄₄H₄₄³⁵ClN₅O₄
[M+H]⁺ calculé: 742.3160
[M+H]⁺ mesuré : 742.3120

### Exemple 22 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-(1-méthyl-1H-indol-5-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *68*.*04* | *5.72* | *8.82* | *4.91* |
| *Trouvé* | *67.84* | *5.46* | *8.64* | *5.21* |

***Pouvoir rotatoire** :* (α)_{D}²⁰ = + 55.9° (c = 7 mg/mL, MeOH)

### Exemple 23 : Bis(chlorhydrate) de 3-{5-chloro-2-[((3S)-3-(4-morpholinylméthyl)-3,4-dihydro-2(1H)-isoquinolinyl)carbonyl]phényl}-N-(4-hydroxyphényl)-N-(1-méthyl-2,3-dihydro-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-1-méthyl-5-indolinamine.

| ***Microanalyse élémentaire :*** | | | | | |
|---|---|---|---|---|---|
| | *%C* | *%H* | *%N* | *%Cl* | *%Cl⁻* |
| *Calculé* | *65.18* | *5.83* | *8.45* | *12.83* | *8.55* |
| *Trouvé* | *65.75* | *5.45* | *8.39* | *11.71* | *7.54* |

***Pouvoir rogatoire:*** (α)_{D}²⁰ = + 56.6° (c = 6 mg/mL, MeOH)

### Exemple 24 : Chlorhydrate de 3-{5-fluoro-2-[((3S)-3-(4-morpholinylméthyl)-3,4-dihydro-2(1H)-isoquinolinyl)carbonyl]phényl}-N-(4-hydroxyphényl)-N-(1-méthyl-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 4, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | %C | %*H* | %*N* | *%Cl⁻* |
| *Calculé* | *69.80* | *5.86* | *9*.*04* | *4.58* |
| *Trouvé* | *69.57* | *5.62* | *8.76* | *4.47* |

***Pouvoir rotatoire** :* (α)_{D}²⁰= + 55.0° (c = 5 mg/mL, MeOH)

### Exemple 25 : Chlorhydrate de 3-{5-fluoro-2-[((3S)-3-(4-morpholinylméthyl)-3,4-dihydro-2(1H)-isoquinolinyl)carbonyl]phényl}-N-(4-hydroxyphényl)-N-phényl-5,6,7,8-tétrahydro-1-indolizine carboxamide

On procède selon le procédé de l'Exemple 1 en remplaçant le composé de la Préparation 1 utilisé au Stade A par le composé de la Préparation 4.

| ***Microanalyse élémentaire:*** | | | | |
|---|---|---|---|---|
| | *%C* | *%H* | *%N* | *%Cl⁻* |
| *Calculé* | *69.94* | *5.87* | *7*.*77* | *4.92* |
| *Trouvé* | *69.70* | *5.32* | *7*.*72* | *4.80* |

### Exemple 26 : Chlorhydrate de 3-{5-chloro-2-{((3S)-3-(4-morpholinylméthyl)-3,4-dihydro-2(1H)-isoquinolinyl)carbonyl]phényl}-N-(4-hydroxyphényl)-N-(1-méthyl-1,2,3,4-tétrahydro-6-quinolinyl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-1-méthyl-1,2,3,4-tétrahydro-6-quinolinamine.

| ***Microanalyse élémentaire :*** (théorie pour 1.5 HCl) | | | | |
|---|---|---|---|---|
| | %*C* | %*H* | *%N* | *%Cl⁻* |
| *Calculé* | *66*.*97* | *6.05* | *8*.*49* | *6*.*45* |
| *Trouvé* | *66.80* | *5*.*55* | *8.32* | *6.23* |

### Exemple 27 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-(4-méthyl-3,4-dihydro-2H-1,4-benzoxazin-7-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-4-méthyl-3,4-dihydro-2*H*-1,4-benzoxazin-7-amine.

| ***Microanalyse élémentaire :*** (théorie pour 1.1 HCl) | | | | |
|---|---|---|---|---|
| | %*C* | *%H* | *%N* | *%Cl⁻* |
| *Calculé* | *66.53* | *5.84* | *8.62* | *4.80* |
| *Trouvé* | *66.59* | *5.39* | *8.47* | *4.80* |

### Exemple 28 : Chlorhydrate de 3-{5-chloro-2-[((3S)-3-(4-morpholinylméthyl)-3,4-dihydro-2(1H)-isoquinolinyl)carbonyl]phényl}-N-[4-(diéthylamino)phényl]-N-(4-hydroxyphényl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*¹-(4-{[*tert-*butyl(diméthyl)silyl]oxy}phényl)-*N*⁴,*N*⁴-diéthyl-1,4-benzenediamine.

| ***Microanalyse élémentaire :*** (théorie pour 1.6 HCl) | | | | |
|---|---|---|---|---|
| | %*C* | *%H* | *%N* | *%Cl⁻* |
| *Calculé* | *66.51* | *6*.*26* | *8.43* | *6.83* |
| *Trouvé* | *66.71* | *5.58* | *8.39* | *7.08* |

### Exemple 29 : Chlorhydrate de 3-{5-chloro-2-[((3S)-3-(4-morpholinylméthyl)-3,4-dihydro-2(1H)-isoquinolinyl)carbonyl]phényl}-N-(2,3-dihydro-1H-indén-5-yl)-N-(4-hydroxyphényl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-5-indanamine.

| ***Microanalyse élémentaire :*** | | | | |
|---|---|---|---|---|
| | %C | %*H* | *%N* | *%Cl⁻* |
| *Calculé* | *69.49* | *5.96* | *7.20* | *4.56* |
| *Trouvé* | *69*.*47* | *5.43* | *7.21* | *4.21* |

### Exemple 30 : Bis(chlorhydrate) de 3-{5-chloro-2-[((3S)-3-[(4-méthyl-1-pipérazinyl)méthyl]-3,4-dihydro-2(1H)-isoquinolinyl)carbonyl]phényl}-N-(4-hydroxyphényl)-N-(1-méthyl-1H-indol-5-yl)-5,6,7,8-tétrahydro-1-indolizine carboxamide

On procède selon le procédé de l'Exemple 1 en remplaçant d'une part les composés des Préparations 1 et l'utilisés au Stade A par les composés respectifs des Préparations 3 et 3', et d'autre part le composé de la Préparation 1" utilisé au Stade C par le *N*-(4-{[*tert-*butyl(diméthyl)silyl]oxy}phényl)-1-méthyl-1*H*-indol-5-amine.

### Masse haute résolution (ESI+) :

Formule brute : C₄₆H₄₇³⁵ClN₆O₃
[M+H]⁺ calculé : 767.3476
[M+H]⁺ mesuré : 767.3476

### Exemple 31 : Chlorhydrate de N-(4-hydroxyphényl)-3-{8-[((3S)-3-(4-morpholinylméthyl)-3,4-dihydro-2(1H)-isoquinolinyl)carbonyl]-3,4-dihydro-2H-1,5-benzodioxepin-7-yl}-N-phényl-5,6,7,8-tétrahydro-1-indolizine carboxamide

On procède selon le procédé de l'Exemple 1 en remplaçant le composé de la Préparation 1 utilisé au Stade A par le composé de la Préparation 5.

### Masse haute résolution (ESI+) :

Formule brute : C₄₅H₄₆N₄O₆
[M+H]⁺ calculé : 739.3496
[M+H]⁺ mesuré : 739.3479

### Exemple 32 : Chlorhydrate de N-(4-hydroxyphényl)-N-(1-méthyl-1H-indol-5-yl)-3-{8-[((3S)-3-(4-morpholinylméthyl)-3,4-dihydro-2(1H)-isoquinolinyl)carbonyl]-3,4-dihydro-2H-1,5-benzodioxepin-7-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 5, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | %*C* | *%H* | *%N* | *%Cl⁻* |
| *Calculé* | *69*.*59* | *6*.*08* | *8.45* | *4.78* |
| *Trouvé* | *69.23* | *5.46* | *8.41* | *4*.*16* |

### Exemple 33: Chlorhydrate de N-(4-hydroxyphényl)-3-{5-méthoxy-2-[((3S)-3-(4-morpholinylméthyl)-3,4-dihydro-2(1H)-isoquinolinyl)carbonyl}phényl}-N-(1-méthyl-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 6, et d'autre part le composé de la Préparation 1" utilisé au Stade C par le *N*-(4-{[*tert-*butyl(diméthyl)silyl}oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *70.26* | *6.15* | *8.91* | *4.51* |
| *Trouvé* | *69.78* | *5.36* | *8.90* | 4.29 |

***Pouvoir rotatoire** :* (α) _{D}²⁰ = + 42.1° (c = 6 mg/mL, MeOH)

### Exemple 34 : Chlorhydrate de N-(4-hydroxyphényl)-3-{7-[((3S)-3-(4-morpholinylméthyl)-3,4-dihydro-2(1H)-isoquinolinyl)carbonyl]-2,3-dihydro-1,4-benzodioxin-6-yl}-N-phényl-5,6,7,8-tétrahydro-1-indolizine carboxamide

On procède selon le procédé de l'Exemple 1 en remplaçant le composé de la Préparation 1 utilisé au Stade A par le composé de la Préparation 7.

| ***Microanalyse élémentaire:*** | | | | |
|---|---|---|---|---|
| | %*C* | *%H* | *%N* | *%Cl⁻* |
| *Calculé* | *69.42* | *5.96* | *7.36* | *4.66* |
| *Trouvé* | *69.39* | *5.56* | *7.30* | *4.49* |

***Pouvoir rotatoire:*** (α)_{D}²⁰= + 34.5° (c = 6 mg/mL, MeOH)

### Exemple 35 : N-(4-hydroxyphényl)-N-(1-méthyl-1H-indol-5-yl)-3-{7-[((3S)-3-(4-morpholinylméthyl)-3,4-dihydro-2(1H)-isoquinolinyl)carbonyl]-2,3-dihydro-1,4-benzodioxin-6-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 7, et d'autre part le composé de la Préparation 1" utilisé au Stade C par le *N*-(4-{[*tert-*butyl(diméthyl)silyl]oxy}phényl)-1-méthyl-1*H*-indol-5-amine, étant entendu que le produit ainsi obtenu n'est pas soumis à une étape de salification en présence d'éther chlorhydrique.

| ***Microanalyse élémentaire :*** | | | |
|---|---|---|---|
| | %*C* | *%H* | *%N* |
| *Calculé* | *72.57* | *6.09* | *9.00* |
| *Trouvé* | *73.11* | *5.70* | *8.95* |

***Pouvoir rotatoire** :* (α) _{D}²⁰ = + 88.2° (c = 7 mg/mL, MeOH)

### Exemple 36: Chlorhydrate de 3-{5-éthoxy-2-[((3S)-3-(4-morpholinylméthyl)-3,4-dihydro-2(1H)-isoquinolinyl)carbonyl]phényl}-N-(4-hydroxyphényl)-N-(1-méthyl-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 8, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | %*N* | *%Cl* |
| *Calculé* | *70.53* | *6.30* | *8*.*75* | *4.43* |
| *Trouvé* | *70.77* | *5.59* | *8.66* | *4.22* |

### Exemple 37 : Chlorhydrate de N-(4-hydroxyphényl)-N-(1-méthyl-1H-indol-5-yl)-3-{2,2-dideutério-6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 9, et d'autre part le composé de la Préparation 1" utilisé au Stade C par le *N*-(4-{[*tert-*butyl(diméthyl)silyl]oxy}phényl)-1-méthyl-1*H*-indol-5-amine.

### Masse haute résolution (ESI+) :

Formule brute : C₄₆H₄₃N₅O₆D₂
[M+H]⁺ calculé : 766.3573
[M+H]⁺ mesuré : 766.3533

***Pouvoir rotatoire :*** (α)_{D}²⁰= + 35.5° (c = 3.5 mg/mL, MeOH)

### Exemple 38 : Chlorhydrate de N-(4-hydroxyphényl)-3-{2,2-dideutério-6-[((3S)-3-(4-morpholinylméthyl)-3,4-dihydro-2(1H)-isoquinolinyl)carbonyl]-1,3-benzodioxol-5-yl}-N-phényl-5,6,7,8-tétrahydro-1-indolizine carboxamide

### Stade A : N-(4-hydroxyphényl)-3-{2,2-dideutério-6-[((3S)-3-(4-morpholinylméthyl)-3,4-dihydro-2(1H)-isoquinolinyl)carbonyl]-1,3-benzodioxol-5-yl}-N-phényl-5,6,7,8-tétrahydro-1-indolizine carboxamide

On procède selon le procédé de l'Exemple 1 en remplaçant le composé de la Préparation 1 utilisé au Stade A par le composé de la Préparation 9, étant entendu que l'étape de salification en présence d'éther chlorhydrique n'est réalisée qu'au Stade B suivant.

### Stade B : Chlorhydrate de N-(4-hydroxyphényl)-3-{2,2-dideutério-6-[((3S)-3-(4-morpholinylméthyl)-3,4-dihydro-2(1H)-isoquinolinyl)carbonyl]-1,3-benzodioxol-5-yl}-N-phényl-5,6,7,8-tétrahydro-1-indolizine carboxamide

Le produit obtenu au Stade A est solubilisé dans le dichlorométhane et 2 mL d'éther chlorhydrique 1N sont ajoutés. Le tout est agité pendant 1 heure, puis évaporé à sec. Le chlorhydrate ainsi obtenu est dissout dans un mélange eau / acétonitrile jusqu'à solubilisation totale, puis est lyophilisé.

| ***Microanalyse élémentaire:*** | | | | | |
|---|---|---|---|---|---|
| | %*C* | %*H* | %*N* | *%Cl* | *%Cl⁻* |
| *Calculé* | *68.93* | *5.80* | *7*.*48* | *4.73* | *4.73* |
| *Trouvé* | *68.45* | *5.49* | *7.57* | *4.63* | *4.54* |

### Exemple 39 : Chlorhydrate de N-(4-hydroxyphényl)-N-(1-méthyl-1H-indazol-5-yl)-3-{7-[((3S)-3-(4-morpholinylméthyl)-3,4-dihydro-2(1H)-isoquinolinyl)carbonyl]-2,3-dihydro-1,4-benzodioxin-6-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 7, et d'autre part le composé de la Préparation 1" utilisé au Stade C par le *N-(4-{[tert-*butyl(diméthyl)silyl]oxy}phényl)-1-méthyl-1*H*-indazol-5-amine.

| ***Microanalyse élémentaire:*** | | | | |
|---|---|---|---|---|
| | %*C* | *%H* | *%N* | *%Cl⁻* |
| *Calculé* | *67.76* | *5.81* | *10.31* | *4.35* |
| *Trouvé* | *67.82* | *5.25* | *9.87* | *4.18* |

***Pouvoir rotatoire :*** (α)_{D}²⁰ = + 42.6° (c = 5 mg/mL, MeOH)

### Exemple 40 : Chlorhydrate de N-(4-hydroxyphényl)-N-(1-méthyl-1,2,3,4-tétrahydro-6-quinolinyl)-3-{7-[((3S)-3-(4-morpholinylméthyl)-3,4-dihydro-2(1H)-isoquinolinyl)carbonyl]-2,3-dihydro-1,4-benzodioxin-6-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 7, et d'autre part le composé de la Préparation 1" utilisé au Stade C par le *N*-(4-{[*tert-*butyl(diméthyl)silyl]oxy}phényl)-1-méthyl-1,2,3,4-tétrahydro-6-quinolinamine.

| ***Microanalyse élémentaire :*** | | | | | |
|---|---|---|---|---|---|
| | %*C* | *%H* | *%N* | *%Cl* | *%Cl⁻* |
| *Calculé* | *66.51* | *6*.*16* | *8.08* | *8.18* | *8.18* |
| *Trouvé* | *66.85* | *5.88* | *8.04* | *6.47* | *6.25* |

### Exemple 41 : Chlorhydrate de N-(4-hydroxyphényl)-N-(4--méthyl-3,4-dihydro-2H-1,4-benzoxazin-7-yl)-3-{7-[((3S)-3-(4-morpholinylméthyl)-3,4-dihydro-2(1H)-isoquinolinyl)carbonyl]-2,3-dihydro-1,4-benzodioxin-6-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 7, et d'autre part le composé de la Préparation 1" utilisé au Stade C par le *N*-(4-{[*tert-*butyl(diméthyl)silyl]oxy}phényl)-4-méthyl-3,4-dihydro-2*H*-1,4-benzoxazin-7-amine.

### Masse haute résolution (ESI+) :

Formule brute : C₄₇H₄₉N₅O₇
[M+H]⁺ calculé : 796.3710
[M+H]⁺ mesuré : 796.3687

### Exemple 42: Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-(1-méthyl-1H-indazol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-1-méthyl-1*H*-indazol-5-amine.

| ***Microanalyse élémentaire :*** | | | | | |
|---|---|---|---|---|---|
| | %*C* | *%H* | *%N* | *%Cl* | %*Cl⁻* |
| *Calculé* | *66.75* | *5.60* | *10.61* | *8.96* | *4.48* |
| *Trouvé* | *66.63* | *5.06* | *10.42* | *8.83* | *4.46* |

### Exemple 43 : Chlorhydrate de 1-{5-chloro-2-[((3S)-3-(4-morpholinylméthyl)-3,4-dihydro-2(1H)-isoquinolinyl)carbonyl]phényl}-N-(4-hydroxyphényl)-N-(1-méthyl-2,3-dihydro-1H-indol-5-yl)-5,6,7,8-tétrahydro-3-indolizine carboxamide

On procède selon le procédé de l'Exemple 1 en remplaçant d'une part le composé de la Préparation 1 utilisé au Stade A par le composé de la Préparation 24, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | %*C* | *%H* | *%N* | *%Cl* |
| *Calculé* | *68.35* | *5.74* | *8.86* | *8.97* |
| *Trouvé* | *68.29* | *5.21* | *8.76* | *9.04* |

### Exemple 44 : Chlorhydrate de 3-{5-fluoro-2-[((3S)-3-(4-morpholinylméthyl)-3,4-dihydro-2(1H)-isoquinolinyl)carbonyl]phényl}-N-(4-hydroxypleényl)-N-(1-méthyl-1H-indazol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 4, et d'autre part le composé de la Préparation 1" utilisé au Stade C par le *N-*(4-{[*tert-*butyl(diméthyl)silyl]oxy}phényl)-1-méthyl-1*H*-indazol-5-amine.

| ***Microanalyse élémentaire :*** | | | | |
|---|---|---|---|---|
| | %*C* | *%H* | *%N* | *%Cl⁻* |
| *Calculé* | *68.16* | *5.72* | *10.84* | *4.57* |
| *Trouvé* | *67.93* | *5.01* | *10.89* | *4.24* |

### Exemple 45 : 3-{5-Chloro-2-[((3S)-3-(4-morpholinylméthyl)-3,4-dihydro-2(1H)-isoquinolinyl)carbonyl]phényl}-N-(4-hydroxyphényl)-N-(1'-méthyl-1',2'-dihydrospiro [cyclopropane-1,3'-indol]-5'-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-(1-méthyl-1',2'-dihydrospiro[cyclopropane-1,3'-indol]-5'-yl)amine.

| ***Microanalyse élémentaire :*** | | | |
|---|---|---|---|
| | %*C* | %*H* | *%N* |
| *Calculé* | *72.15* | *6.18* | *8.95* |
| *Trouvé* | *71.86* | *5.76* | *8.85* |

### Exemple 46 : Chlorhydrate de 3-{5-chloro-2-[((3S)-3-(1,4-oxazépan-4-ylméthyl)-3,4-dihydro-2(1H)-isoquinolinyl)carbonyl]phényl}-N-(4-hydroxyphényl)-N-(1-méthyl-1H-indol-5-yl)-5,6,7,8-tétrahydro-1-indolizine carboxamide

On procède selon le procédé de l'Exemple 1 en remplaçant d'une part les composés des Préparations 1 et l'utilisés au Stade A par les composés respectifs des Préparations 3 et 4', et d'autre part le composé de la Préparation 1" utilisé au Stade C par le *N-(4-{[tert-*butyl(diméthyl)silyl]oxy}phényl)-1-méthyl-1*H*-indol-5-amine.

| ***Microanalyse élémentaire :*** | | | |
|---|---|---|---|
| | %*C* | *%H* | *%N* |
| *Calculé* | *68.65* | *5.89* | *8.70* |
| *Trouvé* | *68.33* | *5.45* | *8.54* |

***Pouvoir rotatoire :*** (α)_{D}²⁰ = + 13.6° (c = 4 mg/mL, MeOH)

### Exemple 47 : Chlorhydrate de N-(4-hydroxyphényl)-N-(1-méthyl-1H-indol-5-yl)-3-[2-[((3S)-3-(4-morpholinylméthyl)-3,4-dihydro-2(1H)-isoquinolinyl)carbonyl]-5-(trifluorornéthyl)phényl]-5,6,7,8-tétrahydro-1-indolizine carboxamide

On procède selon le procédé de l'Exemple 1 en remplaçant d'une part le composé de la Préparation 1 utilisé au Stade A par le composé de la Préparation 10, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | %*C* | *%H* | *%N* | *%Cl⁻* |
| *Calculé* | *67.02* | *5.50* | *8.50* | *4.30* |
| *Trouvé* | *67.01* | *5.11* | *8.47* | *4.21* |

### Exemple 48 : Chlorhydrate de N-(4-hydroxyphényl)-N-(1-méthyl-1H-indol-5-yl)-3-[2-[((3S)-3-(4-morpholinylméthyl)-3,4-dihydro-2(1H)-isoquinolinyl)carbonyl]-5-(trifluorométhoxy)phényl]-5,6,7,8-tetrahydro-1-indolizine carboxamide

On procède selon le procédé de l'Exemple 1 en remplaçant d'une part le composé de la Préparation 1 utilisé au Stade A par le composé de la Préparation 11, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | | |
|---|---|---|---|---|---|
| | *%C* | *%H* | *%N* | *%Cl* | *%Cl* |
| *Calculé* | *65.75* | *5.40* | *8.33* | *4.22* | *4.22* |
| *Trouvé* | *65.78* | *5.00* | *8*.*35* | *4.33* | *4.50* |

### Exemple 49 : 3-{5-Chloro-2-[((3S)-3-(4-morpholinylméthyl)-3,4-dihydro-2(1H)-isoquinolinyl)carbonyl]phényl}-N-(4-hydroxyphényl)-N-(1,3,3-triméthyl-2,3-dihydro-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-1,3,3-triméthyl-5-indolinamine, étant entendu que le produit ainsi obtenu n'est pas soumis à une étape de salification en présence d'éther chlorhydrique.

| ***Microanalyse élémentaire :*** | | | |
|---|---|---|---|
| | *%C* | *%H* | %*N* |
| *Calculé* | *71.97* | *6.42* | *8.93* |
| *Trouvé* | *71. 87* | *6.22* | *8.94* |

### Exemple 50 : Chlorhydrate de N-(4-hydroxyphényl)-N-(1-méthyl-1H-indazol-5-yl)-3-{2,2-dideutério-6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 9, et d'autre part le composé de la Préparation 1" utilisé au Stade C par le *N-(4-{[tert-*butyl(diméthyl)silyl]oxy}phényl)-1-méthyl-1*H*-indazol-5-amine.

| ***Microanalyse élémentaire :*** | | | | |
|---|---|---|---|---|
| | *%C* | *%H* | *%N* | *%Cl⁻* |
| *Calculé* | *67.28* | *5.66* | *10.46* | *4.41* |
| *Trouvé* | *66.77* | *5*.*07* | *10.25* | *3*.*92* |

### Exemple 51 : Chlorhydrate de N-(3-fluoro-4-méthylphényl)-N-(4-hydroxyphényl)-3-{2,2-dideutério-6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizine carboxamide

On procède selon le procédé de l'Exemple 1 en remplaçant d'une part le composé de la Préparation 1 utilisé au Stade A par le composé de la Préparation 9, et d'autre part le composé de la Préparation 1" utilisé au Stade C par le *N*-(4-{[*tert-*butyl(diméthyl)silyl]oxy}phényl)-3-fluoro-4-méthylaniline.

| ***Microanalyse élémentaire :*** | | | | |
|---|---|---|---|---|
| | *%C* | *%H* | *%N* | *%Cl⁻* |
| *Calculé* | *67.64* | *5*.*69* | *7.17* | *4.54* |
| *Trouvé* | *67.08* | *5.18* | *7.04* | *4*.*28* |

### Exemple 52 : Chlorhydrate de 3-{5-chloro-2-[((3S)-3-[(diméthylamino)méthyl]-3,4-dihydro-2(1H)-isoquinolinyl)carbonyl]phényl}-N-(4-hydroxyphényl)-N-(1-méthyl-1H-indol-5-yl)-5,6,7,8-tétrahydro-1-indolizine carboxamide

On procède selon le procédé de l'Exemple 1 en remplaçant d'une part les composés des Préparations 1 et l'utilisés au Stade A par les composés respectifs des Préparations 3 et 8', et d'autre part le composé de la Préparation 1" utilisé au Stade C par le *N*-(4-{[*tert-*butyl(diméthyl)silyl]oxy}phényl)-1-méthyl-1*H*-indol-5-amine,

### Masse haute résolution (ESI+) :

Formule brute : C₄₃H₄₂³⁵ClN₅O₃
[M+H]⁺ calculé : 712.3054
[M+H]⁺ mesuré: 712.3060

***Pouvoir rotatoire :*** (α)_{D}²⁰ = + 35.8° (c = 6 mg/mL, MeOH)

### Exemple 53 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-[4-méthoxy-3-(trifluorométhyl)phényl]-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-4-méthoxy-3-(trifluorométhyl)aniline.

| ***Microanalyse élémentaire :*** | | | | |
|---|---|---|---|---|
| | *%C* | *%H* | *%N* | *%Cl⁻* |
| *Calculé* | *63.23* | *5.19* | *6.70* | *4.24* |
| *Trouvé* | *63.08* | *4.68* | *6.79* | *3.92* |

### Exemple 54 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-[4-(méthylsulfanyl)-3-{trifluorométhyl)phényl]-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(*4*-{[*tert-*butyl(diméthyl)silyl]oxy}phényl)-4-(méthylsulfanyl)-3-(trifluorométhyl)aniline.

| ***Microanalyse élémentaire :*** | | | | | |
|---|---|---|---|---|---|
| | *%C* | *%H* | *%N* | *%S* | *%Cl⁻* |
| *Calculé* | *62.04* | *5.09* | *6*.*58* | *3.76* | *4.16* |
| *Trouvé* | *62.10* | *4.90* | *6*.*61* | *3.37* | *3.99* |

### Exemple 55 : Chlorhydrate de 3-{5-chloro-2-[((3S)-3-(4-morpholinylméthyl)-3,4-dihydro-2(1H)-isoquinolinyl)carbonyl]phényl}-N-(3-fluoro-4-hydroxyphényl)-N-(1-méthyl-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diaméthyl)silyl]oxy}-3-fluorophényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *66.83* | *5.48* | *8.66* | *4.38* |
| *Trouvé* | *66.84* | *4.92* | *8.68* | *3.63* |

### Exemple 56 : Chlorhydrate de N-(4-hydroxyphényl)-N-(1-méthyl-1H-indol-5-yl)-3-{2-[((3S)-3-(4-morpholinylméthyl)-3,4-dihydro-2(1H)-isoquinolinyl)carbonyl]phényl}-1-indolizine carboxamide

On procède selon le procédé de l'Exemple 1 en remplaçant d'une part le composé de la Préparation 1 utilisé au Stade A par le composé de la Préparation 12, et d'autre part le composé de la Préparation 1" utilisé au Stade C par le *N-(4-{[tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *71.84* | *5*.*63* | *9.31* | *4.71* |
| *Trouvé* | *71.74* | *5.25* | *9.24* | *4.38* |

### Exemple 57 : Bis(chlorhydrate) de N-(4-hydroxyphényl)-N-(1-méthyl-1H-indol-5-yl)-3-{6-[((3S)-3-[(4-méthyl-1-pipérazinyl)méthyl]-3,4-dihydro-2(1H)-isoquinolinyl)carbonyl]-1,3-benzodioxol-5-yl}-1-indolizine carboxamide

On procède selon le procédé de l'Exemple 1 en remplaçant d'une part les composés des Préparations 1 et l'utilisés au Stade A par les composés respectifs des Préparations 13 et 3', et d'autre part le composé de la Préparation 1" utilisé au Stade C par le *N*-(4-{[*tert-*butyl(diméthyl)silyl]oxy}phényl)-1-méthyl-1*H*-1-indol-5-amine.

| ***Microanalyse élémentaire :*** | | | | |
|---|---|---|---|---|
| | *%C* | *%H* | %*N* | *%Cl⁻* |
| *Calculé* | *66.74* | *5.48* | *9.94* | *8.38* |
| *Trouvé* | *66.69* | *5.25* | *9.80* | *8*.*03* |

***Pouvoir rotatoire :*** (α)_{D}²⁰ = + 113.2° (c = 6 mg/mL, MeOH)

### Exemple 58 : Chlorhydrate de N-(4-hydroxyphényl)-3-{6-[((3S)-3-{4-morpholmylméthyl)-3,4-dihydro-2(1H)-isoquinolinyl)carbonyl]-1,3-benzodioxol-5-yl}-N-phényl-1-indolizine carboxamide,

On procède selon le procédé de l'Exemple 1 en remplaçant d'une part le composé de la Préparation 1 utilisé au Stade A par le composé de la Préparation 13.

| ***Microanalyse élémentaire :*** | | | | |
|---|---|---|---|---|
| | *%C* | *%H* | %*N* | *%Cl⁻* |
| *Calculé* | *69.49* | *5.29* | *7.54* | *4.77* |
| *Trouvé* | *69.41* | *5.00* | *7*.*61* | *4.45* |

***Pourvoir rotatoire :*** (α)_{D}²⁰ = + 111.4° (c = 6 mg/mL, MeOH)

### Exemple 59 : Chlorhydrate de N-(4-hydroxyphényl)-N-(1-méthyl-1H-indol-5-yl)-3-{6-[((3S)-3-(4-morpholinylméthyl)-3,4-dihydro-2(1H)-isoquinolinyl)carbonyl]-1,3-benzodioxol-5-yl}-1-indolizine carboxamide

On procède selon le procédé de l'Exemple 1 en remplaçant d'une part le composé de la Préparation 1 utilisé au Stade A par le composé de la Préparation 13, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *69.38* | *5.32* | *8*.*79* | *4.45* |
| *Trouvé* | *68.95* | *5.12* | *8*.*57* | *3.92* |

***Pouvoir rotatoire :*** (α)_{D}²⁰ = + 116.8° (c = 5 mg/mL, MeOH)

### Exemple 60 : Chlorhydrate de N-(1-éthyl-1H-indol-5-yl)-N-(4-hydroxyphényl)-3-{6-[((3S)-3-(4-morpholinylméthyl)-3,4-dihydro-2(1H)-isoquinalinyl)carbonyl]-1,3-benzodioxol-5-yl}-1-indolizine carboxamide

On procède selon le procédé de l'Exemple 1 en remplaçant d'une part le composé de la Préparation 1 utilisé au Stade A par le composé de la Préparation 13, et d'autre part le composé de la Préparation 1" utilisé au Stade C par le *N-(4-{[tert-*butyl(diméthyl)silyl]oxy}phényl)-1-éthyl-1*H*-indol-5-amine.

| ***Microanalyse élémentaire :*** | | | | |
|---|---|---|---|---|
| | *%C* | %*H* | *%N* | *%Cl⁻* |
| *Calculé* | *69*.*66* | *5.47* | *8.64* | *4.38* |
| *Trouvé* | *69.81* | *5.13* | *8.64* | *4.30* |

### Exemple 61 : Chlorhydrate de N-(4-hydroxyphényl)-N-(1-méthyl-2,3-dihydro-1H-indol-5-yl)-3-{6-[((3S)-3-(4-morpholinylméthyl)-3,4-dihydro-2(1H)-isoquinolinyl)carbonyl]-1,3-benzodioxol-5-yl}-1-indolizine carboxamide

On procède selon le procédé de l'Exemple 1 en remplaçant d'une part le composé de la Préparation 1 utilisé au Stade A par le composé de la Préparation 13, et d'autre part le composé de la Préparation 1" utilisé au Stade C par le *N-(4-{[tert-*butyl(diméthyl)silyl]oxy}phényl)-1-méthyl-5-indolinamine.

### Masse haute résolution (ESI+) :

Formule brute : C₄₆H₄₃N₅O₆
[M+H]⁺ calculé : 762.3292
[M+H]⁺ mesuré: 762.3284

### Exemple 62 : Chlorhydrate de N-(4-hydroxyphényl)-N-(1-isopropyl-1H-indol-5-yl)-3-{6-[((3S)-3-(4-morpholinylméthyl)-3,4-dihydro-2(1H)-isoquinolinyl)carbonyl]-1,3-benzodioxol-5-yl}-1-indolizine carboxamide

On procède selon le procédé de l'Exemple 1 en remplaçant d'une part le composé de la Préparation 1 utilisé au Stade A par le composé de la Préparation 13, et d'autre part le composé de la Préparation 1" utilisé au Stade C par le *N*-(4-{[*tert-*butyl(diméthyl)silyl]oxy}phényl)-1-isopropyl-1*H*-indol-5-amine.

| ***Microanalyse élémentaire :*** (théorie pour 1.3 HCl) | | | | |
|---|---|---|---|---|
| | *%C* | *%H* | *%N* | *%Cl⁻* |
| *Calculé* | *69.94* | *5.62* | *8.50* | *4.30* |
| *Trouvé* | *69.66* | *5.50* | *8.42* | *4.28* |

### Exemple 63 : Chlorhydrate de N-(1-éthyl-2,3-dihydro-1H-indol-5-yl)-N-(4-hydroxyphényl)-3-{6-[((3S)-3-(4-morpholinylméthyl)-3,4-dihydro-2(1H)-isoquinolinyl)carbonyl]-1,3-benzodioxol-5-yl}-1-indolizine carboxamide

On procède selon le procédé de l'Exemple 1 en remplaçant d'une part le composé de la Préparation 1 utilisé au Stade A par le composé de la Préparation 13, et d'autre part le composé de la Préparation 1" utilisé au Stade C par le *N*-(4-{[*tert-*butyl(diméthyl)silyl]oxy}phényl)-1-éthyl-5-indolinamine.

| ***Microanalyse élémentaire :*** (théorie pour 1.3 HCl) | | | | |
|---|---|---|---|---|
| | *%C* | *%H* | %*N* | *%Cl⁻* |
| *Calculé* | *68.57* | *5*.*67* | *8.51* | *5.60* |
| *Trouvé* | *68.11* | *5.23* | *8.36* | *5.65* |

### Exemple 64 : Chlorhydrate de N-(4-hydroxyphényl)-N-(1-isopropyl-2,3-dihydro-1H-indol-5-yl)-3-{6-[((3S)-3-(4-morpholinylméthyl)-3,4-dihydro-2(1H)-isoquinolinyl) carbonyl]-1,3-benzodioxol-5-yl}-1-indolizine carboxamide

On procède selon le procédé de l'Exemple 1 en remplaçant d'une part le composé de la Préparation 1 utilisé au Stade A par le composé de la Préparation 13, et d'autre part le composé de la Préparation 1" utilisé au Stade C par le *N*-(4-{[*tert-*butyl(diméthyl)silyl]oxy}phényl)-1-isopropyl-5-indolinamine.

| ***Microanalyse élémentaire :*** (théorie pour 1.3 HCl) | | | | |
|---|---|---|---|---|
| | *%C* | *%H* | *%N* | %*Cl⁻* |
| *Calculé* | *68.85* | *5.81* | *8.36* | *5.50* |
| *Trouvé* | *68.58* | *5. 68* | *8.49* | *5.10* |

### Exemple 65 : Bis(chlorhydrate) de 3-{5-chloro-2-[((3S)-3-[(4-méthyl-1-pipérazinyl)méthyl]-3,4-dihydro-2(1H)-isoquinolinyl)carbonyl]phényl}-N-(4-hydroxyphényl)-N-(1-méthyl-1H-indol-5-yl)-1-indolizine carboxamide

On procède selon le procédé de l'Exemple 1 en remplaçant d'une part les composés des Préparations 1 et 1' utilisés au Stade A par les composés respectifs des Préparations 14 et 3', et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** (théorie pour 1.9 HCl) | | | | |
|---|---|---|---|---|
| | *%C* | *%H* | *%N* | *%Cl⁻* |
| *Calculé* | *66.36* | *5*.*44* | *10.09* | *8.09* |
| *Trouvé* | *66*.*35* | *5*.*38* | *9.86* | *7.70* |

### Exemple 66 : Bis(chlorhydrate) de 3-{5-chloro-2-[((3S)-3-(4-morpholinylmethyl)-3,4-dihydro-2(1H)-isoquinolinyl)carbonyl]phényl}-N-(4-hydroxyphényl)-N-(1-méthyl-2,3-dihydro-1H-indol-5-yl)-1-indolizine carboxamide

On procède selon le procédé de l'Exemple 1 en remplaçant d'une part le composé de la Préparation 1 utilisé au Stade A par le composé de la Préparation 14, et d'autre part le composé de la Préparation 1" utilisé au Stade C par le *N*-(4-{[*tert-*butyl(diméthyl)silyl]oxy}phényl)-1-méthyl-5-indolinamine.

| ***Microanalyse élémentaire :*** | | | | |
|---|---|---|---|---|
| | *%C* | *%H* | *%N* | *%Cl* |
| *Calculé* | *65.50* | *5.37* | *8.49* | *12.89* |
| *Trouvé* | *66.02* | *4.91* | *8.50* | *12.11* |

***Pouvoir rotatoire :*** (α)_{D}²⁰ = + 142.2° (c = 3.5 mg/mL, MeOH)

### Exemple 67 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-(1-méthyl-1H-indol-5-yl)-l-indolizine carboxamide

On procède selon le procédé de l'Exemple 1 en remplaçant d'une part le composé de la Préparation 1 utilisé au Stade A par le composé de la Préparation 14, et d'autre part le composé de la Préparation 1" utilisé au Stade C par le *N-(4-{[tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *68.70* | *5.25* | *8.90* | *4.51* |
| *Trouvé* | *68.43* | *4.88* | *8.83* | *4.13* |

***Pourvoir rotatoire :*** (α)_{D}²⁰ = + 133.3° (c = 3.5 mg/mL, MeOH)

### Exemple 68 : Chlorhydrate de 3-{5-chloro-2-[((3S)-3-(4-morpholinylméthyl)-3,4-dihydro-2(1H)-isoquinolinyl)carbonyl]phényl}-N-(4-fluorophényl)-N-(1-méthyl-1H-indol-5-yl)-1-indolizine carboxamide

On procède selon le procédé de l'Exemple 1 en remplaçant d'une part le composé de la Préparation 1 utilisé au Stade A par le composé de la Préparation 14, et d'autre part le composé de la Préparation 1" utilisé au Stade C par le *N*-(4-fluorophényl)-1-méthyl-1*H-*indol-5-amine, étant entendu que le Stade D se limite alors à une étape de formation du chlorhydrate.

| ***Microanalyse élémentaire :*** | | | | |
|---|---|---|---|---|
| | *%C* | *%H* | *%N* | *%Cl⁻* |
| *Calculé* | *68*.*53* | *5.11* | *8.88* | *4.49* |
| *Trouvé* | *68*.*48* | *4.71* | *9.09* | *4.28* |

**Exemple 69 : Chlorhydrate de *N-*(4-hydroxyphényl)-*N-*(1-méthyl-1,2,3,4-tétrahydro-6-quinolinyl)-3-{7-[((3*S*)-3-(4-morpholinylméthyl)-3,4-dihydro-2(1*H*)-isoquinolinyl)carbonyl]-2,3-dihydro-1,4-benzodioxin-6-yl}-1-indolizine carboxamide**

On procède selon le procédé de l'Exemple 1 en remplaçant d'une part le composé de la Préparation 1 utilisé au Stade A par le composé de la Préparation 15, et d'autre part le composé de la Préparation 1" utilisé au Stade C par le *N*-(4-{[*tert-*butyl(diméthyl)silyl]oxy}phényl)-1-méthyl-1,2,3,4-tétrahydro-6-quinolinamine.

### Masse haute résolution (ESI+) :

Formule brute : C₄₈H₄₇N₅O₆
[M+H]⁺ calculé : 790.3605
[M+H]⁺ mesuré : 790.3591

### Exemple 70 : Chlorhydrate de N-(4-hydroxyphényl)-N-(1-méthyl-1H-indol-5-yl)-3-{7-[((3S)-3-(4-morpholinylméthyl)-3,4-dihydro-2(1H)-isoquinolinyl)carbonyl]-2,3-dihydro-1,4-benzodioxin-6-yl}-1-indolizine carboxamide

On procède selon le procédé de l'Exemple 1 en remplaçant d'une part le composé de la Préparation 1 utilisé au Stade A par le composé de la Préparation 15, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | | |
|---|---|---|---|---|---|
| | *%C* | *%H* | *%N* | *%Cl* | *%Cl⁻* |
| *Calculé* | *69.66* | *5.47* | *8.64* | *4.38* | *4.38* |
| *Trouvé* | *69.45* | *4.68* | *8*.*56* | *4*.*64* | *4.21* |

### Exemple 71 : Chlorhydrate de N-(4-hydroxyphényl)-3-{5-méthoxy-2-[((3S)-3-(4-morpholinylméthyl)-3,4-dihydro-2(1H)-isoquinolinyl)carbonyl]phényl}-N-(1-méthyl-1H-indol-5-yl)-1-indolizine carboxamide

On procède selon le procédé de l'Exemple 1 en remplaçant d'une part le composé de la Préparation 1 utilisé au Stade A par le composé de la Préparation 16, et d'autre part le composé de la Préparation 1" utilisé au Stade C par le *N*-{4-{[*tert-*butyl(diméthyl)silyl]oxy}phényl)-1-méthyl-1*H*-indol-5-amine.

### Masse haute résolution (ESI+) :

Formule brute : C₄₆H₄₃N₅O₅
[M+H]⁺ calculé : 746.3342
[M+H]⁺ mesuré : 746.3348

### Exemple 72 : 8-{[4-Hydroxy(1-méthyl-1H-indol-5-yl)anilino]carbonyl}-6-{6-[((3S)-3-(4-morpholinylméthyl)-3,4-dihydro-2(1H)-isoquinolinyl)carbonyl]-1,3-benzodioxol-5-yl}-3,4-dihydropyrrolo[1,2-a]pyrazine-2(1H)-carboxylate de tert-butyle

On procède selon le procédé de l'Exemple 1 en remplaçant d'une part le composé de la Préparation 1 utilisé au Stade A par le composé de la Préparation 17, et d'autre part le composé de la Préparation 1" utilisé au Stade C par le *N*-(4-{[*tert-*butyl(diméthyl)silyl]oxy}phényl)-1-méthyl-1*H*-indol-5-amine, étant entendu que le produit ainsi obtenu n'est pas soumis à une étape de salification en présence d'éther chlorhydrique.

| ***Microanalyse élémentaire :*** | | | |
|---|---|---|---|
| | *%C* | %*H* | %*N* |
| *Calculé* | *69*.*43* | *6.06* | *9*.*72* |
| *Trouvé* | *69.41* | *5.84* | *9*.*68* |

### Exemple 73 : 8-{[4-Hydroxy(1-méthyl-2,3-dihydro-1H-indol-5-yl)anilino]carbonyl}-6-{6-[((3S)-3-(4-morpholinylméthyl)-3,4-dihydro-2(1H)-isoquinolinyl)carbonyl]-1,3-benzodioxol-5-yl}-3,4-dihydropyrrolo[1,2-a]pyrazine-2(1H)-carboxylate de tert-butyle

On procède selon le procédé de l'Exemple 1 en remplaçant d'une part le composé de la Préparation 1 utilisé au Stade A par le composé de la Préparation 17, et d'autre part le composé de la Préparation 1" utilisé au Stade C par le *N*-(4-{[*tert-*butyl(diméthyl)silyl]oxy}phényl)-1-méthyl-5-indolinamine, étant entendu que le produit ainsi obtenu n'est pas soumis à une étape de salification en présence d'éther chlorhydrique.

| ***Microanalyse élémentaire :*** | | | |
|---|---|---|---|
| | *%C* | *%H* | *%N* |
| *Calculé* | *69.27* | *6.28* | *9.69* |
| *Trouvé* | *69.20* | *6.11* | *9.77* |

### Exemple 74 : Bis(chlorhydrate) de N-(4-hydroxyphényl)-N-(1-méthyl-1H-indol-5-yl)-6-{6-[((3S)-3-(4-morpholinylméthyl)-3,4-dihydro-2(1H)-isoquinolinyl)carbonyl]-1,3-benzodioxol-5-yl}-1,2,3,4-tétrahydropyrrolo[1,2-a]pyrazine-8-carboxamide

On procède selon le procédé de l'Exemple 1 en remplaçant d'une part le composé de la Préparation 1 utilisé au Stade A par le composé de la Préparation 17, et d'autre part le composé de la Préparation 1" utilisé au Stade C par le N-(4-{[tert-butyl(diméthyl)silyl]oxy}phényl)-1-méthyl-1*H*-indol-5-amine, étant entendu que le produit ainsi obtenu n'est pas soumis à une étape de salification en présence d'éther chlorhydrique comme cela est décrit au Stade D de l'Exemple 1. Le composé ainsi obtenu est déprotégé en présence de 10 équivalents d'acide trifluoroacétique dans le dichlorométhane (10 mL/mmol) à température ambiante pendant une nuit. Puis, le produit est ensuite isolé par concentration à sec du milieu réactionnel. Il est enfin soumis à une étape de salification en présence d'éther chlorhydrique.

| ***Microanalyse élémentaire :*** (théorie pour 1.9 HCl) | | | | |
|---|---|---|---|---|
| | *%C* | *%H* | *%N* | *%Cl⁻* |
| *Calculé* | *64.80* | *5.55* | *10.08* | *8.08* |
| *Trouvé* | *64*.*48* | *5.41* | *9.85* | *7.84* |

### Exemple 75 : Tris(chlorhydrate) de N-(4-hydroxyphényl)-N-(1-méthyl-2,3-dihydro-1H-indol-5-yl)-6-{6[((3S)-3-(4-morpholinylméthyl)-3,4-dihydro-2(1H)-isoquinolinyl) carbonyl]-1,3-benzodioxol-5-yl}-1,2,3,4-tétrahydropyrrolo[1,2-a]pyrazine-8-carboxamide

On procède selon le procédé de l'Exemple 1 en remplaçant d'une part le composé de la Préparation 1 utilisé au Stade A par le composé de la Préparation 17, et d'autre part le composé de la Préparation 1" utilisé au Stade C par le *N*-(4-{[*tert-*butyl(diméthyl)silyl]oxy}phényl)-1-méthyl-5-indolinamine, étant entendu que le produit ainsi obtenu n'est pas soumis à une étape de salification en présence d'éther chlorhydrique comme cela est décrit au Stade D de l'Exemple 1.. Le composé ainsi obtenu est déprotégé en présence de 10 équivalents d'acide trifluoroacétique dans le dichlorométhane (10 mL/mmol) à température ambiante pendant une nuit. Puis, le produit est ensuite isolé par concentration à sec du milieu réactionnel. Il est enfin soumis à une étape de salification en présence d'éther chlorhydrique.

| ***Microanalyse élémentaire :*** | | | | |
|---|---|---|---|---|
| | *%C* | *%H* | *%N* | *%Cl⁻* |
| *Calculé* | *61.68* | *5.64* | *9.59* | *12.14* |
| *Trouvé* | *61.65* | *5.35* | *9.45* | *11.72* |

### Exemple 76 : Bis(chlorhydrate) de N-(4-hydroxyphényl)-N-(1-méthyl-1H-indol-5-yl)-5-{6-[((3S)-3-[(4-méthyl-1-pipérazinyl)méthyl]-3,4-dihydro-2(1H)-isoquinolinyl) carbonyl]-1,3-benzodioxol-5-yl}-2,3-dihydro-1H-pyrrolizine-7-carboxamide

On procède selon le procédé de l'Exemple 1 en remplaçant d'une part les composés des Préparations 1 et l'utilisés au Stade A par les composés respectifs des Préparations 18 et 3', et d'autre part le composé de la Préparation 1" utilisé au Stade C par le *N*-(4-{[*tert-*butyl(diméthyl)silyl]oxy}phényl)-1-méthyl-1*H*-indol-5-amine.

### Masse haute résolution (ESI+) :

Formule brute : C₄₆H₄₆N₆O₅
[M+H]⁺ calculé : 763.3608
[M+H]⁺ mesuré : 763.3594

### Exemple 77 : Bis(chlorhydrate) de 5-{5-chloro-2-[((3S)-3-[(4-méthyl-1-pipérazinyl)méthyl]-3,4-dihydro-2(1H)-isoquinolinyl)carbonyl]phényl}-N-(4-hydroxyphényl)-N-(1-méthyl-1H-indol-5-yl)-2,3-dihydro-1H-pyrrolizine-7-carboxamide

On procède selon le procédé de l'Exemple 1 en remplaçant d'une part les composés des Préparations 1 et l'utilisés au Stade A par les composés respectifs des Préparations 19 et 3', et d'autre part le composé de la Préparation 1" utilisé au Stade C par le *N-(4-{[tert-*butyl(diméthyl)silyl]oxy}phényl)-1-méthyl-1*H*-indol-5-amine.

| ***Microanalyse élémentaire :*** (théorie pour pour 1.9 HCl) | | | | |
|---|---|---|---|---|
| | *%C* | *%H* | *%N* | *%Cl⁻* |
| *Calculé* | *65.70* | *5.75* | *10.22* | *8.19* |
| *Trouvé* | *65.46* | *5.55* | *10.68* | *7.65* |

### Exemple 78 : Chlorhydrate de 6-{5-chloro-2-[((3S)-3-(4-morpholinylméthyl)-3,4-dihydro-2(1H)-isoquinolinyl)carbonyl]phényl}-N-(4-hydroxyphényl)-N-phényl-3,4-dihydro-1H-pyrrolo[2,1-c][1,4]oxazine-8-carboxamide

On procède selon le procédé de l'Exemple 1 en remplaçant le composé de la Préparation 1 utilisé au Stade A par le composé de la Préparation 20.

| ***Microanalyse élémentaire :*** | | | | |
|---|---|---|---|---|
| | *%C* | *%H* | *%N* | *%Cl⁻* |
| *Calculé* | *66.57* | *5.45* | *7.57* | *4.79* |
| *Trouvé* | *66.22* | *5.23* | *7.53* | *4.57* |

### Exemple 79 : Bis(chlorhydrate) de 6-{5-chloro-2-[((3S)-3-(4-morpholinylméthyl)-3,4-dihydro-2(1H)-isoquinolinyl)carbonyl]phényl}-N-(2,3-dihydro-1H-indol-5-yl)-N-(4-hydroxyphényl)-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 20, et d'autre part le composé de la Préparation 1" utilisé au Stade C par le *N*-(4-{[*tert-*butyl(diméthyl)silyl]oxy}phényl)-5-indolinamine.

| ***Microanalyse élémentaire :*** | | | | |
|---|---|---|---|---|
| | *%C* | *%H* | *%N* | *%Cl⁻* |
| *Calculé* | *63.20* | *5*.*43* | *8.57* | *8.68* |
| *Trouvé* | *62.64* | *5.19* | *8.39* | *8.03* |

### Exemple 80 : N-(4-Hydroxyphényl)-N-(1-méthyl-1H-indol-5-yl)-6-{6-[((3S)-3-(4-morpholinylméthyl)-3,4-dihydro-2(1H)-isoquinolinyl)carbonyl]-1,3-benzodioxol-5-yl}-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 21, et d'autre part le composé de la Préparation 1" utilisé au Stade C par le *N*-(4-{[*tert-*butyl(diméthyl)silyl]oxy}phényl)-1-méthyl-1*H*-indol-5-amine, étant entendu que le produit ainsi obtenu n'est pas soumis à une étape de salification en présence d'éther chlorhydrique.

| ***Microanalyse élémentaire :*** | | | |
|---|---|---|---|
| | *%C* | *%H* | *%N* |
| *Calculé* | *70.57* | *5*.*66* | *9.14* |
| *Trouvé* | *69.99* | *5.53* | *9*.*04* |

### Exemple 81 : Chlorhydrate de N-(4-hydroxyphényl)-N-(1-méthyl-2,3-dihydro-1H-indol-5-yl)-6-{6-[((3S)-3-(4-morpholinylméthyl)-3,4-dihydro-2(1H)-isoquinolinyl) carbonyl]-1,3-benzodioxol-5-yl}-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 21, et d'autre part le composé de la Préparation 1" utilisé au Stade C par le *N*-(4-{[*tert-*butyl(diméthyl)silyl]oxy}phényl)-1-méthyl-5-indolinamine.

| ***Microanalyse élémentaire :*** | | | | |
|---|---|---|---|---|
| | %*C* | *%H* | *%N* | *%Cl⁻* |
| *Calculé* | *67.20* | *5.76* | *8.71* | *4.41* |
| *Trouvé* | *66*.*67* | *5.60* | *8*.*66* | *5.10* |

### Exemple 82 : Chlorhydrate de 6-{5-chloro-2-[((3S)-3-(4-morpholinylméthyl)-3,4-dihydro-2(1H)-isoquinolinyl)carbonyl]phényl}-N-(3-fluoro-4-méthylphényl)-N-{4-hydroxyphényl)-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 20, et d'autre part le composé de la Préparation 1" utilisé au Stade C par le *N*-(4-{[*tert-*butyl(diméthyl)silyl]oxy}phényl)-3-fluoro-4-méthylaniline.

| ***Microanalyse élémentaire :*** | | | | | |
|---|---|---|---|---|---|
| | *%C* | *%H* | *%N* | *%Cl* | *%Cl⁻* |
| *Calculé* | *65.37* | *5*.*35* | *7.26* | *9.19* | *4.59* |
| *Trouvé* | *65.49* | *4.67* | *7.45* | *9.18* | *4.40* |

***Pourvoir rotatoire :*** (α)_{D}²⁰= + 35.0° (c = 6 mg/mL, MeOH)

### Exemple 83 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-(1-méthyl-1H-indol-6-yl)-6,7,8,9-tétrahydro-5H-pyrrolo[1,2-a]azépine-1-carboxamide

On procède selon le procédé de l'Exemple 1 en remplaçant d'une part le composé de la Préparation 1 utilisé au Stade A par le composé de la Préparation 22, et d'autre part le composé de la Préparation 1" utilisé au Stade C par le *N*-(4-{[*tert-*butyl(diméthyl)silyl]oxy}phényl)-1-méthyl-1*H*-indol-5-amine.

### Masse haute résolution (ESI+) :

Formule brute : C₄₆H₄₆³⁵ClN₅O₄
[M+H]⁺ calculé : 768.3317
[M+H]⁺ mesuré: 768.3254

### Exemple 84 : Chlorhydrate de N-(4-hydroxyphényl)-N-(1-méthyl-1H-indol-6-yl)-3-{6-[((3S)-3-(4-morpholinylméthyl)-3,4-dihydro-2(1H)-isoquinolinyl)carbonyl]-1,3-benxodioxol-5-yl}-6,7,8,9-tétrahydro-5H-pyrrolo[1,2-a]azépine-1-carboxamide

On procède selon le procédé de l'Exemple 1 en remplaçant d'une part le composé de la Préparation 1 utilisé au Stade A par le composé de la Préparation 23, et d'autre part le composé de la Préparation 1" utilisé au Stade C par le *N*-(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *69.32* | *5.94* | *8.60* | *4.35* |
| *Trouvé* | *69.42* | *5.52* | *8.74* | *4.05* |

***Pourvoir rotatoire :*** (α)_{D}²⁰ = + 86.6° (c = 8 mg/mL, MeOH)

### Exemple 85 : Chlorhydrate de 3-{5-chloro-2-[((3S)-3-(4-morpholinylméthyl)-3,4-dihydro-2(1H)-isoquinolinyl)carbonyl]phényl}-N-(4-hydroxyphényl)-N-(3-thiényl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-3-thiophénamine.

| ***Microanalyse élémentaire :*** | | | | | |
|---|---|---|---|---|---|
| | *%C* | *%H* | *%N* | %*S* | *%Cl⁻* |
| *Calculé* | *64.60* | *5.42* | *7.53* | *4.31* | *4.77* |
| *Trouvé* | *64.67* | *5.05* | *7.37* | *3.90* | *4.19* |

### Exemple 86 : Chlorhydrate de 6-{5-chloro-2-[((3S)-3-(4-morpholinylméthyl)-3,4-dihydro-2(1H)-isoquinolinyl)carbonyl]phényl}-N-(4-hydroxyphényl)-N-(1-méthyl-1H-indol-5-yl)-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 20, et d'autre part le composé de la Préparation 1" utilisé au Stade C par le *N-*(*4-*{[*tert-*butyl(diméthyl)silyl]oxy}phényl)-1-méthyl-1*H*-indol-5-amine.

### Masse haute résolution (ESI+) :

Formule brute : C₄₄H₄₂³⁵ClN₅O₅
[M+H]⁺ calculé : 756.2953
[M+H]⁺mesuré : 756.2936

### Exemple 87 : Chlorhydrate de N-butyl-N-(4-hydroxyphényl)-3-{6-[((3S)-3-{4-morpholinylméthyl)-3,4-dihydro-2(1H)-isoquinolinyl)carbonyl]-1,3-benzodioxol-5-yl} -5,6,7,8-tétrahydro-1-indolizine carboxamide

On procède selon le procédé de l'Exemple 1 en remplaçant le composé de la Préparation 1" utilisé au Stade C par le *N*-butyl-4-{[*tert*-butyl{diméthyl)silyl]oxy)aniline.

| ***Microanalyse élémentaire :*** | | | | |
|---|---|---|---|---|
| | %C | %*H* | *%N* | *%Cl⁻* |
| *Calculé* | *67.71* | *6*.*51* | *7.70* | *4.87* |
| *Trouvé* | *67.48* | *6.30* | *7.74* | *5.01* |

### Exemple 88 : Chlorhydrate de N-butyl-N-[(3-{6-[((3S)-3-(4-morpholinylméthyl)-3,4-dihydro-2(1H)-isoquinolinyl)carbonyl]-1,3-benzodioxol-5-yl}-5,6,7,8-tétrahydro-1-indolizinyl)méthyl]-1-butanamine

On procède selon le procédé de l'Exemple 1 en remplaçant le composé de la Préparation 1" utilisé au Stade C par la *N,N-*dibutylamine, étant entendu que le Stade D se limite alors à une étape de formation du chlorhydrate.

### Masse haute résolution (ESI+) :

Formule brute : C₃₉H₅₀N₄O₅
[M+H]⁺ calculé : 655.3859
[M+H]⁺ mesurée: 655.3826

### Exemple 89: Chlorhydrate de 3-{5-chloro-2-[((3S)-3-(4-morpholinylméthyl)-3,4-dihydro-2(1H)-isoquinolinyl)carbonyl]phényl}-N-(3-hydroxypropyl)-N-(1-méthyl-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N-*(3*-*{[*tert-*butyl(diméthyl)silyl]oxy}propyl)-1-méthyl-1*H*-indol-5-amine.

| ***Microanalyse élémentaire :*** | | | | | |
|---|---|---|---|---|---|
| | *%C* | *%H* | *%N* | *%Cl* | *%Cl⁻* |
| *Calculé* | *66.66* | *6.26* | *9.25* | *9.37* | *4.68* |
| *Trouvé* | *66.51* | *5.87* | *9.25* | *9.12* | *4.21* |

### Exemple 90: Chlorhydrate de 3-{5-chloro-2-[((3S)-3-(4-morpholinylméthyl)-3,4-dihydro-2(1H)-isoquinolinyl)carbonyl]phényl}-N-(4-hydroxybutyl)-N-(1-méthyl-1H-indol-5-yl)-5,6,7,8-tétrahydro-1-indolizine carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N-*(3-{[*tert-*butyl(diméthyl)silyl]oxy}butyl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *67.01* | *6.41* | *9.09* | *4.60* |
| *Trouvé* | *66.93* | *5.88* | *9.23* | *4.30* |

### Exemple 91 : Chlorhydrate de N-(3-fluoro-4-méthylphényl)-3-(5-fluoro-2-{[(3S)-3-(morpholin-4-ylméthyl)-3,4-dihydroisoquinolin-2(1H)-yl]carbonyl}phényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 4, et d'autre part le composé de la Préparation 1" utilisé au Stade C par le *N-*(4-{[*tert-*butyl(diméthyl)silyl]oxy}phényl)-3-fluoro-4-méthylaniline.

| ***Microanalyse élémentaire :*** | | | | |
|---|---|---|---|---|
| | *%C* | *%H* | *%N* | *%Cl⁻* |
| *Calculé* | *68.56* | *5.75* | *7.44* | *4.71* |
| *Trouvé* | *68.57* | *5.23* | *7.53* | *4.74* |

### Exemple 92 : Chlorhydrate de N-(3-fluoro-4-méthylphényl)-N-(4-hydroxyphényl)-6-(6-{[(3S)-3-(morpholin-4-ylméthyl)-3,4-dihydroisoquinolin-2(1H)-yl]carbonyl}-1,3-benzodioxol-5-yl)-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 21, et d'autre part le composé de la Préparation 1" utilisé au Stade C par le *N-*(4-{[*tert-*butyl(diméthyl)silyl]oxy}phényl)-3-fluoro-4-méthylaniline.

| ***Microanalyse élémentaire :*** | | | | |
|---|---|---|---|---|
| | *%C* | *%H* | *%N* | *%Cl⁻* |
| *Calculé* | *66.11* | *5.42* | 7.17 | *4.54* |
| *Trouvé* | *66.02* | *4.92* | *7.13* | *4.26* |

### Exemple 93: Chlorhydrate de 3-(5-chloro-2-{[(3R)-3-{morpholin-4-ylméthyl)-3,4-dihydroisoquinolin-2(1H)-yl]carbonyl}phényl)-N-(4-hydroxyphényl)-N-(1-méthyl-1H-indol-5-yl)-5,6,7,8-tétrahydroindolizine-1-carboxamide

On procède selon le procédé de l'Exemple 1 en remplaçant d'une part les composés des Préparations 1 et 1' utilisés au Stade A par les composés des Préparations 3 et 2', et d'autre part le composé de la Préparation 1" utilisé au Stade C par le *N-*(4-{[*tert-*butyl(diméthyl)silyl]oxy}phényl)-1-méthyl-1*H*-indol-5-amine.

| ***Microanalyse élémentaire :*** | | | | | |
|---|---|---|---|---|---|
| | %C | *%H* | *%N* | *%Cl* | *%Cl⁻* |
| *Calculé* | *68.35* | *5.74* | *8.86* | *8.97* | *4.48* |
| *Trouvé* | *68.27* | *5.14* | *8.92* | *8.70* | *4.08* |

### Exemple 94 Chlorhydrate de N-(4-hydroxyphényl)-N-(1-méthyl-1H-indazol-5-yl)-3-(6-{[(3S)-3-(morpholin-4-ylméthyl)-3,4-dihydroisoquinolin-2(1H)-yl]carbonyl}-1,3-benzodioxol-5-yl)-5,6,7,8-tétrahydroindolizine-1-carboxamide

On procède selon le procédé de l'Exemple 1 en remplaçant le composé de la Préparation 1" utilisé au Stade C par le *N-*(4-{[*tert*-butyl(diméthyl)silyl]oxy}phényl)-1-méthyl-1*H-*indazol-5-amine.

| ***Microanalyse élémentaire :*** | | | | |
|---|---|---|---|---|
| | *%C* | *%H* | *%N* | *%Cl⁻* |
| *Calculé* | *67.45* | *5.66* | *10.49* | *4.42* |
| *Trouvé* | *67.40* | *5.19* | *10.40* | *4.11* |

### Exemple 95 : Chlorhydrate de N-(4-hydroxyphényl)-N-(1-méthyl-1H-indol-5-yl)-3-(5-méthyl-2-{[(3S)-3-(morpholin-4-ylméthyl)-3,4-dihydroisoquinolin-2(1H)-yl]carbonyl} phényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 25, et d'autre part le composé de la Préparation 1" utilisé au Stade C par le *N-*(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *71.72* | *6.28* | *9.09* | *4.60* |
| *Trouvé* | *71.17* | *5.66* | *8.85* | *4*.*47* |

### Exemple 96 : Chlorhydrate de N-(4-hydroxyphényl)-N-(1-méthyl-1H-indol-5-yl)-5-(6-{[(3S)-3-(morpholin-4-ylméthyl)-3,4-dihydroisoquinolin-2(1H)-yl]carbonyl}-1,3-benzodioxol-5-yl)-2,3-dihydro-1H-pyrrolizine-7-carboxamide

On procède selon le procédé de l'Exemple 1 en remplaçant d'une part le composé de la Préparation 1 utilisé au Stade A par le composé de la Préparation 18, et d'autre part le composé de la Préparation 1" utilisé au Stade C par le *N-*(4-{[*tert-*butyl(diméthyl)silyl]oxy}phényl)-1-méthyl-1*H*-indol-5-amine.

| ***Microanalyse élémentaire :*** | | | | |
|---|---|---|---|---|
| | %*C* | *%H* | *%N* | *%Cl⁻* |
| *Calculé* | *68.74* | *5.64* | *8.91* | *4.51* |
| *Trouvé* | *68.41* | *4.96* | *8.84* | *4.31* |

### Exemple 97 : Chlorhydrate de N-(3-chloro-4-méthylphényl)-3-{5-chloro-2-{[(3S)-3-(morpholin-4-ylméthyl)-3,4-dihydroisoquinolin-2(1H)-yl]carbonyl}phényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N-*(4-{[*tert-*butyl(diméthyl)silyl]oxy}phényl)-3-chloro-4-méthylaniline.

| ***Microanalyse élémentaire :*** | | | | |
|---|---|---|---|---|
| | *%C* | *%H* | *%N* | *%Cl⁻* |
| *Calculé* | *65.69* | *5.51* | *7.13* | *4.51* |
| *Trouvé* | *65.58* | *5.03* | *7.05* | *4.25* |

### Exemple 98 : Chlorhydrate de N-(3-fluoro-4-méthylphényl)-6-(5-fluoro-2-{[(3S)-3-(morpholin-4-ylméthyl)-3,4-dihydroisoquinolin-2(1H)-yl]carbonyl}phényl)-N-(4-hydroxyphényl)-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 26, et d'autre part le composé de la Préparation 1" utilisé au Stade C par le *N-*(4-{[*tert-*butyl(diméthyl)silyl]oxy}phényl)-3-fluoro-4-méthylaniline.

### Masse haute résolution (ES1+) :

Formule brute : C₄₂H₄₀F₂N₄O₅
[M+H]⁺ calculé : 719.3045
[M+H]⁺ mesuré: 719.3030

### Exemple 99 : Chlorhydrate de 6-(5-fluoro-2-{[(3S)-3-(morpholin-4-ylméthyl)-3,4-dihydroisoquinolin-2(1H)-yl]carbonyl}phényl)-N-(4-fluorophényl)-N-(4-hydroxyphényl)-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 26, et d'autre part le composé de la Préparation 1" utilisé au Stade C par le 4-([*tert*-butyl(diméthyl)silyl]oxy}-*N*-(4-fluorophényl)aniline.

### Masse haute résolution (ESI+) :

Formule brute : C₄₁H₃₈F₂N₄O₅
[M+H]⁺ calculé : 705.2889
[M+H]⁺ mesuré : 705.2882

### Exemple 100: Chlorhydrate de 3-(5-fluoro-2-{[(3S)-3-(morpholin-4-ylméthyl)-3,4-dihydroisoquinolin-2(1H)-yl]carbonyl}phényl)-7-hydroxy-N-(4-hydroxyphényl)-N-phényl-5,6,7,8-tétrahydroindolizine-1-carboxamide

### Stade A : 3'-(5-Fluoro-2-{[(3S)-3-(morpholin-4-ylméthyl)-3,4-dihydroisoquinolin-2(1H)-yl]carbonyl}phényl)-5',6'-dihydro-8'H-spiro[1,3-dioxolane-2,7'-indolizine]-1'-carboxylate de méthyle

On procède selon le procédé du Stade A de l'Exemple 1 en remplaçant le composé de la Préparation 1 par le composé de la Préparation 27.

### Stade B : 3-(5-Fluoro-2-{[(3S)-3-(morpholin-4-ylméthyl)-3,4-dihydroisoquinolin-2(1H)-yl]carbonyl]phényl)-7-(prop-2-én-1-yloxy)-5,6,7,8-tétrahydroindolizine-1-carboxylate de méthyle

On procède selon le procédé des Stades B, C et D de l'Exemple 113.

### Stade C : N-(4-{[tert-Butyl(diméthyl)silyl]oxy}phényl)-3-(5-fluoro-2-{[(3S)-3-(morpholin-4-ylméthyl)-3,4-dihydroisoquinolin-2(1H)-yl]carbonyl}phényl)-N-phényl-7-(prop-2-en-1-yloxy)-5,6,7,8-tétrahydroindolizine-1-carboxamine

On procède selon les procédés des Stades B et C de l'Exemple 1.

### Stade D : N-(4-{[tert-Butyl(diméthyl)silyl]oxy}phényl)-3-(5-fluoro-2-{[(3S)-3-(morpholin-4-ylméthyl)-3,4-dihydroisoquinolin-2(1H)-yl]carbonyl}phényl)-N-phényl-7-hydroxy-5,6,7,8-tétrahydroindolizine-1-carboxamide

On réalise ensuite une réaction de déprotection du groupement allyle en présence de 1,3-diméthylpyrimidine-2,4,6(1*H*,3*H*,5*H*)-trione (aussi appelé diméthylbarbiturate) et de tétrakis(triphénylphosphine)palladium dans un mélange de méthanol et de dichlorométhane.

### Stade E : Chlorhydrate de 3-(5-fluoro-2-{[(3S)-3-(morpholin-4-ylméthyl)-3,4-dihydroisoquinolin-2(1H)-yl]carbonyl}phényl)-7-hydroxy-N-(4-hydroxyphényl)-N-phényl-5,6,7,8-tétrahydroindolizine-1-carboxamide

La déprotection du groupement *tert*-butylsilyloxy est réalisée selon le procédé du Stade D de l'Exemple 1.

| ***Microanalyse élémentaire :*** | | | |
|---|---|---|---|
| | *%C* | *%H* | *%N* |
| *Calculé* | *71.98* | *5.90* | *7.99* |
| *Trouvé* | *71.18* | *5.98* | *7.18* |

### Masse haute résolution (ESI+) :

Formule brute : C₄₂H₄₂FN₄O₅
[M+H]⁺ calculé: 701.3139
[M+H]⁺ mesuré: 701.3134

### Exemple 101 : Chlorhydrate de 6-(5-chloro-2-{[(3S)-3-(morpholin-4-ylméthyl)-3,4-dihydroisoquinolin-2(1H)-yl]carbonyl}phényl)-N-(4-hydroxyphényl)-N-(1-méthyl-1H-indazol-5-yl)-3,4-dihydro-1H-pyrrolo[2,1-c][1,4]oxazine-8-carboxamide

On procède selon le procédé de l'Exemple 1 en remplaçant d'une part le composé de la Préparation 1 utilisé au Stade A par le composé de la Préparation 20, et d'autre part le composé de la Préparation 1" utilisé au Stade C par le *N-*(4-{[*tert-*butyl(diméthyl)silyl]oxy}phényl)-1-méthyl-1*H*-indazol-5-amine.

| ***Microanalyse élémentaire :*** | | | |
|---|---|---|---|
| | *%C* | *%H* | *%N* |
| *Calculé* | *65.07* | *5.33* | *10.59* |
| *Trouvé* | *65.54* | 4.86 | *10.57* |

### Exemple 102 : Chlorhydrate de (2R)-5-(5-fluoro-2-{[(3S)-3-(morpholin-4-ylméthyl)-3,4-dihydroisoquinolin-2(1H)-yl]carbonyl}phényl)-2-hydroxy-N-(4-hydroxyphényl)-N-phényl-2,3-dihydro-1H-pyrrolizine-7-carboxamide

### Stade A : (2R)-2-{[tert-Butyl(diméthyl)silyl]oxy}-5-(5-fluoro-2-{[(3S)-3-(morpholin-4-ylméthyl)-3,4-dihydroisoquinolin-2(1H)-yl]carbonyl}phényl)-2,3-dihydro-1H-pyrrolizine-7-carboxylate de méthyle

On procède selon le procédé du Stade A de l'Exemple 1 en remplaçant le composé de la Préparation 1 par le composé de la Préparation 28.

### Stade B : (2R)-5-(5-Fluoro-2-{[(3S)-3-(morpholin-4-ylméthyl)-3,4-dihydro isoquinolin-2(1H)-yl]carbonyl}phényl)-2-hydroxy-2,3-dihydro-1H-pyrrolizine-7 carboxylate de méthyle

A une solution du composé du Stade A (1g ; 1,54 mmol) dans du THF (8 mL) est ajouté une solution 1 M de fluorure de tétrabutylammonium (1,7 mL ; 1,7 mmol). Le milieu réactionnel est agité 3 h à température ambiante, puis le THF est évaporé et remplacé par de l'acétate d'éthyle. La phase organique est lavée avec de l'eau, de la saumure, avant d'être séchée sur MgSO_{4,} filtrée et concentrée à sec. Le produit brut obtenu (830 mg) est engagé tel quel dans l'étape suivante.

**RMN ¹H:** δ (500 MHz ; dmso-d6 ; 300°K) 7.55-6.9 (m, 7H, H aromatiques) ; 6.55-6.35 (m, 1H, H aromatique dihydropyrrolizine) ; 5.5-5.3 (m, 1H, alcool) ; 5.25-4.6 (m, 1H, H tertiaire tétrahydroisoquinoline) ; 5.0-4.2 (m, 2H, H aliphatique tétrahydroisoquinoline) ; 4.95-4.62 (m, 1H, HCOH); 3.7-3.6 (sl, 3H, OMe) ; 3.6-3.2. (m, 2H, 2H, H aliphatique dihyropyrrolizine) ; 3.7-3.5 (m, 4H, H morpholine) ; 3.0-2.4 (m, 2H, H aliphatique tétrahydroisoquinoline); 2.6-1.96 (m,4H,H morpholine); 2.6-1.96 (m, 2H, H aliphatique dihydropyrrolizine)

### Stade C: (2R)-5-(5-Fluoro-2-{[(3S)-3-(morpholin-4-ylméthyl)-3,4-dihydro isoquinolin-2(1H)-yl]carbonyl}phényl)-2-(prop-2-en-1-yloxy)-2,3-dihydro-1H-pyrrolizine-7-carboxylate de méthyle

A une suspension de 62 mg (1,54 mmol) d'hydrure de sodium dans 8 mL de THF anhydre refroidi à 0°C, on ajoute une solution du composé du Stade B (820 mg ; 1.54mmol) dans le THF (6 mL). Cette suspension est agitée 15 minutes à 0°C. On additionne ensuite 0,15 mL (1,69 mmol) de bromure d'allyle au goutte à goutte. Le milieu réactionnel est agité pendant 5 h à température ambiante, puis hydrolysé avec une solution aqueuse saturée en bicarbonate de sodium, et extrait au dichlorométhane. La phase organique est séchée sur MgSO_{4,} filtrée et concentrée à sec. L'huile ainsi obtenue est purifiée par chromatographie flash (gradient : dichlorométhane/méthanol ammoniacal) pour conduire au produit du titre sous forme solide.

**RMN ¹H**: δ (500 MHz ; dmso-d6 ; 300°K): 7.5-6.8 (m, 7H, H aromatiques) ; 6.55-6.3 (m, 1H, H aromatique dihydropyrrolizine) ; 5.9 (s, 1H, H allyle);5.3-5.2 (m, 2H, H allyle) ; 5.2-5.0 (m, 1H, H tertiaire tétrahydroisoquinoline) ; 5.0-4.2 (m, 2H, H aliphatique tétrahydroindolizine); 4.7-4.42 (m, 1H, HCOallyle); 4.6-3.85 (m, 2H, H aliphatique dihydropyrrolizine) ; 4.6-3.85 (m, 2H, CH2 allylique) ; 3.7-3.5 (m, 3H, OMe ); 3.65-3.5 (m, 4H , morpholine) ; 3.3-2.4 (m, 4H, morpholine et H aliphatique dihydropyrrolidine); 2.4-1.7 (m, 6H, morpholine et CH₂N)

### Stade D : (2R)-N-(4-{[tert-butyl(diméthyl)silyl]oxy}phényl)-5-(5-fluoro-2-{[(3S)-3-(morpholin-4-ylméthyl)-3,4-dihydroisoquinolin-2(1H)-yl]carbonyl}phényl)-N-phényl-2-(prop-2-en-1-yloxy)-2,3-dihydro-1H-pyrrolizine-7-carboxamide

On procède selon les procédés des Stades B et C de l'Exemple 1.

### Stade E : (2R)-N-(4-{[tert-butyl(diméthyl)silyl]oxy}phényl)-5-(5-fluoro-2-{[(3S)-3-(morpholin-4-ylméthyl)-3,4-dihydroisoquinolin-2(1H)-yl]carbonyl}phényl)-2-hydroxy-N-phényl-2,3-dihydro-1H-pyrrolizine-7-carboxamide

On réalise ensuite une réaction de déprotection du groupement allyle en présence de 1,3-diméthylpyrimidine-2,4,6(1*H*,3*H*,5*H*)-trione (aussi appelé diméthylbarbiturate) et de tétrakis(triphénylphosphine)palladium (*Synlett,* **2007,** 21, p 3136).

### Stade F: Chlorhydrate de (2R)-5-(5-fluoro-2-{[(3S)-3-(morpholin-4-ylméthyl)-3,4-dihydroisoquinolin-2(1H)-yl]carbonyl}phényl)-2-hydroxy-N-(4-hydroxyphényl)-N-phényl-2,3-dihydro-1H-pyrrolizine-7-carboxamide

La déprotection du groupement *tert*-butylsilyloxy est réalisée selon le procédé du Stade D de l'Exemple 1.

| ***Microanalyse élémentaire :*** | | | | |
|---|---|---|---|---|
| | *%C* | *%H* | *%N* | *%Cl⁻* |
| *Calculé* | *68*.*09* | *5.57* | *7.75* | *4.90* |
| *Trouvé* | *67.73* | *5.10* | *7.54* | *4.84* |

### Masse haute résolution (ESI+) :

Formule brute : C₄₁FH₃₉N₄O₅
[M+H]⁺ calculé : 687.2983
[M+H]⁺ mesuré : 687.2958

### Exemple 103 3-(5-Chloro-2-{[(3S)-3-(morpholin-4-ylméthyl)-3,4-dihydroisoquinolin-2(1H)-yl]carbonyl}phényl)-N-(4-hydroxyphényl)-N-(1-méthyl-1H-pyrrolo[2,3-b]pyridin-5-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N-*(4-{[*tert-*butyl(diméthyl)silyl]oxy}phényl)-1-méthyl-1*H*-pyrrolo[2,3-*b*]pyridin-5-amine.

### Masse haute résolution (ESI+) :

Formule brute : C₄₄ClH₄₄N₆O₄
[M+H]^{*} calculé : 755.3113
[M+H]⁺ mesuré : 755.3088

### Exemple 104 : Chlorhydrate de N-(4-hydroxyphényl)-3-(5-méthoxy-2-{[(3S)-3-(morpholin-4-ylméthyl)-3,4-dihydroisoquinolin-2(1H)-yl]carbonyl}phényl)-N-(1-méthyl-1H-indazol-5-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 6, et d'autre part le composé de la Préparation 1" utilisé au Stade C par le *N*-(4-{[*tert-*butyl(diméthyl)silyl]oxy}phényl)-1-méthyl-1*H*-indazol-5-amine.

| ***Microanalyse élémentaire :*** | | | | |
|---|---|---|---|---|
| | *%C* | *%H* | %*N* | *%Cl⁻* |
| *Calculé* | *68.65* | *6.02* | *10*.*67* | *4.50* |
| *Trouvé* | *67.91* | *5.52* | *10.53* | *4.08* |

### Exemple 105: Bis(chlorhydrate) de N-(4-hydroxyphényl)-3-(6-{[(3S)-3-{[4-(2-méthoxyéthyl)piperazin-1-yl]méthyl}-3,4-dihydroisoquinolin-2(1H)-yl]carbonyl}-1,3-benzodioxol-5-yl)-N-phényl-5,6,7,8-tétrahydroindolizine-1-carboxamide

On procède selon le procédé de l'Exemple 1 en remplaçant le composé de la Préparation 1' utilisé au Stade A par le composé de la Préparation 9'.

| ***Microanalyse élémentaire :*** | | | |
|---|---|---|---|
| | %*C* | *%H* | %*N* |
| *Calculé* | *65.71* | *6.11* | *8.33* |
| *Trouvé* | *66.57* | *6.16* | *8.44* |

### Exemple 106 : Chlorhydrate de (2S)-5-(5-fluoro-2-{[(3S)-3-(morpholin-4-ylméthyl)-3,4-dihydroisoquinolin-2(1H)-yl]carbonyl}phényl)-2-hydroxy-N-(4-hydroxyphényl)-N-phényl-2,3-dihydro-1H-pyrrolizine-7-carboxamide

On procède selon le procédé de l'Exemple 102 en remplaçant le composé de la Préparation 28 utilisé au Stade A par le composé de la Préparation 29.

| ***Microanalyse élémentaire :*** | | | | |
|---|---|---|---|---|
| | *%C* | *%H* | *%N* | *%Cl⁻* |
| *Calculé* | *68.09* | *5.57* | *7.75* | *4.90* |
| *Trouvé* | *68.16* | *5.13* | *7.74* | *4.97* |

### Exemple 107: Chlorhydrate de 3-(5-chloro-2-{[(3S)-3-[(4-méthyl-3-oxopiperazin-1-yl)méthyl]-3,4-dihydroisoquinolin-2(1H)-yl]carbonyl}phényl)-N-(4-hydroxyphényl)-N-(1-méthyl-1H-indol-5-yl)-5,6,7,8-tétrahydroindolizine-1-carboxamide

On procède selon le procédé de l'Exemple 1 en remplaçant d'une part les composés des Préparations 1 et 1' utilisés au Stade A par les composés respectifs des Préparations 3 et 10', et d'autre part le composé de la Préparation 1" utilisé au Stade C par le *N-*(4-{[*tert-*butyl(diméthyl)silyl]oxy}phényl)-1-méthyl-1*H*-indol-5-amine.

| ***Microanalyse élémentaire :*** | | | | | |
|---|---|---|---|---|---|
| | *%C* | *%H* | *%N* | *%Cl* | *%Cl⁻* |
| *Calculé* | *67.56* | *5.67* | *10.28* | *8.67* | *4.34* |
| *Trouvé* | *67.31* | *5.14* | *10.18* | *8.27* | *3.76* |

### Exemple 108: Chlorhydrate de 3-(5-chloro-2-{[(3S)-3-{[(2-méthoxyéthyl) (méthyl)amino]méthyl}-3,4-dihydroisoquinolin-2(1H)-yl]carbonyl}phényl)-N-(4-hydroxyphényl)-N-(1-méthyl-1H-indol-5-yl)-5,6,7,8-tétrahydroindolizine-1-carboxamide

On procède selon le procédé de l'Exemple 1 en remplaçant d'une part les composés des Préparations 1 et 1' utilisés au Stade A par les composés respectifs des Préparations 3 et 11', et d'autre part le composé de la Préparation 1" utilisé au Stade C par le *N-*(4*-*{[*tert-*butyl(diméthyl)silyl]oxy}phényl)-1-méthyl-1*H*-indol-5-amine.

| ***Microanalyse élémentaire :*** | | | | |
|---|---|---|---|---|
| | *%C* | *%H* | *%N* | *%Cl⁻* |
| *Calculé* | *68.18* | *5.98* | *8.83* | *4.47* |
| *Trouvé* | *68.30* | *5.61* | *8.78* | *4.46* |

### Exemple 109 : N-(4-Hydroxyphényl)-3-(6-{[(3S)-3-[(4-oxidomorpholin-4-yl)méthyl]-3,4-dihydroisoquinolin-2(1H)-yl]carbonyl}-1,3-benzodioxol-5-yl)-N-phényl-5,6,7,8-tétrahydroindolizine-1-carboxamide

A une solution du composé de l'Exemple 1, pris sous la forme de base libre, dans 10 mL de CH₂Cl₂, on ajoute par portion l'acide *méta*-chloroperbenzoïque (0,242 mg ;1,4 mmol). L'ensemble est ensuite agité à température ambiante pendant une nuit. Le milieu réactionnel est ensuite concentré à sec, puis le résidu est purifié par chromatographie flash en phase inverse (gradient : acétonitrile/ eau/ acide trifluoroacétique). On obtient, après concentration, le produit du titre sous la forme d'un solide.

| ***Microanalyse élémentaire :*** | | | |
|---|---|---|---|
| | *%C* | *%H* | *%N* |
| *Calculé* | *71.06* | *5.82* | *7.71* |
| *Trouvé* | *70.59* | *5.36* | *7.69* |

### Masse haute résolution (ESI+) :

Formule brute : C₄₃H₄₂N₄O₇
[M+H]⁺calculé : 727.3132
[M+H]⁺ mesuré : 727.3110

### Exemple 110 : Chlorhydrate de 3-(5-chloro-2-{[(3S)-3-{[éthyl(2-méthoxyéthyl)amino]méthyl}-3,4-dihydroisoquinolin-2(1H)-yl]carbonyl}phényl)-N-(4-hydroxyphényl)-N-(1-méthyl-1H-indol-5-yl)-5,6,7,8-tétrahydroindolizine-1-carboxamide

On procède selon le procédé de l'Exemple 1 en remplaçant d'une part les composés des Préparations 1 et 1' utilisés au Stade A par les composés respectifs des Préparations 3 et 12', et d'autre part le composé de la Préparation 1" utilisé au Stade C par le *N*-(4-{[*tert-*butyl(diméthyl)silyl]oxy}phényl)-1-méthyl-1*H-*indol-5-amine.

| ***Microanalyse élémentaire :*** | | | | |
|---|---|---|---|---|
| | *%C* | *%H* | *%N* | *%Cl⁻* |
| *Calculé* | *68.48* | *6.12* | *8.68* | *4.39* |
| *Trouvé* | *68.40* | *5.78* | *8.61* | *4.23* |

### Exemple 111 : Chlorhydrate de 3-(5-chloro-2-{[(3S)-3-(morpholin-4-ylméthyl)-3,4-dihydroisoquinolin-2(1H)-yl]carbonyl}phényl)-N-(4-fluorophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 4-{[*tert*-butyl(diméthyl)silyl]oxy}-*N-*(4-fluorophényl)aniline.

### Masse haute résolution (ESI+) :

Formule brute : C₄₂H₄₀ClN₄O₄
[M+H]⁺ calculé : 719.2722
[M+H]⁺ mesuré : 719.2806

### Exemple 112 : Chlorhydrate de N-(4-hydroxyphényl)-3-(5-méthoxy-2-{[(3S)-3-(morpholin-4-ylméthyl)-3,4-dihydroisoquinolin-2(1H)-yl]carbonyl}phényl)-N-phényl-5,6,7,8-tétrahydroindolizine-1-carboxamide

On procède selon le procédé de l'Exemple 1 en remplaçant le composé de la Préparation 1 utilisé au Stade A par le composé de la Préparation 6.

| ***Microanalyse élémentaire :*** | | | | |
|---|---|---|---|---|
| | *%C* | *%H* | *%N* | *%Cl⁻* |
| *Calculé* | *70.43* | *6.19* | *7.64* | *4.83* |
| *Trouvé* | *70.17* | *5.79* | *7.60* | *4.69* |

### Exemple 113: Chlorhydrate de 7-hydroxy-N-(4-hydroxyphényl)-3-(6-{[(3S)-3-(morpholin-4-ylméthyl)-3,4-dihydroisoquinolin-2(1H)-yl]carbonyl}-1,3-benzodioxol-5-yl)-N-phényl-5,6,7,8-tétrahydroindolizine-1-carboxamide

### Stade A : 3'-(6-{[(3S)-3-(Morpholin-4-ylméthyl)-3,4-dihydroisoquinolin-2(1H)-yl]carbonyl}-1,3-benzodioxol-5-yl)-5',6',-dihydro-8'H-spiro[1,3-dioxolane-2,7'-indolizine] -1'-carboxylate de méthyle

On procède selon le procédé du Stade A de l'Exemple 1 en remplaçant le composé de la Préparation 1 par le composé de la Préparation 30.

### Stade B : 3-(6-{[(3S)-3-(Morpholin-4 ylméthyl)-3,4-dihydroisoquinolin-2(1H)-yl] carbonyl}-1,3-benzodioxol-5-yl)-7-oxo-5,6,7,8-tétrahydroindolizine-1-carboxylate de méthyle

2,75 g du composé du Stade A (4,47 mmol) en solution dans 75 mL de THF sont agités en présence de 37 mL de HCl 1 M à reflux pendant 15 h. 100 mL d'eau et 100 mL d'acétate d'éthyle sont ajoutés au milieu réactionnel. On ajoute ensuite 4 g de NaHCO₃ (4,7 mmol) en poudre jusqu'à atteindre un pH basique. On extrait le composé avec de l'acétate d'éthyle, la phase organique est séchée sur MgSO_{4,} filtrée et concentrée à sec. Le produit du titre est obtenu sous la forme d'une huile.

**RMN ¹H:** δ (500MHz ; dmso-d6 ; 300°K) : 7.9-7.2 (m, 4H, H aromatiques) ; 7.02 (m, 1H, H aromatiques) ; 6.88 (m, 1H, H aromatiques); 6.44-5.87 (m, 1H, H aromatiques tétrahydroindolizine); 6.17 (d, 2H, CH₂ méthylène dioxy); 5.07/4.85/3.79 (m, 1H, H tertiaire tétrahydroisoquinoline) ; 4.88/4.27/4.24 (m, 2H, H aliphatique tétrahydroisoquinoline) ; 4.22-3.43 (m, 4H, H aliphatique tétrahydroindolizine) ; 3.59-3.49 (m, 4H, H aliphatique morpholine); 3.75-3.52 (s, 3H, Me); 2.93-2.49 (m, 2H, H aliphatique tétrahydroindolizine) ; 2.75-2.28 (m, 2H, H aliphatique tétrahydroindolizine) ; 2.68-1.68 (m, 6H, H aliphatique morpholine + CH₂)

### Stade C: 7-Hydroxy-3-(6-{[(3S)-3-(morpholin-4-ylméthyl)-3,4-dihydroisoquinolin - 2(1H)-yl]carbonyl}-1,3-benzodioxol-5-yl)-5,6,7,8-tétrahydroindolizine-1-carboxylate de méthyle

A une solution de 2,55 g du composé obtenu au Stade B (4,47 mmol) dans 30 mL de méthanol sont ajoutés par portion 558 mg (14,75 mmol) de borohydrure de sodium. Le milieu réactionnel est agité pendant 1 h à température ambiante. 50 mL de HCl 1M sont ensuite ajoutés et le méthanol est évaporé. La phase aqueuse est ensuite neutralisée avec NaHCO₃, puis extraite avec du dichlorométhane. La phase organique est successivement lavée avec H₂O, séchée sur MgSO_{4,} filtrée et concentrée à sec. L'huile ainsi obtenue est purifiée par chromatographie flash (gradient dichlorométhane/éthanol-ammoniac). Le produit du titre est obtenu sous la forme d'un solide.

**RMN ¹H:** δ (500MHz; dmso-d6 ; 300°K): 7.22-6.97 (m, 4H, H aromatiques tétrahydroisoquinoline); 7.05 (s, 1H, H aromatique) ; 6.89 (s, 1H, H aromatique); 6.37/6.3/6.07 (m, 1H, H aromatiques tétrahydroindolizine) ; 6.16 (d, 2H, CH₂ méthylène dioxy) ; 5.09 (m, 1H, H tertiaire tétrahydroisoquinoline) ; 4.87-4.21 (m, 2H, H aliphatique tétrahydroindolizine); 4.20-3.67 (m, 2H, H aliphatique tétrahydroindolizine); 4.10-3.86 (m, 1H, H tertiaire tétrahydroindolizine) ; 3.69-3.58 (s, 3H, Me) ; 3.69-3.52 (m, 4H, H aliphatique morpholine) ; 2.96 + 2.43 (m, 2H, H aliphatique tétrahydroisoquinoline) ; 2.55-2.0 (m, 6H, H aliphatique morpholine + CH₂); 2.4-1.5 (m, 4H, H aliphatique tétrahydroindolizine)
**IR :** OH : 3239 cm⁻¹ ; -C=O (ester) : 1696 cm⁻¹ ; -C=O (amide) : 1624 cm⁻¹ ; C-C-OH (alcool secondaire) : 1034 cm⁻¹

### Stade D : 3-(6-{[(3S)-3-(Morpholin-4-ylméthyl)-3,4-dihydroisoquinolin-2(1H)-yl]carbonyl}-1,3-benzodioxol-5-yl)-7-(prop-2-én-1-yloxy)-5,6,7,8-tétrahydroindolizine-1-carboxylate de méthyle

A une suspension de 331 mg (8,26 mmol) d'hydrure de sodium dans 15 mL de THF anhydre refroidi à 0°C, on ajoute 2,37g (4,13 mmol) du composé obtenu au Stade C. Cette suspension est agitée pendant 15 min à 0°C, puis une solution de 790 (µL (9,1 mmol) de bromure d'allyle dans 10 mL de THF est additionnée lentement (sur 15 min). Le milieu réactionnel est agité pendant 1h à 0°C, puis 15 h à température ambiante. Cette solution est hydrolysée avec une solution aqueuse saturée avec NH₄Cl. On extrait le composé avec de l'acétate d'éthyle ; la phase organique est séchée sur MgSO_{4,} filtrée et concentrée à sec. L'huile ainsi obtenue est purifiée par chromatographie flash (gradient cyclohexane/acétate d'éthyle). Le produit du titre est obtenu sous la forme d'un solide.

**RMN ¹H:** δ (500 MHz ; dmso-d6 ; 300°K): 7.2-6.9 (m, 4H, H aromatiques tétrahydroisoquinoline) ; 7.2-6.8 (m, 2H, H aromatiques) ; 6.4-6.0 (m, 1H, H aromatiques tétrahydroindolizine); 6.10 (d, 2H, CH₂ méthylène dioxy); 5.9 (m, 1H, allyl) ; 5.35-5.10 (m, 2H, allyle) ; 5.1+4.75 (m, 1H, H tertiaire tétrahydroisoquinoline); 4.15-3.9 (m, 2H, CH₂ allyle) ; 3.9-3.6 (m, 1H, H tertiaire tétrahydroindolizine) ; 4.1-3.4 (m, 4H, H aliphatique morpholine) ; 4.9-3.4 (m, 4H, 2H aliphatiques tétrahydroindolizine + 2H aliphatiques tétrahydroisoquinoline); 3.8-3.6 (s, 3H, Me) ; 2.55-1.6 (m, 6H, H aliphatique morpholine + CH₂) ; 3.3-1.5 (m, 6H, 4H aliphatiques tétrahydroindolizine + 2H aliphatiques tétrahydroisoquinoline)

### Stade E : N-(4-{[tert-Butyl(diméthyl)silyl]oxy}phényl)-3-(6-{[(3S)-3-(morpholin-4-ylméthyl)-3,4-dihydroisoquinolin-2(1H)-yl]carbonyl}-1,3-benzodioxol-5-yl)-N-phényl-7-(prop-2-en-1-yloxy)-5,6,7,8-tétrahydroindolizine-1-carboxamide

On procède selon les procédés des Stades B et C de l'Exemple 1.

### Stade F: N-(4-{[tert-Butyl(diméthyl)silyl]oxy}phényl)-7-hydroxy-3-(6-{[(3S)-3-(morpholin-4-ylméthyl)-3,4-dihydroisoquinolin-2(1H)-yl]carbonyl}-1,3-benzodioxol-5-yl)-N-phényl-5,6,7,8-tétrahydroindolizine-1-carboxamide

On réalise ensuite une réaction de déprotection du groupement allyle en présence de 1,3-diméthylpyrimidine-2,4,6(1*H*,3*H,*5*H*)-trione (aussi appelé diméthylbarbiturate) et de tétrakis(triphénylphosphine)palladium dans un mélange de méthanol et de dichlorométhane.

### Stade G : Chlorhydrate de 7-hydroxy-N-(4-hydroxyphényl)-3-(6-{[(3S)-3-(morpholin-4-ylméthyl)-3,4-dihydroisoquinolin-2(1H)-yl]carbonyl}-1,3-benzodioxol-5-yl)-N-phényl-5,6,7,8-tétrahydroindolizine-1-carboxamide

La déprotection du groupement silyloxy est réalisée selon le procédé du Stade D de l'Exemple 1.

| ***Microanalyse élémentaire :*** | | | | |
|---|---|---|---|---|
| | *%C* | *%H* | *%N* | *%Cl⁻* |
| *Calculé* | *67.66* | *5.68* | *7.34* | *4.64* |
| *Trouvé* | *67.02* | *5.27* | *7.36* | *4.61* |

### Masse haute résolution (ESI+) :

Formule brute : C₄₃H₄₃N₄O₇
[M+H]⁺ calculé : 727.3132
[M+H]⁺ mesuré : 727.3121

### Exemple 114 : Chlorhydrate de N-(3-fluoro-4-méthylphényl)-N-(4-hydroxyphényl)-3-(5-méthoxy-2-{[(3S)-3-(morpholin-4-ylméthyl)-3,4-dihydroisoquinolin-2(1H)-yl] carbonyl}phényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 6, et d'autre part le composé de la Préparation 1" utilisé au Stade C par le *N*-(4-{[*tert-*butyl(diméthyl)silyl]oxy}phényl)-3-fluoro-4-méthylaniline.

| ***Microanalyse élémentaire :*** | | | | |
|---|---|---|---|---|
| | *%C* | *%H* | *%N* | *%Cl⁻* |
| *Calculé* | *69.05* | *6.06* | *7.32* | *4.63* |
| *Trouvé* | *68.90* | *5.56* | *7.33* | *4.41* |

### Exemple 115 : Chlorhydrate de 3-(5-chloro-2-{[(3S)-3-(morpholin-4-ylméthyl)-3,4-dihydroisoquinolin-2(1H)-yl]carbonyl}phényl)-N-(2-fluorophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 4-{[*tert*-butyl(diméthyl)silyl]oxy}-*N-*(2-fluorophényl)aniline.

### Masse haute résolution (ESI+) :

Formule brute : C₄₂H₄₀ClN₄O₄
[M+H]⁺ calculé: 719.2722
[M+H]⁺ mesuré: 719.2802

### Exemple 116 : Chlorhydrate de 3-(5-chloro-2-([(3S)-3-(morpholin-4-ylméthyl)-3,4-dihydroisoquinolin-2(1H)-yl]carbonyl}phényl)-N-(3-fluorophényl)-N-(4)-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 4-{[*tert*-butyl(diméthyl)silyl]oxy}-*N*-(3-fluorophényl)aniline.

### Masse haute résolution (ESI+) :

Formule brute : C₄₂H₄₀ClN₄O₄
[M+H]⁺ calculé: 719.2722
[M+H]⁺ mesuré: 719.2819

### Exemple 117 : Chlorhydrate de 3-(5-chloro-2-{([(3S)-3-(morpholin-4-ylméthyl)-3,4-dihydroisoquinolin-2(1H)-yl]carbonyl}phényl)-N-(2,4-difluorophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl (diméthyl)silyl]oxy}phényl)-2,4-difluoroaniline.

### Masse haute résolution (ESI+) :

Formule brute : C₄₂H₃₉ClF₂N₄O₄
[M+H]⁺ calculé : 737.2628
[M+H]⁺ mesuré : 737.2660

### Exemple 118: Chlorhydrate de 3-(5-chloro-2-{[(3S)-3-(morpholin-4-ylméthyl)-3,4-dihydroisoquinolin-2(1H)-yl]carbonyl}phényl)-N-(3,4-difluorophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-3,4-difluoroaniline.

| ***Microanalyse élémentaire :*** | | | | |
|---|---|---|---|---|
| | *%C* | *%H* | *%N* | *%Cl⁻* |
| *Calculé* | *65.20* | *5.21* | *7.24* | *4.58* |
| *Trouvé* | *65.26* | *5.01* | *6.90* | *4.57* |

### Exemple 119 : Chlorhydrate de 3-(5-chloro-2-{[(3S)-3-(morpholin-4-ylméthyl)-3,4-dihydroisoquinolin-2(1H)-yl]carbonyl}phényl)-N-(3-cyanophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 3-[(4-{[*tert-*butyl(diméthyl)silyl]oxy}phényl)amino]benzonitrile.

| ***Microanalyse élémentaire :*** | | | | |
|---|---|---|---|---|
| | %*C* | %*H* | *%N* | *%Cl⁻* |
| *Calculé* | *67.71* | *5.42* | *9.18* | 4.65 |
| *Trouvé* | *67.00* | *5.20* | *8.89* | *4.54* |

### Exemple 120 : Chlorhydrate de N-(4-hydroxyphényl)-6-(6-{[(3S)-3-(morpholin-4-ylméthyl)-3,4-dihydroisoquinolin-2(1H)-yl]carbonyl}-1,3-benzodioxol-5-yl)-N phénylpyrrolo[1,2-a]pyrazine-5-carboxamide

On procède selon le procédé de l'Exemple 1 en remplaçant le composé de la Préparation 1 utilisé au Stade A par le composé de la Préparation 31.

### Masse haute résolution (ESI+) :

Formule brute : C₄₂H₃₇N₅O₆
[M+H]⁺ calculé: 708.2822
[M+H]⁺ mesuré : 708.2788

### Exemple 121 : Chlorhydrate de N-(3-fluorophényl)-N-(4-hydroxyphényl)-6-(6-{[(3S)-3-(morpholin-4-ylméthyl)-3,4-dihydroisoquinolin-2(1H)-yl]carbonyl}-1,3-benzodioxol-5-yl)pyrrolo[1,2-a]pyrazine-8-carboxamide

On procède selon le procédé de l'Exemple 1 en remplaçant d'une part le composé de la Préparation 1 utilisé au Stade A par le composé de la Préparation 31, et d'autre part le composé de la Préparation 1" utilisé au Stade C par le 4-{[*tert*-butyl(diméthyl)silyl]oxy}-N-(3-fluorophényl)aniline.

### Masse haute résolution (ESI+) :

Formule brute : C₄₂H₃₆FN₅O₆
[M+H]⁺ calculé : 726.2728
[M+H]⁺ mesuré : 726.2723

### Exemple 122 : Chlorhydrate de N-(3-fluorophényl)-N-(4-hydroxyphényl)-3-(6-{[(3S)-3-(morpholin-4-ylméthyl)-3,4-dihydroisoquinolin-2(1H)-yl]carbonyl}-1,3-benzodioxol -5-yl)-5,6,7,8-tétrahydroindolizine-1-carboxamide

On procède selon le procédé de l'Exemple 1 en remplaçant le composé de la Préparation 1" utilisé au Stade C par le 4-{[*tert*-butyl(diméthyl)silyl]oxy}-*N*-(3-fluorophényl)aniline.

### Masse haute résolution (ESI+) :

Formule brute : C₄₃H₄₁FN₄O₆
[M+H]⁺ calculé: 729.3088
[M+H]⁺ mesuré : 729.3068

### Exemple 123 : Chlorhydrate de N-(4-hydroxyphényl)-3-(6-{[(3S)-3-{[4-(méthylsulfonyl)piperazin-1-yl]méthyl}-3,4-dihydroisoquinolin-2(1H)yl]carbonyl}-1,3-benzodioxol-5-yl)-N-phényl-5,6,7,8-tétrahydroindolizine-1-carboxamide

On procède selon le procédé de l'Exemple 1 en remplaçant le composé de la Préparation 1' utilisé au Stade A par le composé de la Préparation 13'.

| ***Microanalyse élémentaire :*** | | | | | |
|---|---|---|---|---|---|
| | *%C* | *%H* | *%N* | %*S* | *%Cl⁻* |
| *Calculé* | *64.11* | *5*.*62* | *8.50* | *3*.*89* | *4.30* |
| *Trouvé* | *64.19* | *5*.*07* | *8.52* | *3*.*87* | *4.02* |

### Exemple 124 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(3-méthoxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-3-méthoxyaniline.

| ***Microanalyse élémentaire :*** | | | | |
|---|---|---|---|---|
| | *%C* | *%H* | *%N* | *%Cl⁻* |
| *Calculé* | *67.27* | *5.78* | *7.30* | *4.62* |
| *Trouvé* | *67.54* | *5.35* | *7.32* | *4.62* |

### Exemple 125 : Chlorhydrate de 3-(5-chloro-2-{[(3S)-3-(morpholin-4-ylméthyl)-3,4-dihydroisoquinolin-2(1H)-yl]carbonyl}phényl)-N-(3,5-difluorophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-3,5-difluoroaniline.

| ***Microanalyse élémentaire :*** | | | | |
|---|---|---|---|---|
| | *%C* | *%H* | *%N* | *%Cl⁻* |
| *Calculé* | *65.20* | *5.21* | *7.24* | *4.58* |
| *Trouvé* | *65.85* | *4.93* | *7.04* | *4.76* |

### Exemple 126: Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(3-méthyl phényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-3-méthylaniline.

| ***Microanalyse élémentaire :*** | | | | |
|---|---|---|---|---|
| | *%C* | *%H* | *%N* | *%Cl⁻* |
| *Calculé* | *68.70* | *5.90* | *7.45* | *4.72* |
| *Trouvé* | *68.94* | *5.72* | *7.21* | *4*.*84* |

### Exemple 127 : Chlorhydrate de N-(4-hydroxyphényl)-N-phényl-3-(6-{[(3S)-3-{[4-(2,2,2-trifluoroéthyl)piperazin-1-yl]méthyl}-3,4-dihydroisoquinolin-2(1H)-yl] carbonyl}-1,3-benzodioxol-5-yl)-5,6,7,8-tétrahydroindolizine-1-carboxamide

On procède selon le procédé de l'Exemple 1 en remplaçant le composé de la Préparation 1' utilisé au Stade A par le composé de la Préparation 14'.

| ***Microanalyse élémentaire :*** | | | | |
|---|---|---|---|---|
| | *%C* | *%H* | *%N* | *%Cl⁻* |
| *Calculé* | *65.25* | *5.48* | *8.45* | *4.28* |
| *Trouvé* | *64.91* | *5.23* | *8.37* | *4.96* |

### Exemple 128: Chlorhydrate de N-(4-hydroxyphenyl)-3-(6-{[(3S)-3-[{4-méthyl-3-oxopiperazin-1-yl)méthyl]-3,4-dihydroisoquinolin-2(1H)-yl]carbonyl}-1,3-benzodioxol-5-yl)-N-phényl-5,6,7,8-tétrahydroindolizine-1-carboxamide

On procède selon le procédé de l'Exemple 1 en remplaçant le composé de la Préparation 1' utilisé au Stade A par le composé de la Préparation 10'.

| ***Microanalyse élémentaire :*** | | | | |
|---|---|---|---|---|
| | *%C* | *%H* | *%N* | *%Cl⁻* |
| *Calculé* | *68.25* | *5.73* | *9.04* | *4.58* |
| *Trouvé* | *68.04* | *5. 09* | *8.82* | *4.64* |

### Exemple 129 : Chlorhydrate de 3-(5-chloro-2-{[(3S)-3-(morpholin-4-ylméthyl)-3,4-dihydroisoquinolin-2(1H)-yl]carbonyl}phényl)-N-(4-cyanophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 4-(4-{[*tert-*butyl(diméthyl)silyl]oxy}phényl)amino]benzonitrile.

| ***Microanalyse élémentaire :*** | | | |
|---|---|---|---|
| | *%C* | *%H* | *%N* |
| *Calculé* | *67.71* | *5.42* | *9.18* |
| *Trouvé* | *68.17* | *5.15* | *8.71* |

### Exemple 130: Chlorhydrate de 3-(5-chloro-2-{[(3S)-3-(morpholin-4-ylméthyl)-3,4-dihydroisoquinolin-2(1H)-yl]carbonyl}phényl)-N,N-diphényl-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par la *N*-phénylaniline.

### Masse haute résolution (ESI+) :

Formule brute : C₄₂H₄₀Cl₂N₄O₄
[M+H]⁺ calculé : 735.2427
[M+H]⁺ mesuré : 735.2524

### Exemple 131 : Chlorhydrate de 3-(5-chloro-2-{[(3S)-3-(morpholin-4-ylméthyl)-3,4-dihydroisoquinolin-2(1H)-yl]carbonyl}phényl)-N-(3-chlorophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxylphényl)-3-chloroaniline.

| ***Microanalyse élémentaire :*** | | | | |
|---|---|---|---|---|
| | *%C* | *%H* | *%N* | *%Cl⁻* |
| *Calculé* | *65.33* | *5.35* | *7.26* | *4.59* |
| *Trouvé* | *64.08* | *5.29* | *6.92* | *4.59* |

### Exemple 132 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(pyrimidin-2-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)pyrimidin-2-amine.

| ***Microanalyse élémentaire :*** | | | |
|---|---|---|---|
| | *%C* | *%H* | %*N* |
| *Calculé* | *64.95* | *5.45* | *11.36* |
| *Trouvé* | 64.62 | *5.07* | *10.92* |

### Exemple 133 : Chlorhydrate de 3-(5-chloro-2-{[(3S)-3-(morpholin-4-ylméthyl)-3,4-dihydroisoquinolin-2(1H)-yl]carbonyl}phényl)-N-(cyclobutylméthyl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 4-{[*tert*-butyl(diméthyl)silyl]oxy}-*N*-(cyclobutylméthyl)aniline.

| ***Microanalyse élémentaire :*** | | | | |
|---|---|---|---|---|
| | %*C* | *%H* | *%N* | *%Cl⁻* |
| *Calculé* | *68.16* | *6.39* | *7.76* | *3.93* |
| *Trouvé* | *68.69* | *5.93* | *7.45* | *3.81* |

### Exemple 134 : Chlorhydrate de 3-{5-chloro-2-{[(3S)-3-(morpholin-4-ylméthyl)-3,4-dihydroisoquinolin-2(1H)-yl]carbonyl}phényl}-N-(2-cyanophényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 2-[(4-{[*tert-*butyl(diméthyl)silyl]oxy}phényl)amino]benzonitrile.

| ***Microanalyse élémentaire :*** | | | |
|---|---|---|---|
| | *%C* | *%H* | %*N* |
| *Calculé* | *67.71* | 5.42 | *9.18* |
| *Trouvé* | *67.34* | *4.95* | *8.73* |

### Exemple 135 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(1-méthyl-1H-pyrazol-4-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-1-méthyl-1*H*-pyrazol-4-amine.

| ***Microanalyse élémentaire :*** | | | | |
|---|---|---|---|---|
| | %*C* | %*H* | *%N* | *%Cl⁻* |
| *Calculé* | *64.77* | *5.71* | *11.33* | *4.78* |
| *Trouvé* | *64.62* | *5.33* | *10.71* | *4.10* |

### Exemple 136 : Chlorhydrate de 3-(5-chloro-2-{[(3S)-3-(morpholin-4-ylméthyl)-3,4-dihydroisoquinolin-2(1H)-yl]carbonyl}phényl)-N-(cyclopropylméthyl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 4-{[*tert*-butyl(diméthyl)silyl]oxy}-*N*-(cyclopropylmethyl)aniline.

| ***Microanalyse élémentaire :*** | | | | |
|---|---|---|---|---|
| | %C | *%H* | %*N* | *%Cl⁻* |
| *Calculé* | *67.13* | *6.20* | *7.83* | *4.95* |
| *Trouvé* | *67.58* | *5.79* | *7.36* | *4.16* |

### Exemple 137 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(1-méthyl-1H-pyrazol-3-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-1-méthyl-1*H*-pyrazol-3-amine.

| ***Microanalyse élémentaire :*** | | | | |
|---|---|---|---|---|
| | *%C* | *%H* | %*N* | *%Cl⁻* |
| *Calculé* | *64.77* | *5.71* | *11.33* | *4.78* |
| *Trouvé* | *64.20* | *5.47* | *10.78* | *5.27* |

### Exemple 138 : Chlorhydrate de N-(but-2-yn-1-yl)-3-(5-chloro-2-{[(3S)-3-(morpholin-4-ylméthyl)-3,4-dihydroisoquinolin-2(1H)-yl]carbonyl}phényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 4-{[*tert*-butyl(diméthyl)silyl]oxy}-*N*-(but-2-yn-1-yl)aniline.

| ***Microanalyse élémentaire :*** | | | | |
|---|---|---|---|---|
| | *%C* | *%H* | *%N* | *%Cl⁻* |
| *Calculé* | *64.95* | *5.45* | *11.36* | *4.79* |
| *Trouvé* | *65.53* | *5.19* | *10.88* | *5.38* |

### Exemple 139 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(pyridin-2-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N-(*4-{[*tert-*butyl(diméthyl)silyl]oxy}phényl)pyridin-2-amine.

| ***Microanalyse élémentaire :*** | | | |
|---|---|---|---|
| | *%C* | *%H* | %*N* |
| *Calculé* | *66.66* | *5*.*59* | *9*.*48* |
| *Trouvé* | *67*.*12* | *5.37* | *9.11* |

### Exemple 140 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(pyridazin-3-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)pyridazin-3-amine.

### Masse haute résolution (ESI+) :

Formule brute : C₄₂H₃₉ClN₆O₄
[M+H]⁺ calculé: 703.2721
[M+H]⁺ mesuré: 703.2783

### Exemple 141 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(pyrimidin-5-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)pyrimidin-5-amine.

| ***Microanalyse élémentaire :*** | | | | |
|---|---|---|---|---|
| | *%C* | *%H* | *%N* | *%Cl⁻* |
| *Calculé* | *68.32* | *5*.*59* | *11.95* | *5.04* |
| *Trouvé* | *68.05* | *5.52* | *11.83* | *5.50* |

### Exemple 142 : Bis(chlorhydrate) de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(pyridin-3-yl)-5,6,7,5-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N-(*4-{[*tert-*butyl(diméthyl)silyl]oxy}phényl)pyridin-3-amine,

### Masse haute résolution (ESI+) :

Formule brute : C₄₁H₄₀ClN₅O₄
[M+H]⁺ calculé : 702.2769
[M+H]⁺ mesuré : 702.2858

### Exemple 143 : Chlorhydrate de 3-(5-chloro-2-{[(3S)-3-(morpholin-4-ylméthyl)-3,4-dihydroisoquinolin-2(1H)-yl]carbonyl}phényl)-N-(3,5-difluoro-4-méthoxyphényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-3,5-difluoro-4-méthoxyaniline.

### Masse haute résolution (ESI+) :

Formule brute : C₄₃H₄₁ClF₂N₄O₅
[M+H]⁺ calculé : 767.2734
[M+H]⁺ mesuré : 767.2804

### Exemple 144 : Bis(chlorhydrate) de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(pyridin-4-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)pyridin-4-amine.

### Masse haute résolution (ESI+) :

Formule brute : C₄₁H₄₀ClN₅O₄
[M+H]⁺ calculé : 702.2842
[M+H]⁺ mesuré : 702.2842

### Exemple 145 : Chlorhydrate de 3-(5-chloro-2-{[(3S)-3-(morpholin-4-ylméthyl)-3,4-dihydroisoquinolin-2(1H)-yl]carbonyl}phényl)-N-(3-fluoro-4-méthoxyphényi)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N-*(4-{[*tert-*butyl(diméthyl)silyl]oxy}phényl)-3-fluoro-4-méthoxyaniline.

### Masse haute résolution (ESI+) :

Formule brute : C₄₃H₄₂ClFN₄O₅
[M+H]⁺ calculé : 749.2828
[M+H]⁺ mesuré : 749.2878

### Exemple 146 : Chlorhydrate de N-(4-hydroxyphényl)-3-(6-{[(3S)-3-{[(2-méthoxyéthyl)(méthyl)amino]méthyl}-3,4-dihydroisoquinolin-2(1H)-yl]carbonyl}-1,3-benzodioxol-5-yl)-N-phényl-5,6,7,8-tétrahydroindolizine-1-carboxamidc

On procède selon le procédé de l'Exemple 1 en remplaçant le composé de la Préparation 1' utilisés au Stade A par le composé de la Préparation 11'.

| ***Microanalyse élémentaire :*** | | | | |
|---|---|---|---|---|
| | *%C* | *%H* | *%N* | *%Cl⁻* |
| *Calculé* | *68.93* | *6.05* | *7.48* | *4.73* |
| *Trouvé* | *68*.*84* | *5.85* | *7.56* | *4.60* |

### Exemple 147: Chlorhydrate de 3-{6-{[(3S)-3-[(1,1-dioxidothiomorpholin-4-yl)méthyl]-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

On procède selon le procédé de l'Exemple 1 en remplaçant le composé de la Préparation 1' utilisés au Stade A par le composé de la Préparation 15'.

| ***Microanalyse élémentaire :*** | | | | | |
|---|---|---|---|---|---|
| | *%C* | *%H* | *%N* | %*S* | *%Cl⁻* |
| *Calculé* | *64.94* | *5.45* | *7.04* | *4.03* | *4.46* |
| *Trouvé* | *65.27* | *5.13* | 7.14 | *3.90* | *4.30* |

### Exemple 148 : Trifluoroacétate de N-(5-hydroxypyrimidin-2-yl)-3-(6-{[(3S)-3-(morpholin-4-ylméthyl)-3,4-dihydroisoquinolin-2(1H)-yl]carbonyl}-1,3-benzodioxol-5-yl)-N-phényl-5,6,7,8-tétrahydroindolizine-1-carboxamide

### Stade A : N-[5-(benzyloxy)pyrimidin-2-yl]-3-(6-{[(3S)-3-(morpholin-4-ylméthyl)-3,4-dihydroisoquinolin-2(1H)-yl]carbonyl}-1,3-benzodioxol-5-yl)-N-phényl-5,6,7,8-tétrahydroindolizine-1-carboxamide

On procède selon les Stades A, B et C du procédé de l'Exemple 1 en remplaçant le composé de la Préparation 1 " utilisé au Stade C par la 5-(benzyloxy)-*N*-phénylpyrimidin-2-amine.

### Stade B : Trifluoroacétate de N-(5-hydroxypyrimidin-2-yl)-3-(6-{[(3S)-3-(morpholin-4-ylméthyl)-3,4-dihydroisoquino/in-2(1H)-yl]carbonyl}-1,3-benzodioxol-5-y/)-N-phényl-5,6,7,8-tétrahydroindo/izine-1-carboxamide

Le composé du Stade A (150 mg ; 0.2 mmol) est mis en solution dans 8 mL de méthanol, et 30 mg de Pd/C (10% massique de palladium) sont ajoutés. Le milieu réactionnel est laissé sous agitation sous atmosphère d'hydrogène (1,2 bar) pendant 15 h, puis filtré sur Whatman, concentré sous vide et purifié par chromatographie en phase inverse (gradient : acétonitrile/eau en présence d'acide trifluoroacétique). On obtient le produit désiré sous la forme d'un sel de trifluoroacétate.

### Masse haute résolution (ESI+) :

Formule brute : C₄₁H₄₀N₆O₆
[M+H]⁺ calculé : 713.3082
[M+H]⁺ mesuré : 713.3080

### Exemple 149 : Chlorhydrate de N-(4-hydroxyphényl)-3-(6-{[(3S)-3-[(3-méthoxypyrrolidin-1-yl)méthyl]-3,4-dihydroisoquinolin-2(1H)-yl]carbonyl}-1,3-benzodioxol-5-yl)-N-phényl-5,6,7,8-tétrahydroindolizine-1-carboxamide

On procède selon le procédé de l'Exemple 1 en remplaçant le composé de la Préparation 1' utilisé au Stade A par le composé de la Préparation 16'.

| ***Microanalyse élémentaire:*** | | | | |
|---|---|---|---|---|
| | %*C* | *%H* | %*N* | *%Cl⁻* |
| *Calculé* | *69*.*42* | *5.96* | *7.36* | *4.66* |
| *Trouvé* | *70.19* | *5.48* | *7.22* | *4.53* |

### Exemple 150 : Chlorhydrate de 3-(5-chloro-2-{[(3S)-3-(morpholin-4-ylméthyl)-3,4-dihydroisoquinolin-2(1H)-yl]carbonyl}phényl)-N-(3-cyano-4-méthoxyphényl)-N-(4-hydroxyphényl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le 5-[(4-{[*tert-*butyl(diméthyl)silyl]oxy}phényl)amino]-2-méthoxybenzonitrile.

### Masse haute résolution (ESI+) :

Formule brute : C₄₄H₄₂ClN₅O₅
[M+H]⁺ calculé : 756.2874
[M+H]⁺ mesuré: 756.2917

### Exemple 151 : Chlorhydrate de N-(4-hydroxyphényl)-3-(5-méthoxy-2-{[(3S)-3-(morpholin-4-ylméthyl)-3,4-dihydroisoquinolin-2(1H)-yl]carbonyl}phényl)-N-(1-méthyl-1H-pyrazol-4-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 6, et d'autre part le composé de la Préparation 1" utilisé au Stade C par le N-(4-{[*tert-*butyl(diméthyl)silyl]oxy}phényl)-1-méthyl-1*H*-pyrazol-4-amine.

### Masse haute résolution (ESI+) :

Formule brute : C₄₁H₄₄N₆O₅
[M+H]⁺ calculé : 701.3446
[M+H]⁺ mesuré: 701.3446

### Exemple 152 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-[3-(trifluorométhyl)phényl]-5,6,7,8-tétrahydroindolizine-1-carboxamide

On procède selon le procédé de l'Exemple 1 en remplaçant d'une part le composé de la Préparation 1 utilisé au Stade A par le composé de la Préparation 3, et d'autre part le composé de la Préparation 1" utilisé au Stade C par le *N*-(4-{[*tert-*butyl(diméthyl)silyl]oxy}phényl)-3-(trifluorométhyl)aniline.

### Masse haute résolution (ESI+) :

Formule brute : C₄₃H₄₀ClF₃N₄O₄
[M+H]⁺ calculé : 769.2690
[M+H]⁺ mesuré : 769.2718

### Exemple 153 : Chlorhydrate de 3-(6-{[(3S)-3-[(3,3-difluoropyrrolidin-1-yl)méthyl]-3,4-dihydroisoquinolin-2(1H)-yl]carbonyl}-1,3-benzodioxol-5-yl)-N-(4-hydroxyphényl)-N-phényl-5,6,7,8-tétrahydroindolizine-1-carboxamide

On procède selon le procédé de l'Exemple 1 en remplaçant le composé de la Préparation 1' utilisé au Stade A par le composé de la Préparation 17'.

| ***Microanalyse élémentaire :*** | | | |
|---|---|---|---|
| | *%C* | *%H* | %*N* |
| *Calculé* | *67.31* | *5.39* | *7.30* |
| *Trouvé* | *68*.*07* | *5.60* | *7.23* |

### Exemple 154 : Chlorhydrate de N-(4-hydroxyphényl)-N-(1-méthyl-1H-pyrazol-4-yl)-3-(7-{[(3S)-3-(morpholin-4-ylméthyl)-3,4-dihydroisoquinolin-2(1H)-yl]carbonyl}-2,3-dihydro-1,4-benzodioxin-6-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 7, et d'autre part le composé de la Préparation 1" utilisé au Stade C par le *N*-(4-{[*tert-*butyl(diméthyl)silyl]oxy}phényl)-1-méthyl-1H-pyrazol-4-amine.

### Exemple 155 : Chlorhydrate de N-(4-hydroxyphényl)-3-(7-{[(3S)-3-(morpholin-4-ylméthyl)-3,4-dihydroisoquinolin-2(1H)-yl]carbonyl}-2,3-dihydro-1,4-benzodioxin-6-yl)-N-(pyrimidin-5-yl)-5,6,7,8-tétrahydroindolizme-1-carboxamide

On procède selon le procédé de l'Exemple 1 en remplaçant d'une part le composé de la Préparation 1 utilisé au Stade A par le composé de la Préparation 7, et d'autre part le composé de la Préparation 1" utilisé au Stade C par le *N*-(4-{[*tert-*butyl(diméthyl)silyl]oxy}phényl)pyrimidin-5-amine.

### Exemple 156 : N-(4-Hydroxyphényl)-3-(6-{[(3S)-3-[(3-méthoxyazétidin-1-yl)méthyl]-3,4-dihydroisoquinolin-2(1H)-yl]carbonyl}-1,3-benzodioxol-5-yl)-N-phényl-5,6,7,8-tétrahydroindolizine-1-carboxamide

On procède selon le procédé de l'Exemple 1 en remplaçant le composé de la Préparation 1' utilisé au Stade A par le composé de la Préparation 18'.

| ***Microanalyse élémentaire :*** | | | |
|---|---|---|---|
| | *%C* | *%H* | *%N* |
| *Calculé* | *72.66* | *5.96* | *7.88* |
| *Trouvé* | *72.32* | *5.51* | *7.96* |

### Masse haute résolution (ESI+) :

Formule brute : C₄₃H₄₂N₄O₆
[M+H]⁺ calculé : 711.3177
[M+H]⁺ mesuré: 711.3178

### Exemple 157 : Chlorhydrate de 3-(6-{[(3S)-3-[(3-fluoroazétidin-1-yl)méthyl]-3,4-dihydroisoquinolin-2(1H)-yl]carbonyl}-1,3-benzodioxol-5-yl)-N-(4-hydroxyphényl)-N-phényl-5,6,7,8-tétrabydroindolizine-1-carboxamide

On procède selon le procédé de l'Exemple 1 en remplaçant le composé de la Préparation 1' utilisé au Stade A par le composé de la Préparation 19'.

### Exemple 158 : Chlorhydrate de 3-(5-fluoro-2-{[(3S)-3-(morpholin-4-ylméthyl)-3,4-dihydroisoquinolin-2(1H)-yl]carbonyl}phényl)-N-(4-hydroxyphényl)-N-(1-methyl-1H-pyrazol-4-yl)-5,6,7,8-tétrahydroindolizine-1-carboxamide

On procède selon le procédé de l'Exemple 1 en remplaçant d'une part le composé de la Préparation 1 utilisé au Stade A par le composé de la Préparation 4, et d'autre part le composé de la Préparation 1" utilisé au Stade C par le *N*-(4-{[*tert-*butyl(diméthyl)silyl]oxy}phényl)-1-méthyl-1*H*-pyrazol-4-amine.

### Exemple 159: Chlorhydrate de 3-(5-fluoro-2-{[(3S)-3-(morpholin-4-ylméthyl)-3,4-dihydroisoquinolin-2(1H)-yl]carbonyl}phényl)-N-(4-hydroxyphényl)-N-(1-méthyl-1H-pyrazol-4-yl)indolizine-1-carboxamide

On procède selon le procédé de l'Exemple 1 en remplaçant d'une part le composé de la Préparation 1 utilisé au Stade A par le composé de la Préparation 32, et d'autre part le composé de la Préparation 1" utilisé au Stade C par le *N*-(4-{[*tert-*butyl(diméthyl)silyl]oxy}phényl)-1-méthyl-1*H*-pyrazol-4-amine.

### Exemple 160 : Chlorhydrate de 3-(5-chloro-2-{[(3S)-3-(morpholin-4-ylméthyl)-3,4-dihydroisoquinolin-2(1H)-yl]carbonyl}phényl)-N-(4-hydroxyphényl)-N-(1-méthyl-1H-pyrazol-4-yl)indolizine-1-carlnoxamide

On procède selon le procédé de l'Exemple 1 en remplaçant d'une part le composé de la Préparation 1 utilisé au Stade A par le composé de la Préparation 14, et d'autre part le composé de la Préparation 1" utilisé au Stade C par le *N*-(4-{[*tert-*butyl(diméthyl)silyl]oxy}phényl)-1-méthyl-1*H*-pyrazol-4-amine.

### ETUDE PHARMACOLOGIQUE

### EXEMPLE A : Inhibition de Bcl-2 par la technique de polarisation de fluorescence

Les essais de polarisation de fluorescence ont été réalisés en microplaques (384 puits). La protéine Bcl-2, à la concentration finale de 2,50.10⁻⁸M, est mélangée avec un peptide fluorescent (Fluorescein-REIGAQLRRMADDLNAQY), à la concentration finale de 1,00. 10⁻⁸M dans une solution tampon (Hepes 10 mM, NaCl 150 mM, Tween20 0.05%, pH 7.4), en présence ou en absence de concentrations croissantes de composés à tester. Après incubation de 2 heures, la polarisation de fluorescence est mesurée.
Les résultats sont exprimés en IC₅₀ (concentration en composé qui inhibe à 50 % la polarisation de fluorescence) et sont présentés dans le tableau 1 ci-dessous.

Les résultats montrent que les composés de l'invention inhibent l'interaction entre la protéine Bcl-2 et le peptide fluorescent décrit précédemment.

### EXEMPLE B : Cytotoxicité in vitro.

Les études de cytotoxicité ont été réalisées sur la lignée tumorale de leucémie RS4; 11. Les cellules sont réparties dans des microplaques et exposées aux composés à tester pendant 48 heures. La viabilité cellulaire est ensuite quantifiée par un essai colorimétrique, le Microculture Tétrazolium Assay (Cancer Res., 1987, 47, 939-942).
Les résultats sont exprimés en IC₅₀ (concentration en composé qui inhibe à 50 % la viabilité cellulaire) et sont présentés dans le tableau 1 ci-dessous.

Les résultats montrent que les composés de l'invention sont cytotoxiques.

**Tableau 1 : IC₅₀ d'inhibition de Bcl-2 (test de polarisation de fluorescence) et de cytotoxicité pour les cellules RS4 ; 11**

| | IC₅₀ (nM) Bcl-2 FP | IC₅₀ (nM) MTT RS4; 11 | | IC₅₀ (nM) Bcl-2 FP | IC₅₀ (nM) MTT RS4;11 |
|---|---|---|---|---|---|
| **Exemple 1** | 18 | 60,1 | **Exemple 32** | 34 | 71,3 |
| **Exemple 2** | 20 | 49,8 | **Exemple 33** | 23 | 12,2 |
| **Exemple 3** | 20 | 44,7 | **Exemple 34** | 27 | 65,8 |
| **Exemple 4** | 19 | 27,1 | **Exemple 35** | 15 | 11,7 |
| **Exemple 5** | 51 | 98 | **Exemple 36** | 43 | 135 |
| **Exemple 6** | 15 | 30 | **Exemple 37** | 19 | 11,6 |
| **Exemple 8** | 15 | 25,6 | **Exemple 38** | 21 | 61,8 |
| **Exemple 9** | 13 | 16,1 | **Exemple 39** | 16 | 14,1 |
| **Exemple 10** | 18 | 11 | **Exemple 40** | 15 | 32,2 |
| **Exemple 13** | 12 | 8,63 | **Exemple 41** | 26 | 44 |
| **Exemple 14** | 16 | 10,7 | **Exemple 42** | 13 | 53,6 |
| **Exemple 15** | 17 | 20,4 | **Exemple 43** | 110 | 142 |
| **Exemple 16** | 18 | 23,4 | **Exemple 44** | 14 | 45 |
| **Exemple 17** | 81 | 120 | **Exemple 45** | 47 | 76,8 |
| **Exemple 18** | 32 | 24,9 | **Exemple 46** | 21 | 56,8 |
| **Exemple 19** | 27 | 42,3 | **Exemple 47** | 58 | 194 |
| **Exemple 20** | 35 | 105 | **Exemple 48** | 130 | 229 |
| **Exemple 21** | 22 | 34,6 | **Exemple 49** | 45 | 169 |
| **Exemple 22** | 27 | 19,1 | **Exemple 50** | 13 | 4,01 |
| **Exemple 23** | 14 | 23 | **Exemple 51** | 32 | 26 |
| **Exemple 24** | 17 | 29,8 | **Exemple 52** | 16 | 19,8 |
| **Exemple 25** | 29 | 143 | **Exemple 53** | 180 | 138 |
| **Exemple 26** | 17 | 40 | **Exemple 54** | 180 | 106 |
| **Exemple 27** | 17 | 35,4 | **Exemple 55** | 34 | 19,4 |
| **Exemple 28** | 25 | 76,1 | **Exemple 56** | 20 | 25,9 |
| **Exemple 29** | 35 | 85,5 | **Exemple 57** | 12 | 8,62 |
| **Exemple 30** | 13 | 8,59 | **Exemple 58** | 22 | 40,1 |
| **Exemple 31** | 33 | 340 | **Exemple 59** | 13 | 8,13 |
| **Exemple 60** | 19 | 11,7 | **Exemple 98** | 12 | 73,5 |
| **Exemple 61** | 22 | 14,5 | **Exemple 99** | 25 | 213 |
| **Exemple 62** | 22 | 19,3 | **Exemple 100** | 22 | 856 |
| **Exemple 63** | 21 | 18,8 | **Exemple 101** | 5 | 22,1 |
| **Exemple 64** | 31 | 23,3 | **Exemple 103** | 6 | 16,8 |
| **Exemple 65** | 14 | 9,19 | **Exemple 104** | 7 | 21,6 |
| **Exemple 66** | 16 | 21,7 | **Exemple 105** | 10 | 28,9 |
| **Exemple 67** | 24 | 17,7 | **Exemple 107** | 7 | 8,29 |
| **Exemple 69** | 26 | 16,7 | **Exemple 108** | 9 | 36,4 |
| **Exemple 70** | 19 | 14 | **Exemple 109** | 30 | 483 |
| **Exemple 71** | 21 | 24,4 | **Exemple 110** | 29 | 129 |
| **Exemple 72** | 220 | 270 | **Exemple 111** | 15 | 81,5 |
| **Exemple 74** | 24 | 241 | **Exemple 112** | 9 | 139 |
| **Exemple 76** | 17 | 49 | **Exemple 113** | 16 | 190 |
| **Exemple 77** | 23 | 70,4 | **Exemple 114** | 9 | 43,1 |
| **Exemple 78** | 31 | 101 | **Exemple 115** | 8 | 58,1 |
| **Exemple 79** | 31 | 67,5 | **Exemple 116** | 10 | 43,6 |
| **Exemple 80** | 18 | 18,8 | **Exemple 117** | 15 | 194 |
| **Exemple 81** | 28 | 39,3 | **Exemple 118** | 15 | 71 |
| **Exemple 82** | 33 | 21,9 | **Exemple 119** | 9 | 31,6 |
| **Exemple 83** | 18 | 41,2 | **Exemple 121** | 34 | 445 |
| **Exemple 84** | 18 | 20,6 | **Exemple 122** | 16 | 43,5 |
| **Exemple 85** | 41 | 246 | **Exemple 123** | 22 | 92,1 |
| **Exemple 86** | 22 | 29,5 | **Exemple 124** | 28 | 101 |
| **Exemple 87** | 27 | 121 | **Exemple 125** | 38 | 81,8 |
| **Exemple 91** | 8 | 65,5 | **Exemple 126** | 26 | 115 |
| **Exemple 92** | 5 | 40,2 | **Exemple 127** | 26 | 129 |
| **Exemple 94** | 3 | 8,16 | **Exemple 128** | 10 | 63,4 |
| **Exemple 95** | 6 | 13,5 | **Exemple 129** | 9 | 46,7 |
| **Exemple 96** | 11 | 58,8 | **Exemple 131** | 30 | 88,4 |
| **Exemple 97** | 20 | 45,4 | **Exemple 134** | 37 | 305 |
| **Exemple 135** | 6 | 37,4 | **Exemple 144** | n.d. | 59,9 |
| **Exemple 141** | n.d. | 72,5 | **Exemple 145** | n.d. | 28,9 |
| **Exemple 142** | n.d. | 66,1 | **Exemple 146** | n.d. | 153 |
| **Exemple 143** | n.d. | 43,8 | **Exemple 147** | n.d. | 74,2 |

### EXEMPLE C : Induction de l'activité caspase in vivo.

La capacité des composés de l'invention à activer la caspase 3 est évaluée dans un modèle de xénogreffe de cellules leucémiques RS4 ; 11.
1.10⁷ cellules RS4 ;11 sont greffées par voie sous-cutanée dans des souris immunodéprimées (souche SCID). 25 à 30 jours après la greffe, les animaux sont traités par voie orale par les différents composés. Seize heures après le traitement, les masses tumorales sont récupérées, lysées et l'activité caspase 3 est mesurée dans les lysats tumoraux.
Cette mesure enzymatique est réalisée en dosant l'apparition d'un produit de clivage fluorigénique (activité DEVDase, Promega). Elle est exprimée sous la forme d'un facteur d'activation correspondant au rapport entre les deux activités caspases : celle pour les souris traitées divisée par celle pour les souris contrôles.

Les résultats obtenus sont présentés dans le tableau 2 et montrent que les composés de l'invention sont capables d'induire l'apoptose dans les cellules tumorales RS4 ;11 *in vivo.*

**Tableau 2 : Facteurs d'activation des caspases (activité DEVDase dans les tumeurs des souris traitées versus souris contrôles) in vivo, après traitements par voie orale (doses précisées entre parenthèses)**

| Composé testé | Facteur d'activation (versus contrôle) |
|---|---|
| **Exemple 1** | 29.3 (100 mg/kg) |
| **Exemple 2** | 27.8 (100 mg/kg) |
| **Exemple 3** | 5.7 (50 mg/kg) |
| **Exemple 4** | 29.2 (50 mg/kg) |
| **Exemple 8** | 9.8 (50 mg/kg) |
| **Exemple 9** | 20.6 (50 mg/kg) |
| **Exemple 10** | 13.8 (50 mg/kg) |
| **Exemple 22** | 11.3 (50 mg/kg) |
| **Exemple 23** | 22.7 (50 mg/kg) |
| **Exemple 58** | 23.3 (50 mg/kg) |
| **Exemple 59** | 23 (50 mg/kg) |
| **Exemple 66** | 24.7 (50 mg/kg) |
| **Exemple 67** | 18.9 (50 mg/kg) |

### EXEMPLE D : Quantification de la forme clivée de la caspase 3 in vivo.

La capacité des composés de l'invention à activer la caspase 3 est évaluée dans un modèle de xénogreffe de cellules leucémiques RS4 ;11.
1.10⁷ cellules RS4;11 sont greffées par voie sous-cutanée dans des souris immunodéprimées (souche SCID). 25 à 30 jours après la greffe, les animaux sont traités par voie orale par les différents composés. Après le traitement, les masses tumorales sont récupérées, lysées et la forme clivée (activée) de la caspase 3 est quantifiée dans les lysats tumoraux.
Cette quantification est réalisée en utilisant l'essai « Meso Scale Discovery (MSD) ELISA platform » qui dose spécifiquement la forme clivée de la caspase 3. Elle est exprimée sous la forme d'un facteur d'activation correspondant au rapport entre la quantité de caspase 3 clivée chez les souris traitées divisée par la quantité de caspase 3 clivée chez les souris contrôles.

Les résultats obtenus sont présentés dans le tableau 3 et montrent que les composés de l'invention sont capables d'induire l'apoptose dans les cellules tumorales RS4 ;11 *in vivo.*

**Tableau 3 : Facteurs d'activation des caspases (caspase 3 clivée essai MSD dans les tumeurs des souris traitées versus souris contrôles) in vivo, après traitement par voie orale (doses précisées entre parenthèses)**

| Composé testé | Facteur d'activation (versus contrôle) |
|---|---|
| **Exemple 78** | 10.4 (25 mg/kg) |
| **Exemple 87** | 18.3 (50 mg/kg) |
| **Exemple 122** | 17.4 (25 mg/kg) |
| **Exemple 135** | 60 (50 mg/kg) |

### EXEMPLE E : Activité anti-tumorale in vivo.

L'activité anti-tumorale des composés de l'invention est évaluée dans un modèle de xénogreffe de cellules leucémiques RS4 ; 11.
1.10⁷ cellules RS4 ;11 sont greffées par voie sous-cutanée dans des souris immunodéprimées (souche SCID). 25 à 30 jours après la greffe, lorsque la masse tumorale a atteint environ 150 mm³, les souris sont traitées par voie orale par les différents composés dans 2 schémas différents (traitement quotidien pendant cinq jours par semaine durant deux semaines, ou deux traitements par semaine pendant deux semaines). La masse tumorale est mesurée 2 fois par semaine depuis le début du traitement.

Les composés de l'invention présentent des activités antitumorales, par voie orale, dans le modèle de leucémie RS4 ;11 (leucémie aiguë lymphoblastique) avec des ΔT/C (paramètre de qualification de l'activité d'un produit qui se définit comme le ratio volume tumoral du groupe traité / volume tumoral du groupe contrôle non-traité) allant de -15 à -56% en rapport avec une régression tumorale. Les résultats obtenus montrent donc que les composés de l'invention sont capables d'induire une régression tumorale significative durant la période de traitement.

### EXEMPLE F : Composition pharmaceutique : Comprimés

| | |
|---|---|
| 1000 comprimés dosés à 5 mg d'un composé choisi parmi les exemples 1 à 160 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 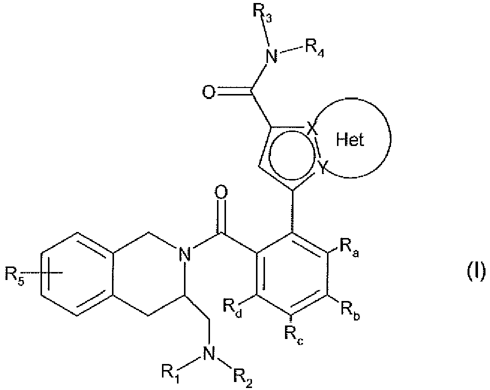 dans laquelle :
◆ X et Y représentent un atome de carbone ou un atome d'azote, étant entendu qu'ils ne peuvent représenter simultanément deux atomes de carbone ou deux atomes d'azote,
◆ la partie Het du groupe 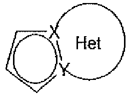 représente un cycle éventuellement substitué, aromatique ou non, constitué de 5, 6 ou 7 chaînons, et pouvant contenir, en plus de l'azote représenté par X ou par Y, un à 3 hétéroatomes choisis indépendamment parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié,
◆ R₁ et R₂ représentent indépendamment l'un de l'autre un atome d'hydrogène ou un groupement alkyle (C₁-C₆) linéaire ou ramifié,
ou bien R₁ et R₂ forment avec l'atome d'azote qui les porte, un hétérocycloalkyle constitué de 4 à 7 chaînons et pouvant contenir, en plus de l'atome d'azote, un autre hétéroatome choisi parmi oxygène, soufre, SO₂ et NR dans lequel R représente un
atome d'hydrogène, un groupement alkyle (C₁-C₆) linéaire ou ramifié, un groupement (C₁-C₆)alkylsulfonyl, un groupement polyhalogénoalkyle (C₁-C₆) linéaire ou ramifié, ou un groupement -C(O)-O-Alk dans lequel Alk est un groupement alkyle (C₁-C₆) linéaire ou ramifié,,
◆ R₃ représente un groupement alkyle (C₁-C₆) linéaire ou ramifié, alkényle (C₂-C₆), alkynyle (C₂-C₆), cycloalkyle, cycloalkyl(C₄-C₁₀)alkyle (C₁-C₆) linéaire ou ramifié, aryle ou hétéroaryle,
◆ R₄ représente un groupement aryle, hétéroaryle, cycloalkyle ou alkyle (C₁-C₆) linéaire ou ramifié,
◆ R₅ représente un atome d'hydrogène ou d'halogène,
◆ Rₐ, R_{b}, R_{c} et R_{d} représentent indépendamment les uns des autres un atome d'hydrogène, un atome d'halogène, un groupement alkyle (C₁-C₆) linéaire ou ramifié, un groupement alkoxy (C₁-C₆) linéaire ou ramifié, un groupe hydroxy, un groupement polyhalogénoalkyle (C₁-C₆) linéaire ou ramifié, ou un groupe trifluorométhoxy, ou bien les substituants de l'un des couples (Rₐ, R_{b}), (R_{b}, R_{c}) ou (Rₑ, R_{d}) forment ensemble avec les atomes de carbone qui les portent un cycle constitué de 5 à 7 chaînons, pouvant contenir de un à 3 hétéroatomes choisis parmi oxygène, soufre et azote, étant entendu que l'azote en question peut être substitué par un groupement représentant un atome d'hydrogène, un groupement alkyle (C₁-C₆) linéaire ou ramifié ou un groupement -C(O)-O-Alk dans lequel Alk est un groupement alkyle (C₁-C₆) linéaire ou ramifié, étant aussi entendu qu'un ou plusieurs atomes de carbone du cycle précédemment défini peut(vent) être deutéré(s),
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 3 hétéroatomes choisis parmi oxygène, soufre ou azote,
- par "cycloalkyle", on entend tout groupement carbocyclique non aromatique, mono ou bi-cyclique, contenant 4 à 10 chaînons
les groupements alkyle, aryle, hétéroaryle, cycloallcyle et hétérocycloalkyle ainsi définis pouvant être substitués par 1 à 3 groupements choisis parmi alkyle (C₁-C₆) linéaire ou ramifié éventuellement substitué, spiro (C₃-C₆), alkoxy (C₁-C₆) linéaire ou ramifié, (C₁-C₆)alkyl-S-, hydroxy, oxo (ou *N*-oxide le cas échéant), nitro, cyano, -COOR', NR'R", polyhalogénoalkyle (C₁-C₆) linéaire ou ramifié, trifluorométhoxy, (C₁-C₆)alkylsulfonyl ou halogène, étant entendu que R' et R" représentent, indépendamment l'un de l'autre, un atome d'hydrogène ou un groupement alkyle (C₁-C₆) linéaire ou ramifié,
la partie Het du groupe 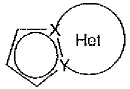 défini ci-dessus pouvant être substituée par un groupement choisi parmi allyle (C₁-C₆) linéaire ou ramifié, hydroxy, NR₁'R₁", ou halogène, étant entendu que R₁' et R₁" ont les mêmes définitions que les groupes R' et R" mentionnés précédemment,
leurs énantiomères et diastéréoisomères, ainsi que leurs sels d'addition à un acide ou à une base pharmaceutiquement acceptable.

2. Composé de formule (I) selon la revendication 1 dans lequel le groupe 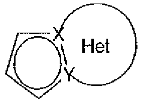 représente l'un des groupements suivants : 5,6,7,8-tétrahydroindolizine éventuellement substitué par un hydroxy, indolizine, 1,2,3,4-tétrahydropyrrolo[1,2-*a*]pyrazine, 3,4-dihydropyrrolo[1,2-*a*]pyrazine-2(1*H*)-carboxylate de tert-butyle, 3,4-dihydro-1*H-*pyrrolo[2,1-*c*][1,4]oxazine, 2,3-dihydro-1*H*-pyrrolizine éventuellement substitué par un hydroxy, 6,7,8,9-tétrahydro-5*H*-pyrrolo[1,2-*a*]azépine ou pyrrolo[1,2-*a*]pyrazine.

3. Composé de formule (I) selon la revendication 1 dans lequel R₁ et R₂ représentent chacun un groupement alkyle éventuellement substitué par un méthoxy, ou bien R₁ et R₂ forment avec l'atome d'azote qui les porte un hétérocycloalkyle choisi parmi les groupes suivants : morpholine éventuellement substituée par un ou plusieurs alkyle(s) (C₁-C₆) linéaire(s) ou ramifié(s), oxidomorpholine, thiomorpholine 1,1-dioxide, 1,4-oxazépane, 3-méthoxypyrrolidine, 3,3-difluoropyrrolidine, 3-méthoxyazétidine, 3-fluoroazétidine, oxopipérazine ou pipérazine, les deux derniers groupes étant substitués par un groupement alkyle (C₁-C₆) linéaire ou ramifié, polyhalogénoalkyle (C₁-C₆) linéaire ou ramifié ou méthylsulfonyl.

4. Composé de formule (I) selon la revendication 1 dans lequel Rₐ, et R_{d} représentent chacun un atome d'hydrogène et (R_{b},R_{c}) forment ensemble avec les atomes de carbone qui les portent un groupe 1,3-dioxolane dont l'un des atomes de carbone est optionnellement deutéré, un groupe 1,4-dioxane, un groupe 1,4-dioxépane, ou bien Rₐ, R_{c} et R_{d} représentent chacun un atome d'hydrogène et R_{b} représente un halogène, un méthyle, un méthoxy, un éthoxy, un trifluorométhyle ou un trifluorométhoxy.

5. Composé de formule (I) selon la revendication 1 dans lequel R₄ représente un groupement 4-hydroxyphényle, 3-fluoro-4-hydroxyphényle ou 5-hydroxypyrimidine.

6. Composé de formule (I) selon la revendication 1 dans lequel R₃ représente un groupement choisi parmi phényle, indole, indoline, 1,2,3,4-tétrahydroquinoline, 3,4-dihydro-2*H*-1,4-benzoxazine, indane, 1*H*-indazole, 1*H*-pyrrolo[2,3-*b*]pyridine, pyrimidine, cyclobutylméthyle, cyclopropylméthyle, 1*H*-pyrazole, pyridine, pyridazine, ces groupements comportant éventuellement un ou plusieurs substituants choisis parmi halogène, alkyle (C₁-C₆) linéaire ou ramifié, cyano ou alkoxy (C₁-C₆) linéaire ou ramifié.

7. Composé de formule (I) selon la revendication 1 choisi parmi le groupe suivant :
- *N*-(4-hydroxyphényl)-3-{6-[((3*S*)-3-(4-morpholinylméthyl)-3,4-dihydro-2(1*H*)-isoquinolinyl)carbonyl]-1,3-bcnzodioxol-5-yl}-*N*-phenyl-5,6,7,8-tetrahydro-1-indolizine carboxamide,
- 3-{5-chloro-2-[((3*S*)-3-(4-morpholinylméthyl)-3,4-dihydro-2(1*H*)-isoquinolinyl) carbonyl]phényl}-*N*-(4-hydroxyphényl)-*N*-(1-méthyl-1H-indol-5-yl)-5,6,7,8-tétrahydro-1-indolizine carboxamide,
- *N*-(4-hydroxyphényl)-*N*-(1-méthyl-1*H*-indazol-5-yl)-3-{2,2-dideutério-6-[(3*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide,
- *N*-(4-hydroxyphényl)-*N*-(1-méthyl-1*H*-indazol-5-yl)-3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizine-1-carboxamide,
- *N*-(4-hydroxyphényl)-3-{7-[((3*S*)-3-(4-morpholinylméthyl)-3,4-dihydro-2(1*H*)-isoquinolinyl)carbonyl]-2,3-dihydro-1,4-benzodioxin-6-yl}-*N*-phényl-5,6,7,8-tétrahydro-1-indolizine carboxamide,
- *N*-(4-hydroxyphényl)-*N*-(1méthyl-1*H*-indol-5-yl)-3-{6-[((3*S*)-3-[(4-méthyl-1-pipérazinyl)méthyl]-3,4-dihydro-2(1*H*)-isoquinolinyl)carbonyl]-1,3-benzodioxol-5-yl}-1-indolizine carboxamide,
- *N*-[4-(hydroxy)phényl]-*N*-(1-méthyl-1*H*-indol-5-yl)-3-{6-[((3*S*)-3-(4-morpholinyl méthyl)-3,4-dihydro-2(1*H*)-isoquinolinyl)carbonyl]-1,3-benzodioxol-5-yl}-5,6,7,8-tétrahydro-1-indolizine carboxamide,
- *N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-1-indolizine carboxamide,
- 3-{5-chloro-2-[((3*S*)-3-(4-morpholinylméthyl)-3,4-dihydro-2(1*H*)-isoquinolinyl)carbonyl]phényl}-*N*-{4-hydroxyphényl}-*N*-(1-méthyl-1*H*-indol-5-yl)-1-indolizine carboxamide,
- 6-{5-chloro-2-[((3*S*)-3-(4-morpholinylméthyl)-3,4-dihydro-2(1*H*)-isoquinolinyl) carbonyl]phényl}-*N*-(3-fluoro-4-méthylphényl)-*N*-(4-hydroxyphényl)-3,4-dihydro-1*H*-pyrrolo[2,1-*c*][1,4]oxazine-8-carboxamide,
- 3-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-*N*-(1-méthyl-1*H*-pyrazol-4-yl)-5,6,7,8-tétrahydroindolizine-1-carboxamide,
- *N*-(3-fluoro-4-méthylphényl)-*N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide,
- *N*-[4-(hydroxy)phényl]-*N*-(1-méthyl-2,3-dihydro-1*H*-indol-5-yl)-3-{6-[((3*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide,
- 3-{5-chloro-2-[((3*S*)-3-(4-morpholinylméthyl)-3,4-dihydro-2(1H)-isoquinolinyl)carbonyl]phényl}-N-(4-hydroxyphényl)-*N*-(1-méthyl-2,3-dihydro-1*H* indol-5-yl)-5,6,7,8-tétrahydro-1-indolizine carboxamide,
- *N*-(4-hydroxyphényl)-*N*-(1-méthyl-1*H*-indol-5-yl)-3-{6-[((3*S*)-3-(4-morpholinylméthyl)-3,4-dihydro-2(1*H*)-isoquinolinyl)carbonyl]-1,3-benzodioxol-5-yl}-1-indolizine carboxamide,
- 3-{5-chloro-2-[((3*S*)-3-(4-morpholinylméthyl)-3,4-dihydro-2(1*H*)-isoquinolinyl) carbonyl]phényl}-*N*-(4-hydroxyphényl)-*N*-(1-méthyl-2,3-dihydro-1*H*-indol-5-yl)-1-indolizine carboxamide,
- 6-{5-chloro-2-[((3*S*)-3-(4-morpholinylméthyl)-3,4-dihydro-2(1*H*)-isoquinolinyl) carbonyl]phényl}-*N*-(4-hydroxyphényl)-*N*-phényl-3,4-dihydro-1*H*-pyrrolo[2,1-*c*][1,4]oxazine-8-carboxamide,
- *N*-(3-fluorophényl)-*N*-(4-hydroxyphényl)-3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizine-1-carboxamide,
leurs énantiomères et diastéréoisomères, ainsi que leurs sels d'addition à un acide ou à une base pharmaceutiquement acceptable.

8. Composé de formule (I) selon la revendication 1 choisi parmi le groupe suivant :
- *N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-5,6,7,8-tétrahydro-1-indolizine carboxamide,
- 3-{5-chloro-2-[((3*S*)-3-(4-morpholinylméthyl)-3,4-dihydro-2(1*H*)-isoquinolinyl) carbonyl]phényl}-*N-*(4-hydroxyphényl)-*N*-(1-méthyl-1H-indol-5-yl)-5,6,7,8-tétrahydro-1-indolizine carboxamide,
- *N*-(4-hydroxyphényl)-*N*-(1-méthyl-1*H*-indazol-5-yl)-3-{2,2-dideutério-6-[((3*S*)-3-(4-morpholinylméthyl)-3,4-dihydro-2(1*H*)-isoquinolinyl)carbonyl]-1,3-benzodioxol -5-yl}-5,6,7,8-tétrahydro-1-indolizine carboxamide,
- *N*-(4-hydroxyphényl)-*N*-(1-méthyl-1*H*-indazol-5-yl)-3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizine-1-carboxamide,
- *N*-(4-hydroxyphényl)-3-{7-[((3*S*)-3-(4-morpholinylméthyl)-3,4-dihydro-2(1*H*)-isoquinolinyl)carbonyl]-2,3-dihydro-1,4-benzodioxin-6-yl}-*N*-phényl-5,6,7,8-tétrahydro-1-indolizine carboxamide,
- *N*-(4-hydroxyphényl)-*N*-(1-méthyl-1*H*-indol-5-y1)-3-{6-[((3*S*)-3-[(4-méthyl-1-pipérazinyl)méthyl]-3,4-dihydro-2(1*H*)-isoquinolinyl)carbonyl]-1,3-bonzodioxol-5-yl}-1-indolizine carboxamide,
- *N*-[4-(hydroxy)phényl]-*N*-(1-méthyl-1*H*-indol-5-yl)-3-{6-[((3*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide,
- N-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-1-indolizine carboxamide,
- 3-{5-chloro-2-[((3*S*)-3-(4-morpholinylméthyl)-3,4-dihydro-2(1*H*)-isoquinolinyl)carbonyl]phényl}-*N*-(4-hydroxyphényl)-*N*-(1-méthyl-1*H*-indol-5-yl)-1-indolizine carboxamide,
- 6-{5-chloro-2-[((3*S*)-3-(4-morpholinylméthyl)-3,4-dihydro-2(1*H*)-isoquinolinyl) carbonyl]phényl}-*N*-(3-fluoro-4-méthylphényl)-*N*-(4-hydroxyphényl)-3,4-dihydro-1*H*-pyrrolo[2,1-c][1,4]oxazine-8-carboxamide,
- 3-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-*N*-(1-méthyl-1*H*-pyrazol-4-yl)-5,6,7,8-tétrahydroindolizine-1-carboxamide,
leurs énantiomères et diastéréoisomères, ainsi que leurs sels d'addition à un acide ou à une base pharmaceutiquement acceptable.

9. Composé de formule (I) selon la revendication 8 qui est le *N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-5,6,7,8-tétrahydro-1-indolizine carboxamide.

10. Composé de formule (I) selon la revendication 8 qui est le 3-{5-chloro-2-[((3*S*)-3-(4-morpholinylméthyl)-3,4-dihydro-2(1*H*)-isoquinolinyl)carbonyl]phényl}-*N*-(4-hydroxyphényl)-*N*-(1-méthyl-1*H*-indol-5-yl)-5,6,7,8-tétrahydro-1-indolizine carboxamide.

11. Composé de formule (I) selon la revendication 8 qui est le *N*-(4-hydroxyphényl)-N-(1-méthyl-1*H*-indazol-5-yl)-3-{2,2-dideutério-6-[((3*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide.

12. Composé de formule (I) selon la revendication 8 qui est le *N*-(4-hydroxyphényl)-*N*-(1-méthyl-1*H*-indazol-5-yl)-3-(6-{[(3*S*)-3-(morpholin-4-ylméthyl)-3,4-dihydroisoquinolin-2(1*H*)-yl]carbonyl}-1,3-benzodioxol-5-yl)-5,6,7,8-tétrahydroindolizine-1-carboxamide.

13. Composé de formule (I) selon la revendication 8 qui est le *N*-(4-hydroxyphényl)-3-{7-[((3*S*)-3-(4-morpholinylméthyl)-3,4-dihydro-2(1*H*)-isoquinolinyl)carbonyl]-2,3-dihydro-1,4-benzodioxin-6-yl}-*N*-phényl-5,6,7,8-tétrahydro-1-indolizine carboxamide.

14. Composé de formule (I) selon la revendication 8 qui est le *N*-(4-hydroxyphényl)-*N*-(1-méthyl-1*H*-indol-5-yl)-3-{6-[((3*S*)-3-[(4-méthyl-1-pipérazinyl)méthyl]-3,4-dihydro-2(1*H*)-isoquinolinyl)carbonyl]-1,3-benzodioxol-5-yl}-1-indolizine carboxamide.

15. Composé de formule (I) selon la revendication 8 qui est le N-[4-(hydroxy)phényl]-N-(1-méthyl-1*H*-indol-5-yl)-3-{6-[((3*S*)-3-(4-morpholinylméthyl)-3,4-dihydro-2(1*H*)-isoquinolinyl)carbonyl]-1,3-benzodioxol-5-yl}-5,6,7,8-tétrahydro-1-indolizine carboxamide.

16. Composé de formule (I) selon la revendication 8 qui est le *N*-(4-hydroxyphényl)-3-{6-[((3*S*)-3-(4-morpholinylméthyl)-3,4-dihydro-2(1*H*)-isoquinolinyl)carbonyl]-1,3-benzodioxol-5-yl}-*N*-phényl-1-indolizine carboxamide.

17. Composé de formule (I) selon la revendication 8 qui est le 3-{5-chloro-2-[((3*S*)-3-(4-morpholinylméthyl)-3,4-dihydro-2(1*H*)-isoquinolinyl)carbonyl]phényl}-*N*-(4-hydroxyphényl)-*N*-(1-méthyl-1*H*-indol-5-yl)-1-indolizine carboxamide.

18. Composé de formule (I) selon la revendication 8 qui est le 6-{5-chloro-2-[((3*S*)-3-(4-morpholinylméthyl)-3,4-dihydro-2(1*H*)-isoquinolinyl)carbonyl]phényl}-*N*-(3-fluoro-4-méthylphényl)-*N*-(4-hydroxyphényl)-3,4-dihydro-1*H*-pyrrolo[2,1-*c*][1,4]oxazine-8-carboxamide.

19. Composé de formule (I) selon la revendication 8 qui est le 3-(5-chloro-2-{[(3*S*)-3-(morpholin-4-ylméthyl)-3,4-dihydroisoquinolin-2(1*H*)-yl]carbonyl}phényl)-*N*-(4-hydroxyphényl)-*N*-(1-méthyl-1*H*-pyrazol-4-yl)-5,6,7,8-tétrahydroindolizine-1-carboxamide.

20. Sel de chlorhydrate d'un composé de formule (I) selon l'une des revendications 9 à 13 et15à19.

21. Sel de dichlorhydrate du composé de formule (I) selon la revendication 14.

22. Procédé de préparation d'un composé de formule (I) selon la revendication 1 **caractérisé en ce que** l'on utilise comme produit de départ le composé de formule (II) : 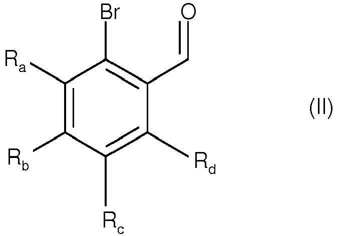 dans laquelle Rₐ, R_{b}, R_{c} et R_{d} sont tels que définis dans la formule (I),
composé de formule (II) qui est soumis à une réaction de Heck, en milieu aqueux ou organique, en présence d'un catalyseur au palladium, d'une base, d'une phosphine et du composé de formule (III) : 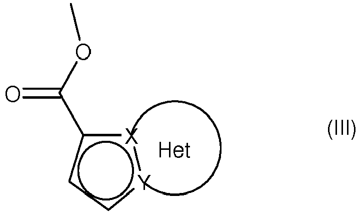 dans laquelle les groupements X, Y et Het sont tels que définis dans la formule (I),
pour obtenir le composé de formule (IV) : 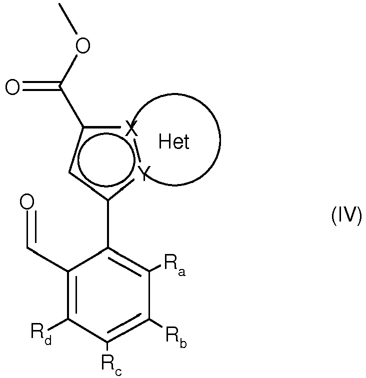 dans laquelle Rₐ, R_{b}, Rₑ, R_{d}, X, Y et Het sont tels que définis dans la formule (I),
composé de formule (IV) dont la fonction aldéhyde est oxydée en acide carboxylique pour former le composé de formule (V) : 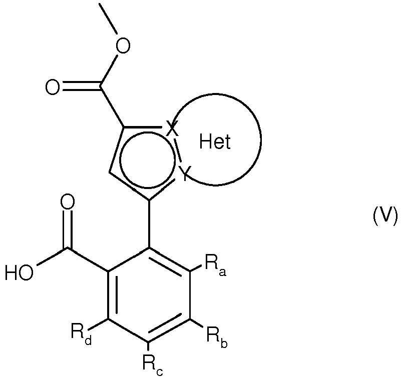 dans laquelle Rₐ, R_{b}, R_{c}, R_{d}, X, Y et Het sont tels que définis dans la formule (I),
composé de formule (V) qui subit ensuite un couplage peptidique avec un composé de formule (VI) : 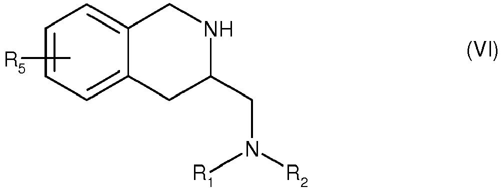 dans laquelle R₁, R₂, et R₅ sont tels que définis dans la formule (I),
pour conduire au composé de formule (VII) : 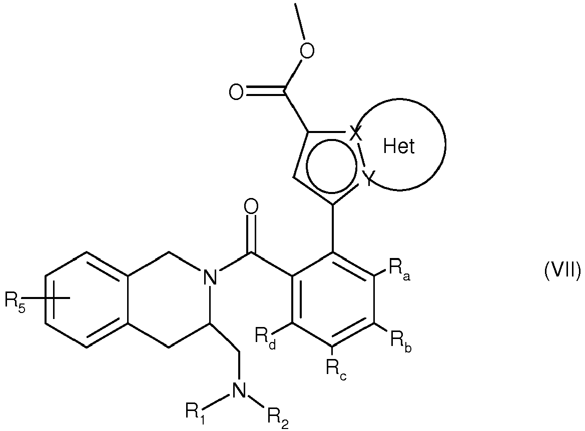 dans laquelle Rₐ, R_{b}, R_{c}, R_{d}, R_{1,} R₂, R₅, X, Y et Het sont tels que définis dans la formule (I), composé de formule (VII) dont la fonction ester est hydrolysée pour conduire à l'acide carboxylique ou au carboxylate correspondant, lequel peut être converti en un dérivé d'acide tel que le chlorure d'acyle ou l'anhydride correspondant, avant d'être couplé avec une amine NHR₃R₄, dans laquelle R₃ et R₄ ont la même signification que dans la formule (I), pour conduire au composé de formule (I),
composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, certains groupements (hydroxy, amino...) des réactifs ou intermédiaires de synthèse peuvent être protégés puis déprotégés pour les besoins de la synthèse.

23. Procédé de préparation selon la revendication 22 d'un composé de formule (I) dans lequel l'un des groupements R₃ ou R₄ est substitué par une fonction hydroxy **caractérisé en ce que** l'amine NHR₃R₄ est préalablement soumise à une réaction de protection de la fonction hydroxy avant tout couplage avec l'acide carboxylique du composé de formule (VII), ou avec l'un des dérivés d'acide correspondant, le composé de formule (I) protégé résultant subit ensuite une réaction de déprotection, puis est éventuellement converti en l'un de ses sels d'addition à un acide ou à une base pharmaceutiquement acceptable.

24. Composition pharmaceutique contenant un composé de formule (I) selon l'une quelconque des revendications 1 à 19 ou un de ses sels d'addition avec un acide ou une base pharmaceutiquement acceptable en combinaison avec un ou plusieurs excipients pharmaceutiquement acceptables.

25. Composition pharmaceutique selon la revendication 24 pour son utilisation en tant qu'agent pro-apoptotique.

26. Composition pharmaceutique selon la revendication 24 pour son utilisation dans le traitement des cancers, des maladies immunitaires et auto-immunes.

27. Composition pharmaceutique selon la revendication 24 pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes folliculaires, des mélanomes, des hémopathies malignes, des myélomes, du cancer de l'ovaire, du cancer du poumon non à petites cellules, du cancer de la prostate et du cancer du poumon à petites cellules.

28. Composé de formule (I) selon l'une des revendications 1 à 19, ou un de ses sels d'addition avec un acide ou une base pharmaceutiquement acceptable, pour son utilisation dans le traitement des cancers de la vessie, du cerveau, du sein, de l'utérus, des leucémies lymphoïdes chroniques, du cancer colorectal, des cancers de l'oesophage, du foie, des leucémies lymphoblastiques, des lymphomes folliculaires, des mélanomes, des hémopathies malignes, des myélomes, du cancer de l'ovaire, du cancer du poumon non à petites cellules, du cancer de la prostate et du cancer du poumon à petites cellules.

29. Association d'un composé de formule (I) selon l'une quelconque des revendications 1 à 21 avec un agent anticancéreux choisi parmi les agents génotoxiques, les poisons mitotiques, les anti-métabolites, les inhibiteurs du protéasome, les inhibiteurs de kinases ou les anticorps.

30. Composition pharmaceutique contenant une association selon la revendication 29 en combinaison avec un ou plusieurs excipients pharmaceutiquement acceptables.

31. Association selon la revendication 29 pour son utilisation dans le traitement des cancers.

32. Composé de formule (I) selon l'une quelconque des revendications 1 à 21 pour son utilisation en association avec une radiothérapie dans le traitement des cancers.

## Patentansprüche

1. Verbindung der Formel (I): 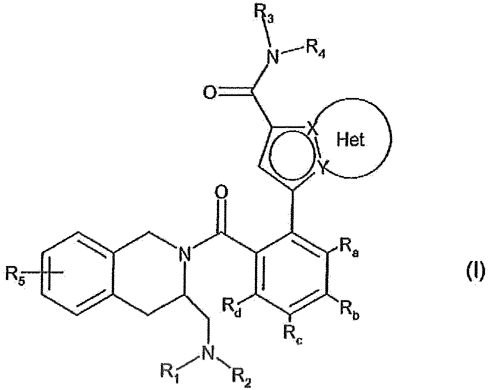 in der :
◆ X und Y ein Kohlenstoffatom oder ein Stickstoffatom bedeuten, wobei es sich versteht, dass sie nicht gleichzeitig zwei Kohlenstoffatome oder zwei Stickstoffatome bedeuten können,
◆ der Rest Het der Gruppe 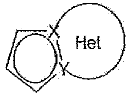 einen gegebenenfalls substituierten, aromatischen oder nichtaromatischen, aus 5, 6 oder 7 Kettengliedern gebildeten Ring bedeutet, der neben dem durch X oder durch Y dargestellten Stickstoff ein bis 3 Heteroatome unabhängig voneinander ausgewählt aus Sauerstoff, Schwefel und Stickstoff enthalten kann, wobei es sich versteht, dass der in Rede stehende Stickstoff durch eine Gruppe substituiert sein kann, die ein Wasserstoffatom, eine geradkettige oder verzweigte (C₁-C₆)-Alkylgruppe oder eine Gruppe -C(O)-O-Alk, in der Alk eine geradkettige oder verzweigte (C₁-C₆)-Alkylgruppe darstellt, ist,
◆ R₁ und R₂ unabhängig voneinander ein Wasserstoffatom oder eine geradkettige oder verzweigte (C₁-C₆)-Alkylgruppe bedeuten,
oder R₁ und R₂ gemeinsam mit dem sie tragenden Stickstoffatom einen aus 4 bis 7 Kettengliedern gebildeten Heterocyclus bilden, der neben dem Stickstoffatom ein weiteres Heteroatom enthalten kann, ausgewählt aus Sauerstoff, Schwefel, SO₂ und NR, worin R ein Wasserstoffatom, eine geradkettige oder verzweigte (C₁-C₆)-Alkylgruppe, eine (C₁-C₆)-Alkylsulfonylgruppe, eine geradkettige oder verzweigte (C₁-C₆)-Polyhalogenalkylgruppe oder eine Gruppe-C(O)-O-Alk, in der Alk eine geradkettige oder verzweigte (C₁-C₆)-Alkylgruppe darstellt, bedeutet,
◆ R₃ eine geradkettige oder verzweigte (C₁-C₆-Alkylgruppe, (C₂-C₆)-Alkenylgruppe, (C₂-C₆)-Alkinylgruppe, Cycloalkylgruppe, geradkettige oder verzweigte (C₄-C₁₀)-Cycloalkyl-(C₁-C₆)-alkylgruppe, Arylgruppe oder Heteroarylgruppe bedeutet,
◆ R₄ eine Arylgruppe, Heteroarylgruppe, Cycloalkylgruppe oder geradkettige oder verzweigte (C₁-C₆)-Alkylgruppe bedeutet,
◆ R₅ ein Wasserstoff- oder Halogenatom bedeutet,
◆ Rₐ, R_{b}, R_{c} und R_{d} unabhängig voneinander ein Wasserstoffatom, ein Halogenatom, eine geradkettige oder verzweigte (C₁-C₆)-Alkylgruppe, eine geradkettige oder verzweigte (C₁-C₆)-Allcoxygruppe, eine Hydroxygruppe, eine geradkettige oder verzweigte (C₁-C₆)-Polyhalogenalkylgruppe oder eine Trifluormethoxygruppe bedeuten, oder die Substituenten eines der Paare (Rₐ,R_{b}), (R_{b},R_{c}) oder (R_{c},R_{d}) zusammen mit den sie tragenden Kohlenstoffatomen einen aus 5 bis 7 Kettengliedern gebildeten Ring bilden, der ein bis 3 Heteroatome ausgewählt aus Sauerstoff, Schwefel und Stickstoff enthalten kann, wobei es sich versteht, dass der in Rede stehende Stickstoff durch eine Gruppe substituiert sein kann, die ein Wasserstoffatom, eine geradkettige oder verzweigte (C₁-C₆)-Alkylgruppe oder eine Gruppe -C(O)-O-Alk, in der Alk eine geradkettige oder verzweigte (C₁-C₆)-Alkylgruppe darstellt, ist, mit der weiteren Maßgabe, dass eines oder mehrere Kohlenstoffatome des oben definierten Rings deuteriert sein kann (können),
wobei es sich versteht, dass:
- man unter "Aryl" eine Phenyl-, Naphthyl-, Biphenyl- oder Indenylgruppe versteht,
- man unter "Heteroaryl" jede mono- oder bicyclische, aus 5 bis 10 Kettengliedern gebildete Gruppe versteht, die mindestens einen aromatischen Teil aufweist und 1 bis 3 Heteroatome ausgewählt aus Sauerstoff, Schwefel oder Stickstoff enthält,
- man unter "cycloalkyl" jede carbocyclische, nichtaromatische, mono- oder bicyclische Gruppe, die 4 bis 10 Kettenglieder enthält, versteht,
wobei die in dieser Weise definierten Alkyl-, Aryl-, Heteroaryl-, Cycloalkyl- und Heterocycloalkylgruppen durch 1 bis 3 Gruppen substituiert sein können, ausgewählt aus geradkettigem oder verzweigtem, gegebenenfalls substituiertem (C₁-C₆)-Alkyl, (C₃-C₆)-Spiro, geradkettigem oder verzweigtem (C₁-C₆)-Alkoxy, (C₁-C₆)-Alkyl-S-, Hydroxy, Oxo (oder gegebenenfalls *N*-Oxid), Nitro, Cyano, -COOR', NR'R", geradkettigem oder verzweigtem (C₁-C₆)-Polyhalogenalkyl, Trifluormethoxy, (C₁-C₆)-Alkylsulfonyl oder Halogen, wobei es sich versteht, dass R' und R" unabhängig voneinander ein Wasserstoffatom oder eine geradkettige oder verzweigte (C₁-C₆)-Alkylgruppe bedeuten,
wobei der Rest Het der oben definierten Gruppe gewählt aus geradkettigem oder verzweigtem 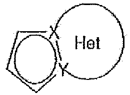 durch eine Gruppe aus-(C₁-C₆)-Alkyl, Hydroxy, NR₁'R₁" oder Halogen substituiert sein kann, wobei es sich versteht, dass R₁' und R₁" die gleichen Bedeutungen besitzen wie die oben erwähnten Gruppen R' und R", deren Enantiomere und Diastereoisomere sowie deren Additionssalze mit einer pharmazeutisch annehmbaren Säure oder Base.

2. Verbindung der Formel (I) nach Anspruch 1, worin die Gruppe 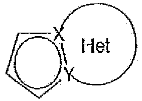 eine der folgenden Gruppen bedeutet: gegebenenfalls durch eine Hydroxygruppe substituiertes 5,6,7,8-Tetrahydroindolizin, Indolizin, 1,2,3,4-Tetrahydropyrrolo[1,2-α]pyrazin, 3,4-Dihydropyrrolo[1,2-*a*]pyrazin-2(1*H*)-tert.-butylcarboxylat, 3,4-Dihydro-1*H-*pyrrolo[2,1-*c*][1,4]oxazin, gegebenenfalls durch eine Hydroxygruppe substituiertes 2,3-Dihydro-1*H-*pyrrolizin, 6,7,8,9-Tetrahydro-5*H-*pyrrolo[1,2-α]azepin oder Pyrrolo[1,2-α]pyrazin.

3. Verbindung der Formel (I) nach Anspruch 1, worin R₁ und R₂ jeweils eine gegebenenfalls durch eine Methoxygruppe substituierte Alkylgruppe bedeuten, oder R₁ und R₂ zusammen mit dem sie tragenden Stickstoffatom einen Heterocyclus bilden ausgewählt aus den folgenden Gruppen: gegebenenfalls durch eine oder mehrere geradkettige oder verzweigte (C₁-C₆)-Alkylgruppe(n) substituiertes Morpholin, Oxidomorpholin, Thiomorpholin-1,1-dioxid, 1,4-Oxazepan, 3-Methoxypyrrolidin, 3,3-Difluorpyrrolidin, 3-Methoxyazetidin, 3-Fluorazetidin, Oxopiperazin oder Piperazin, wobei die beiden letzten Gruppen durch eine geradkettige oder verzweigte (C₁-C₆)-Alkylgruppe, geradkettige oder verzweigte oder verzweigte (C₁-C₆)-Polyhalogenalkylgruppe oder Methylsulfonylgruppe substituiert sind.

4. Verbindung der Formel (I) nach Anspruch 1, worin Rₐ und R_{d} jeweils ein Wasserstoffatom bedeuten und (R_{b},R_{c}) gemeinsam mit den sie tragenden Kohlenstoffatomen eine 1,3-Dioxolangruppe, bei der eines der Kohlenstoffatome gegebenenfalls deuteriert ist, eine 1,4-Dioxangruppe, eine 1,4-Dioxepangruppe bilden oder Rₐ, R_{c} und R_{d} jeweils ein Wasserstoffatom bedeuten und R_{b} ein Halogenatom, eine Methylgruppe, eine Methoxygruppe, eine Ethoxygruppe, eine Trifluormethylgruppe oder eine Trifluormethoxygruppe bedeutet.

5. Verbindung der Formel (I) nach Anspruch 1, worin R₄ eine 4-Hydroxyphenylgruppe, 3-Fluor-4-hydroxyphenylgruppe oder 5-Hydroxypyrimidingruppe bedeutet.

6. Verbindung der Formel (I) nach Anspruch 1, worin R₃ eine Gruppe ausgewählt aus Phenyl, Indol, Indolin, 1,2,3,4-Tetrahydrochinolin, 3,4-Dihydro-2*H-*1,4-benzoxazin, Indan, 1*H-*Indazol, 1*H*-Pyrrolo[2,3-*b*]pyridin, Pyrimidin, Cyclobutylmethyl, Cyclopropylmethyl, 1*H-*Pyrazol, Pyridin oder Pyridazin bedeutet, wobei diese Gruppen gegebenenfalls einen oder mehrere Substituenten ausgewählt aus Halogen, geradkettigem oder verzweigtem (C₁-C₆)-Alkyl, Cyano oder geradkettigem oder verzweigtem (C₁-C₆)-Alkoxy aufweisen.

7. Verbindung der Formel (I) nach Anspruch 1, ausgewählt aus der folgenden Gruppe:
- N-(4-Hydroxyphenyl)-3-{6-[((3*S*)-3-(4-morpholinylmethyl)-3,4-dihydro-2(1*H*)-isochinolinyl)-carbonyl]-1,3-benzodioxol-5-yl}-*N*-phenyl-5,6,7,8-tetrahydro-1*H*)-indolizin-carboxamid,
- 3-{5-Chlor-2-[((3*S*-3-(4-morpholinylmethyl)-3,4-dihydro-2(1*H*)-isochinolinyl)-carbonyl]-phenyl}-*N*-(4-hydroxyphenyl)-*N*-(1-methyl-1*H*-indol-5-yl)-5,6,7,8-tetrahydro-1-indolizin-carboxamid,
- N-(4-Hydroxyphenyl)-*N*-(1-methyl-1*H*-indazol-5-yl)-3-{2,2-dideuterio-6-[((3*S*)-3-(4-morpholinylmethyl)-3,4-dihydro-2(1*H*)-isochinolinyl)-carbonyl]-1,3-benzodioxol-5-yl}-5,6,7,8-tetrahydro-1-indolizin-carboxamid,
- *N*-(4-Hydroxyphenyl)-*N*-(1-methyl-1*H*-indazol-5-yl)-3-(6-{[(3*S*)-3-(morpholin-4-ylmethyl)-3,4-dihydroisochinolin-2(1*H*)-yl]-carbonyl}-1,3-benzodioxol-5-yl)-5,6,7,8-tetrahydroindolizin-1-carboxamid,
- *N*-(4-Hydroxyphenyl)-3-{7-[((3*S*)-3-(4-morpholinylmethyl)-3,4-dihydro-2(1*H*)-isochinolinyl)-carbonyl]-2,3-dihydro-1,4-benzodioxin-6-yl}-*N*-pheny1-5,6,7,8-tetrahydro-1-indolizin-carboxamid,
- *N*-(4-Hydroxyphenyl)-*N*-(1-methyl-1*H*-indol-5-yl)-3-{6-[((3*S*)-3-[(4-methyl-1-piperazinyl)-methyl]-3,4-dihydro-2(1*H*)-isochinolinyl)-carbonyl]-1,3-benzo-dioxol-5-yl}-1-indolizin-carboxamid,
- *N*-[4-(Hydroxy)-phenyl]-*N*-(1-methyl-1*H*-indol-5-yl)-3-{6-[((3*S*)-3-(4-morpholinylmethyl)-3,4-dihydro-2(1*H*)-isochinolinyl)-carbonyl]-1,3-benzodioxol-5-yl}-5,6,7,8- tetrahydro-1-indolizin-carboxamid,
- *N*-(4-Hydroxyphenyl)-3-{6-[((3*S*)-3-(4-morpholinylmethyl)-3,4-dihydro-2(1*H*)-isochinolinyl)-carbonyl]-1,3-benzodioxol-5-yl}-*N-*phenyl-1-indolizin-carboxamid,
- 3-{5-Chlor-2-[((3*S*)-3-(4-morpholinylmethyl)-3,4-dihydro-2(1*H*)-isochinolinyl)-carbonyl]-plienyl}-*N*-(4-hydroxyphenyl)-*N*-(1-methyl-1H-indol-5-yl)-1-indolizin-carboxamid,
- 6-{5-Chlor-2-[((3*S*)-3-(4-morpholinylmethyl)-3,4-dihydro-2(1*H*-isochinolinyl)-carbonyl]-phenyl}-*N*-(3-fluor-4-methylphenyl)-*N*-(4-hydroxyphenyl)-3,4-dihydro-1*H*-pyrrolo[2,1-c][1,4]oxazin-8-carboxamid,
- 3-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-*N*-(1-methyl-1*H*-pyrazol-4-yl)-5,6,7,8-tetrahydroindolizin-1-carboxamid,
- *N*-(3-Fluor-4-methylphenyl)-*N*-(4-hydroxyphenyl)-3-{6-[((3*S*)-3-(4-morpholinylmethyl)-3,4-dihydro-2(1*H*-isochinolinyl)-carbonyl]-1,3-benzodioxol-5-yl}-5,6,7,8-tetrahydro-1-indolizin-carboxamid,
- *N*-[4-(Hydroxy)-phenyl]-*N*-(1-methyl-2,3-dihydro-1*H*-indol-5-yl)-3-{6-[((3*S*)-3-(4-morpholinylmethyl)-3,4-dihydro-2(1*H*-isochinolinyl)-carbonyl]-1,3-benzo-dioxol-5-yl}-5,6,7,8-tetrahydro-1-indolizin-carboxamid,
- 3-{5-Chlor-2-[((3*S*)-3-(4-morpholinylmethyl)-3,4-dihydro-2(1*H*)-isochinolinyl)-carbonyl]-phenyl}-*N-*(4-hydroxyphenyl)-*N-*(1-methyl-2,3-dihydro-1*H-*indol-5-yl)-5,6,7,8-tetrahydro-1-indolizin-carboxamid,
- *N*-(4-Hydroxyphenyl)-*N*-(1-methyl-1*H*-indol-5-yl)-3-{6-[((3*S*)-3-(4-morpholinylmethyl)-3,4-dihydro-2(1*H*-isochinolinyl)-carbonyl]-1,3-benzodioxol-5-yl}-1-indolizin-carboxamid,
- 3-{5-Chlor-2-[((3*S*)-3-(4-morpholinylmethyl)-3,4-dihydro-2(1*H*)-isochinolinyl)-carbonyl]-phenyl}-*N*-(4-hydroxyphenyl)-*N*-(1-methyl-2,3-dihydro-1*H*-indol-5-yl)-1- indolizin-carboxamid,
- 6-{5-Chlor-2-[((3*S*)-3-(4-morpholinylmethyl)-3,4-dihydro-2(1*H*)-isochinolinyl)-carbonyl]-phenyl}-*N*-(4-hydroxyphenyl)-*N*-phenyl-3,4-dihydro-1*H-*pyrrolo[2,1-c][1,4]oxazin-8-carboxamid,
- *N*-(3-Fluorphenyl)-*N*-(4-hydroxyphenyl)-3-(6-{[(3*S*)-3-(morpholin-4-ylmethyl)-3,4-dihydroisochinolin-2(1*H*)-yl]-carbonyl}-1,3-benzodioxol-5-yl)-5,6,7,8-tetrahydroindolizin-1-carboxamid,
deren Enantiomere und Diastereoisomere sowie deren Additionssalze mit einer pharmazeutisch annehmbaren Säure oder Base.

8. Verbindung der Formel (I) nach Anspruch 1, ausgewählt aus der folgenden Gruppe:
- *N*-(4-Hydroxyphenyl)-3-{6-[((3*S*)-3-(4-morpholinylmethyl)-3,4-dihydro-2(1*H-*isochinolinyl)-carbonyl]-1,3-benzodioxol-5-yl}-*N*-phenyl-5,6,7,8-tetrahydro-1-indolizin-carboxamid,
- 3-{5-Chlor-2-[((3*S*)-3-(4-morpholinylmethyl)-3,4-dihydro-2(1*H*-isochinolinyl)-carbonyl]-phenyl}-*N*-(4-hydroxyphenyl)-*N*-(1-methyl-1*H*-indol-5-yl)-5,6,7,8-tetrahydro-1-indolizin-carboxamid,
- *N*-(4-Hydroxyphenyl)-*N*-(1-methyl-1*H*-indazol-5-yl)-3-{2,2-dideuterio-6-[((3*S*)-3-(4-morpholinylmethyl)-3,4-dihydro-2(1*H*)-isochinolinyl)-carbonyl]-1,3-benzodioxol-5-yl}-5,6,7,8-tetrahydro-1-indolizin-carboxamid,
- N-(4-Hydroxyphenyl)-*N*-(1-methyl-1*H-*indazol-5-yl)-3-(6-{[(3*S*)-3-(morpholin-4-ylmethyl)-3,4-dihydroisochinolin-2(1*H*)-yl]-carbonyl}-1,3-benzodioxol-5-yl)-5,6,7,8-tetrahydroindolizin-1-carboxamid,
- *N*-(4-Hydroxyphenyl)-3-{7-[((3*S*)-3-(4-morpholinylmethyl)-3,4-dihydro-2(1H)-isochinolinyl)-carbonyl]-2,3-dihydro-1,4-benzodioxin-6-yl}-*N*-phenyl-5,6,7,8-tetrahydro-1 -indolizin-carboxamid,
- *N*-(4-Hydroxyphenyl)-*N*-(1-methyl-1*H*-indol-5-yl)-3-{6-[((3*S*)-3-[(4-methyl-1-piperazinyl)-methyl]-3,4-dihydro-2(1*H*)-isochinolinyl)-carbonyl]-1,3-benzodioxol-5-yl}-1-indolizin-carboxamid,
- *N*-[4-(Hydroxy)-phenyl]-*N*-(1-methyl-1*H*-indol-5-yl)-3-{6-[((3*S*)-3-(4-morpholinylmethyl)-3,4-dihydro-2(1*H*)-isochinolinyl)-carbonyl]-1,3-benzodioxol-5-yl}-5,6,7,8-tetrahydro-1-indolizin-carboxamid,
- *N*-(4-Hydroxyphenyl)-3-{6-[((3*S*)-3-(4-morpholinylmethyl)-3,4-dihydro-2(1*H*)-isochinolinyl)-carbonyl]-1,3-benzodioxol-5-yl}-*N*-phenyl-1-indolizin-carboxamid,
- 3-{5-Chlor-2-[((3*S*)-3-(4-morpholinylmethyl)-3,4-dihydro-2(1*H*)-isochinolinyl)-carbonyl]-phenyl}-*N*-(4-hydroxyphenyl)-*N*-(1-methyl-1*H*-indol-5-yl)-1-indolizin-carboxamid,
- 6-{5-Chlor-2-[((3*S*)-3-(4-morpholinylmethyl)-3,4-dihydro-2(1*H*)-isochinolinyl)-carbonyl]-phenyl}-*N-*(3-fluor-4-methylphenyl)-*N-*(4-hydroxyphenyl)-3,4-dihydro-1*H*-pyrrolo[2,1-c][1,4]oxazin-8-carboxamid,
- 3-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-*N*-(1-methyl-1*H*-pyrazol-4-yl)-5,6,7,8-tetrahydroindolizin-1-carboxamid,
deren Enantiomere und Diastereoisomere sowie deren Additionssalze mit einer pharmazeutisch annehmbaren Säure oder Base.

9. Verbindung der Formel (I) nach Anspruch 8, nämlich *N*-(4-Hydroxyphenyl)-3-{6-[((3*S*)-3-(4-morpholinylmethyl)-3,4-dihydro-2(1*H*)-isochinolinyl)-carbonyl]-1,3-benzodioxol-5-yl}-*N*-phenyl-5,6,7,S-tetrahydro-1-indolizin-carboxamid.

10. Verbindung der Formel (I) nach Anspruch 8, nämlich 3-{5-Chlor-2-[((3*S*)-3-(4-morpholinylmethyl)-3,4-dihydro-2(1*H*)-isochinolinyl)-carbonyl]-phenyl}-*N*-(4-hydroxyphenyl)-*N*-(1-methyl-1*H*-indol-5-yl)-5,6,7,8-tetrahydro-1-indolizin-carboxamid.

11. Verbindung der Formel (I) nach Anspruch 8, nämlich *N-*(4-Hydroxyphenyl)-*N-*(1-methyl-1*H*-indazol-5-yl)-3-{2,2-dideuterio-6-[((3*S*)-3-(4-morpholinylmethyl)-3,4-dihydro-2(1*H*)-isochinolinyl)-carbonyl]-1,3-benzodioxol-5-yl}-5,6,7,8-tetrahydro-1-indolizin-carboxamid.

12. Verbindung der Formel (I) nach Anspruch 8, nämlich *N*-(4-Hydroxyphenyl)-*N-*(1-methyl-1*H-*indazol-5-yl)-3-(6-{[(3*S*)-3-(morpholin-4-ylmethyl)-3,4-dihydroisochinolin-2(1*H*)-yl]-carbonyl}-1,3-benzodioxol-5-yl)-5,6,7,8-tetrahydroindolizin-1-carboxamid.

13. Verbindung der Formel (I) nach Anspruch 8, nämlich *N*-(4-Hydroxyphenyl)-3-{7-[((3*S*)-3-(4-morpholinylmethyl)-3,4-dihydro-2(1*H*)-isochinolinyl)-carbonyl]-2,3-dihydro-1,4-benzodioxin-6-yl}-*N*-phenyl-5,6,7,8-tetrahydro-1-indolizin-carboxamid.

14. Verbindung der Formel (I) nach Anspruch 8, nämlich *N*-(4-Hydroxyphenyl)-*N-*(1-methyl-1*H*-indol-5-yl)-3-{6-[((3*S*)-3-[(4-methyl-1-piperazinyl)-methyl]-3,4-dihydro-2(1*H*)-isochinolinyl)-carbonyl]-1,3-benzodioxol-5-yl}-1-indolizin-carboxamid.

15. Verbindung der Formel (I) nach Anspruch 8, nämlich *N*-[4-(Hydroxy)-phenyl]-*N*-(1-methyl-1*H-*indol-5-yl)-3-{6-[((3*S*)-3-(4-morpholinylmethyl)-3,4-dihydro-2(1*H*)-isochinolinyl)-carbonyl]-1,3-benzodioxol-5-yl}-5,6,7,8-tetrahydro-1-indolizin-carboxamid.

16. Verbindung der Formel (I) nach Anspruch 8, nämlich *N*-(4-Hydroxyphenyl)-3-{6-[((3*S*)-3-(4-morpholinylmethyl)-3,4-dihydro-2(1*H*)-isochinolinyl)-carbonyl]-1,3-benzodioxol-5-yl}-*N*-phenyl-1-indolizin-carboxamid.

17. Verbindung der Formel (I) nach Anspruch 8, nämlich 3-{5-Chlor-2-[((3*S*)-3-(4-morpholinylmethyl)-3,4-dihydro-2(1*H*)-isochinolinyl)-carbonyl]-phenyl}-*N*-(4-hydroxyphenyl)-*N*-(1-methyl-1*H*-indol-5-yl)-1-indolizin-carboxamid.

18. Verbindung der Formel (I) nach Anspruch 8, nämlich 6-{5-Chlor-2-[((3*S*)-3-(4-morpholinylmethyl)-3,4-dihydro-2(1*H*)-isochinolinyl)-carbonyl]-phenyl}-*N-*(3-fluor-4-methylphenyl)-*N*-(4-hydroxyphenyl)-3,4-dihydro-1*H*-pyrrolo[2,1-c][1,4]oxazin-8-carboxamid.

19. Verbindung der Formel (I) nach Anspruch 8, nämlich 3-(5-Chlor-2-{[(3*S*)-3-(morpholin-4-ylmethyl)-3,4-dihydroisochinolin-2(1*H*)-yl]-carbonyl}-phenyl)-*N*-(4-hydroxyphenyl)-*N*-(1-methyl-1*H*-pyrazol-4-yl)-5,6,7,8-tetrahydroindolizin-1-carboxamid.

20. Hydrochloridsalz einer Verbindung der Formel (I) nach einem der Ansprüche 9 bis 13 und 15 bis 19.

21. Dihydrochloridsalz der Verbindung der Formel (I) nach Anspruch 14.

22. Verfahren zur Herstellung einer Verbindung der Formel (I) nach Anspruch 1, **dadurch gekennzeichnet, dass** man als Ausgangsprodukt die Verbindung der Formel (II): 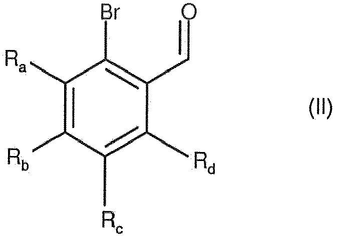 in der Rₐ, R_{b}, R_{c} und R_{d} die bezüglich der Formel (I) angegebenen Bedeutungen besitzen, verwendet,
welche Verbindung der Formel (II) einer Heck-Reaktion in wässrigem oder organischem Medium, in Gegenwart eines Palladiumkatalysators, einer Base, eines Phosphins und der Verbindung der Formel (III): 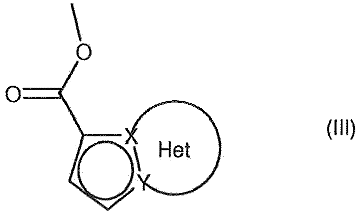 in der die Gruppen X, Y und Het die bezüglich der Formel (I) angegebenen Bedeutungen besitzen, unterworfen wird,
zur Bildung der Verbindung der Formel (IV): 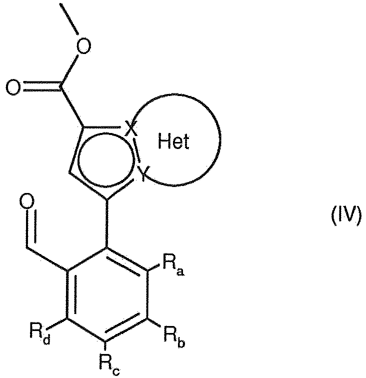 in der Rₐ, R_{b}, R_{c}, R_{d}, X, Y und Het die bezüglich der Formel (I) angegebenen Bedeutungen besitzen,
die Aldehydfunktion der Verbindung der Formel (IV) zu der Carbonsäure oxidiert wird zur Bildung der Verbindung der Formel (V): 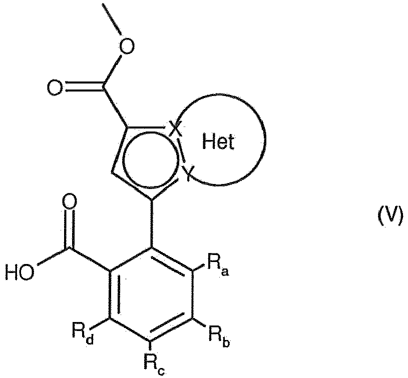 in der Rₐ, R_{b}, R_{c}, R_{d}, X, Y und Het die bezüglich der Formel (I) angegebenen Bedeutungen besitzen,
welche Verbindung der Formel (V) anschließend einer Peptidkupplung mit einer Verbindung der Formel (VI): 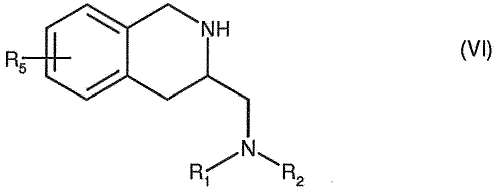 in der R₁, R₂ und R₅ die bezüglich der Formel (I) angegebenen Bedeutungen besitzen, unterzogen wird
zur Bildung der Verbindung der Formel (VII): 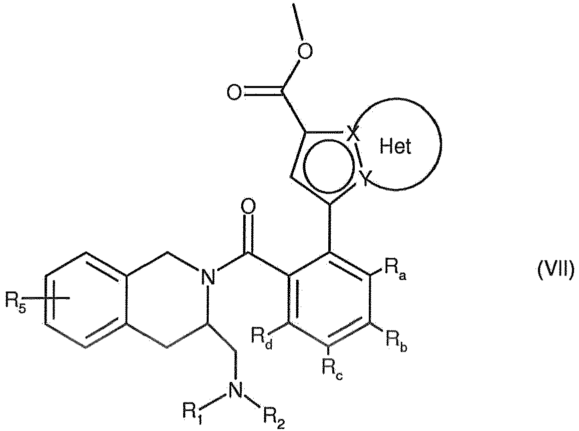 in der Rₐ, R_{b}, R_{c}, R_{d}, R₁, R₂, R₅, X, Y und Het die bezüglich der Formel (I) angegebenen Bedeutungen besitzen,
die Esterfunktion der Verbindung der Formel (VII) hydrolysiert wird zur Bildung der entsprechenden Carbonsäure oder des entsprechenden Carboxylats, welches in ein Säurederivat, wie das entsprechende Acylchlorid oder Anhydrid umgewandelt werden kann, bevor es mit einem Amin NHR₃R₄, worin R₃ und R₄ die bezüglich der Formel (I) angegebenen Bedeutungen besitzen, gekuppelt wird, zur Bildung der Verbindung der Formel (I),
welche Verbindung der Formel (I) gemäß einer klassischen Trennmethode gereinigt werden kann, welche man gewünschtenfalls in ihre Additionssalze mit einer pharmazeutisch annehmbaren Säure oder Base überführt und die man gegebenenfalls mit Hilfe einer klassischen Trennmethode in die Isomeren auftrennt,
wobei es sich versteht, dass in jedem geeigneten Augenblick im Verlaufe des oben beschriebenen Verfahrens bestimmte Gruppen (Hydroxy, Amino...) der Reaktionsteilnehmer oder Synthesezwischenprodukte zum Zwecke der Synthese geschützt und dann von den Schutzgruppen wieder befreit werden können.

23. Verfahren nach Anspruch 22 zur Herstellung einer Verbindung der Formel (I), in der eine der Gruppen R₃ oder R₄ durch eine Hydroxygruppe substituiert ist, **dadurch gekennzeichnet, dass** das Amin NHR₃R₄ zuvor einer Reaktion zum Schutz der Hydroxyfunktion unterworfen wird vor der Kupplung mit der Carbonsäure der Verbindung der Formel (VII) oder einem entsprechenden Säurederivat, die gebildete geschützte Verbindung der Formel (I) anschließend einer Reaktion zur Abspaltung der Schutzgruppe unterworfen und dann gegebenenfalls in eines ihrer Additionssalze mit einer pharmazeutisch annehmbaren Säure oder Base umgewandelt wird.

24. Pharmazeutische Zubereitung enthaltend eine Verbindung der Formel (I) nach einem der Ansprüche 1 bis 19 oder eines ihrer Additionssalze mit einer pharmazeutisch annehmbaren Säure oder Base in Kombination mit einem oder mehreren pharmazeutisch annehmbaren Hilfsstoffen.

25. Pharmazeutische Zubereitung nach Anspruch 24 zur Verwendung als proapoptotisches Mittel.

26. Pharmazeutische Zubereitung nach Anspruch 24 zur Verwendung bei der Behandlung von Krebs, Immunerkrankungen und Autoimmunerkrankungen.

27. Pharmazeutische Zubereitung nach Anspruch 24 zur Verwendung bei der Behandlung von Blasenkrebs, Gehirnkrebs, Brustkrebs, Uteruskrebs, chronisch-lymphatischen Leukämien, Kolorektalkrebs, Speiseröhrenkrebs, Leberkrebs, lymphoblastischen Leukämien, follikulären Lymphomen, Melanomen, bösartigen Hämopathien, Myelomen, Ovarialkrebs, nicht-kleinzelligem Lungenkrebs, Prostatakrebs und kleinzelligem Lungenkrebs.

28. Verbindung der Formel (I) nach einem der Ansprüche 1 bis 19 oder eines ihrer Additionssalze mit einer pharmazeutisch annehmbaren Säure oder Base zur Verwendung bei der Behandlung von Blasenkrebs, Gehirnkrebs, Brustkrebs, Uteruskrebs, chronisch-lymphatischen Leukämien, Kolorektalkrebs, Speiseröhrenkrebs, Leberkrebs, lymphoblastischen Leukämien, follikulären Lymphomen, Melanomen, bösartigen Hämopathien, Myelomen, Ovarialkrebs, nicht-kleinzelligem Lungenkrebs, Prostatakrebs und kleinzelligem Lungenkrebs.

29. Kombination einer Verbindung der Formel (I) nach einem der Ansprüche 1 bis 21 mit einem Antikrebsmittel ausgewählt aus genotoxischen Mitteln, Mitosegiften, Antimetaboliten, Proteasom-Inhibitoren, Kinase-Inhibitoren oder Antikörpern.

30. Pharmazeutische Zubereitung enthaltend eine Kombination nach Anspruch 29 in Kombination mit einem oder mehreren pharmazeutisch annehmbaren Hilfsstoffen.

31. Kombination nach Anspruch 29 zur Verwendung bei der Behandlung von Krebs.

32. Verbindung der Formel (I) nach einem der Ansprüche 1 bis 21 zur Verwendung in Kombination mit einer Strahlentherapie bei der Behandlung von Krebs.

## Claims

1. Compound of formula (I): 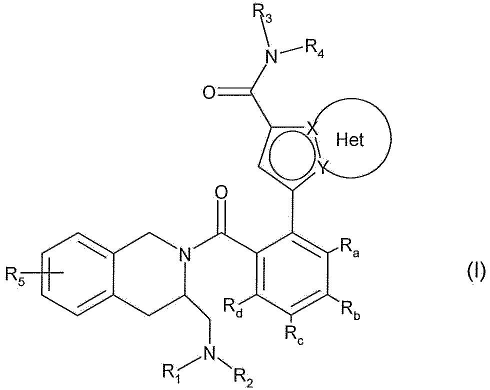 wherein:
◆ X and Y represent a carbon atom or a nitrogen atom, it being understood that they may not simultaneously represent two carbon atoms or two nitrogen atoms,
◆ the Het moiety of the group 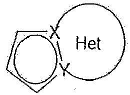 represents an optionally substituted, aromatic or non-aromatic ring composed of 5, 6 or 7 ring members, which may contain, in addition to the nitrogen represented by X or by Y, from one to 3 hetero atoms selected independently from oxygen, sulphur and nitrogen, it being understood that the nitrogen in question may be substituted by a group representing a hydrogen atom, a linear or branched (C₁-C₆)alkyl group or a group -C(O)-O-Alk wherein Alk is a linear or branched (C₁-C₆)alkyl group,
◆ R₁ and R₂ independently of one another represent a hydrogen atom or a linear or branched (C₁-C₆)allcyl group,
or R₁ and R₂ form with the nitrogen atom carrying them a heterocycloalkyl composed of from 4 to 7 ring members, which may contain, in addition to the nitrogen atom, another hetero atom selected from oxygen, sulphur, SO₂ and NR wherein R represents a hydrogen atom, a linear or branched (C₁-C₆)alkyl group, a (C₁-C₆)alkylsulphonyl group, a linear or branched (C₁-C₆)polyhaloalkyl group or a group -C(O)-O-Alk wherein Alk is a linear or branched (C₁-C₆)alkyl group,
◆ R₃ represents a linear or branched (C₁-C₆)alkyl group, a (C₂-C₆)alkenyl group, a (C₂-C₆)alkynyl group, a cycloalkyl group, a linear or branched (C₄-C₁₀)cycloalkyl-(C₁-C₆)alkyl group, an aryl group or a heteroaryl group,
◆ R₄ represents an aryl, heteroaryl, cycloalkyl or linear or branched (C₁-C₆)alkyl group,
◆ R₅ represents a hydrogen atom or a halogen atom,
◆ Rₐ, R_{b}, R_{c} and R_{d} independently of one another represent a hydrogen atom, a halogen atom, a linear or branched (C₁-C₆)alkyl group, a linear or branched (C₁-C₆)alkoxy group, a hydroxy group, a linear or branched (C₁-C₆)polyhaloalkyl group, or a trifluoromethoxy group, or the substituents of one of the pairs (Rₐ,R_{b}), (R_{b},R_{c}) or (R_{c},R_{d}) form together with the carbon atoms carrying them a ring composed of from 5 to 7 ring members, which may contain from one to 3 hetero atoms selected from oxygen, sulphur and nitrogen, it being understood that the nitrogen in question may be substituted by a group representing a hydrogen atom, a linear or branched (C₁-C₆)alkyl group or a group -C(O)-O-Alk wherein Alk is a linear or branched (C₁-C₆)alkyl group, it also being understood that one or more carbon atoms of the ring defined hereinbefore may be deuterated,
it being understood that:
- "aryl" means a phenyl, naphthyl, biphenyl or indenyl group,
- "heteroaryl" means any mono- or bi-cyclic group composed of from 5 to 10 ring members, having at least one aromatic moiety and containing from 1 to 3 hetero atoms selected from oxygen, sulphur and nitrogen,
- "cycloalkyl" means any mono- or bi-cyclic non-aromatic carbocyclic group containing from 4 to 10 ring members,
it being possible for the alkyl, aryl, heteroaryl, cycloalkyl and heterocycloalkyl groups so defined to be substituted by from 1 to 3 groups selected from optionally substituted linear or branched (C₁-C₆)alkyl, (C₃-C₆)spiro, linear or branched (C₁-C₆)alkoxy, (C₁-C₆)alkyl-S-, hydroxy, oxo (or *N*-oxide where appropriate), nitro, cyano, -COOR', NR'R", linear or
branched (C₁-C₆)polyhaloalkyl, trifluoromethoxy, (C₁-C₆)alkylsulphonyl or halogen, it being understood that R' and R" independently of one another represent a hydrogen atom or a linear or branched (C₁-C₆)alkyl group,
it being possible for the Het moiety of the group 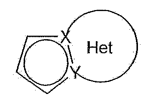 defined hereinbefore to be substituted by a group selected from linear or branched (C₁-C₆)alkyl, hydroxy, NR₁'R₁" and halogen, it being understood that R₁' and R₁" have the same definitions as the groups R' and R" mentioned hereinbefore,
their enantiomers and diastereoisomers, and addition salts thereof with a pharmaceutically acceptable acid or base.

2. Compound of formula (I) according to claim 1, wherein the group 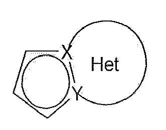 represents one of the following groups: 5,6,7,8-tetrahydroindolizine optionally substituted by a hydroxy, indolizine, 1,2,3,4-tetrahydropyrrolo[1,2-α]pyrazine, tert-butyl 3,4-dihydro-pyrrolo[1,2-α]pyrazine-2(1*H*)-carboxylate, 3,4-dihydro-1*H-*pyrrolo[2,1-*c*][1,4]oxazine, 2,3-dihydro-1*H*-pyrrolizine optionally substituted by a hydroxy, 6,7,8,9-tetrahydro-5*H-*pyrrolo[1,2-α]azepine or pyrrolo[1,2-α]pyrazine.

3. Compound of formula (I) according to claim 1, wherein R₁ and R₂ each represents an alkyl group optionally substituted by a methoxy, or R₁ and R₂ form with the nitrogen atom carrying them a heterocycloalkyl selected from the following groups: morpholine optionally substituted by one or more linear or branched (C₁-C₆)alkyl(s), oxidomorpholine, thiomorpholine 1,1-dioxide, 1,4-oxazepan, 3-methoxypyrrolidine, 3,3-difluoropyrrolidine, 3-methoxyazetidine, 3-fluoroazetidine, oxopiperazine or piperazine, the last two groups being substituted by a linear or branched (C₁-C₆)alkyl group, linear or branched (C₁-C₆)-polyhaloalkyl group or methylsulphonyl group.

4. Compound of formula (I) according to claim 1, wherein Rₐ and R_{d} each represents a hydrogen atom and (R_{b},R_{c}) form together with the carbon atoms carrying them a 1,3-dioxolane group, one of the carbon atoms of which is optionally deuterated, a 1,4-dioxane group, a 1,4-dioxepan group, or Rₐ, R_{c} and R_{d} each represents a hydrogen atom and R_{b} represents a halogen, a methyl, a methoxy, an ethoxy, a trifluoromethyl or a trifluoromethoxy.

5. Compound of formula (I) according to claim 1, wherein R₄ represents a 4-hydroxyphenyl group, a 3-fluoro-4-hydroxyphenyl group or a 5-hydroxypyrimidine group.

6. Compound of formula (I) according to claim 1, wherein R₃ represents a group selected from phenyl, indole, indoline, 1,2,3,4-tetrahydroquinoline, 3,4-dihydro-2*H-*1,4-benzoxazine, indane, 1*H-*indazole, 1*H-*pyrrolo[2,3-*b*]pyridine, pyrimidine, cyclobutylmethyl, cyclopropylmethyl, 1*H*-pyrazole, pyridine, pyridazine, those groups optionally having one or more substituents selected from halogen, linear or branched (C₁-C₆)alkyl, cyano and linear or branched (C₁-C₆)alkoxy.

7. Compound of formula (I) according to claim 1, selected from the following group:
- *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-5,6,7,8-tetrahydro-1-indolizine carboxamide,
- 3-{5-chloro-2-[((3*S*)-3-(4-morpholinylmethyl)-3,4-dihydro-2(1*H*)-isoquinolinyl)-carbonyl]phenyl}-*N-*(4-hydroxyphenyl)-*N*-(1-methyl-1*H-*indol-5-yl)-5,6,7,8-tetrahydro-1-indolizine carboxamide,
- *N*-(4-hydroxyphenyl)-*N*-(1-methyl-1*H*-indazol-5-yl)-3-{2,2-dideuterio-6-[((3*S*)-3-(4-morpholinylmethyl)-3,4-dihydro-2(1*H*)-isoquinolinyl)carbonyl]-1,3-benzodioxol-5-yl}-5,6,7,8-tetrahydro-1-indolizine carboxamide,
- *N*-(4-hydroxyphenyl)-*N*-(1-methyl-1*H*-indazol-5-yl)-3-(6-{[(3*S*)-3-(morpholin-4-ylmethyl)-3,4-dihydroisoquinolin-2(1*H*)-yl]carbonyl}-1,3-benzodioxol-5-yl)-5,6,7,8-tetrahydroindolizine-1-carboxamide,
- *N*-(4-hydroxyphenyl)-3-{7-[((3*S*)-3-(4-morpholinylmethyl)-3,4-dihydro-2(1*H-*isoquinolinyl)carbonyl]-2,3-dihydro-1,4-benzodioxin-6-yl}-*N*-phenyl-5,6,7,8-tetrahydro-1-indolizine carboxamide,
- *N*-(4-hydroxyphenyl)-*N*-(1-methyl-1*H*-indol-5-yl)-3-{6-[((3*S*)-3-[(4-methyl-1-piperazinyl)methyl]-3,4-dihydro-2(1*H*)-isoquinolinyl)carbonyl]-1,3-benzodioxol-5-yl}-1-indolizine carboxamide,
- *N*-[4-(hydroxy)phenyl]-*N*-(1-methyl-1*H*-indol-5-yl)-3-{6-[((3*S*)-3-(4-morpholinylmethyl)-3,4-dihydro-2(*H*)-isoquinolinyl)carbonyl]-1,3-benzodioxol-5-yl}-5,6,7,8-tetrahydro-1-indolizine carboxamide,
- *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-1-indolizine carboxamide,
- 3-{5-chloro-2-[((3*S*)-3-(4-morpholinylmethyl)-3,4-dihydro-2(1*H*)-isoquinolinyl)carbonyl]pbenyl}-*N*-(4-hydroxyphenyl)-*N*-(1-methyl-1*H*-indol-5-yl)-1-indolizine carboxamide,
- 6-{5-chloro-2-[((3*S*)-3-(4-morpholinylmethyl)-3,4-dihydro-2(1*H*)-isoquinolinyl)-carbonyl]phenyl}-*N-*(3-fluoro-4-methylphenyl)-*N-*(4-hydroxyphenyl)-3,4-dihydro-1*H*-pyrrolo[2,1-*c*][1,4] oxazine-8-carboxamide,
- 3-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-*N*-(1-methyl-1*H*-pyrazol-4-yl)-5,6,7,8-tetrahydroindolizine-1-carboxamide,
- *N*-(3-fluoro-4-methylphenyl)-*N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5yl}-5,6,7,8-tetrahydro-1-indolizine carboxamide,
- *N*-[4-(hydroxy)phenyl]-*N*-(1-methyl-2,3-dihydro-1*H*-indol-5-yl)-3-{6-[((3*S*)-3-(4-morpholinylmethyl)-3,4-dihydro-2(1*H*)-isoquinolinyl)carbonyl]-1,3-benzodioxol-5-yl}-5,6,7,8-tetrahydro-1-indolizine carboxamide,
- 3-{5-chloro-2-[((3*S*-3-(4-morpholinylmethyl)-3,4-dihydro-2(1*H*)-isoquinolinyl)carbonyl]phenyl}-*N-*(4-hydroxyphenyl)-*N-*(1-methyl-2,3-dihydro-1*H*-indol-5-yl)-5,6,7,8-tetrahydro-1-indolizine carboxamide,
- *N*-(4-hydroxyphenyl)-*N*-(1-methyl-1*H*-indol-5-yl)-3-{6-[((3*S*)-3-(4-morpholinylmethyl)-3,4-dihydro-2(1*H*)-isoquinolinyl)carbonyl]-1,3-benzodioxol-5-yl}-1-indolizine carboxamide,
- 3-{5-chloro-2-[((3*S*)-3-(4-morpholinylmethyl)-3,4-dihydro-2(1*H*)-isoquinolinyl)-carbonyl]phenyl}-*N*-(-4-hydroxyphenyl)-*N*-(1-methyl-2,3-dihydro-1*H*-indol-5-yl)-1 indolizine carboxamide,
- 6-{5-chloro-2-[((3*S*)-3-(4-morpholinylmethyl)-3,4-dihydro-2(1*H*)-isoquinolinyl)-carbonyl]phenyl}-*N*-(4-hydroxyphenyl)-*N*-phenyl-3,4-dihydro-1*H*-pyrrolo[2,1-c]-[1,4]oxazine-8-carboxamide,
- *N*-(3-fluorophenyl)-*N*-(4-hydroxyphenyl)-3-(6-{[(3*S*)-3-(morpholin-4-ylmethyl)-3,4-dihydroisoquinolin-2(1*H*)-yl]carbonyl}-1,3-benzodioxol-5-yl)-5,6,7,8-tetrahydroindolizine-1-carboxamide,
their enantiomers and diastereoisomers, and addition salts thereof with a pharmaceutically acceptable acid or base.

8. Compound of formula (I) according to claim 1, selected from the following group:
- *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-5,6,7,8-tetrahydro-1-indolizine carboxamide,
- 3-{5-chloro-2-[((3*S*)-3-(4-morpholinylmethyl)-3,4-dihydro-2(1*H*)-isoquinolinyl)-carbonyljphenyl}-*N-*(4-hydroxyphenyl)-*N*-(1-methyl-1*H*-indol-5-yl)-5,6,7,8-tetrahydro-1-indolizine carboxamide,
- *N*-(4-hydroxyphenyl)-*N*-(1-methyl-1*H*-indazol-5-yl)-3-{2,2-dideuterio-6-[((3*S*)-3-(4-morpholinylmethyl)-3,4-dihydro-2(1*H*)-isoquinolinyl)carbonyl]-1,3-benzo-dioxol-5-yl)-5,6,7,8-tetrahydro-1-indolizine carboxamide,
- *N*-(4-hydroxyphenyl)-*N*-(1-methyl-1*H*-indazol-5-yl)-3-(6-{[(3*S*)-3-(morpholin-4-ylmethyl)-3,4-dihydroisoquinolin-2(1*H*)-yl]carbonyl}-1,3-benzodioxol-5-yl)-5,6,7,8-tetrahydroindolizine-1-carboxamide,
- *N*-(4-hydroxyphenyl)-3-{7-[((3*S*-3-(4-morpholinylmethyl)-3,4-dihydro-2(1*H*)-isoquinolinyl)carbonyl]-2,3-dihydro-1,4-benzodioxin-6-yl}-*N*-phenyl-5,6,7,8-tetrahydro-1-indolizine carboxamide,
- *N*-(4-hydroxyphenyl)-*N*-(1-methyl-1*H*-indol-5-yl)-3-{6-[((3S)-3-[(4-methyl-1-piperazinyl)methyl]-3,4-dihydro-2(1*H*)-isoquinolinyl)carbonyl]-1,3-benzodioxol-5-yl}-1-indolizine carboxamide,
- *N*-[4-(hydroxy)phenyl]-*N-*(1-methyl-1*H-*indol-5-yl)-3-{6-[((3*S*)-3-(4-morpholinylmethyl)-3,4-dihydro-2(1*H*)-isoquinolinyl)carbonyl]-1,3-benzodioxol-5-yl}-5,6,7,8-tetrahydro-1-indolizine carboxamide,
- *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-1-indolizine carboxamide,
- 3-{5-chloro-2-[((3*S*)-3-(4-morpho]dihylmethyl)-3,4-dihydro-2(1*H*)-isoquinolinyl)carbonyl]phenyl}-*N*-(4-hydroxyphenyl)-*N*-(1-methyl-1*H*-indol-5-yl)-1-indolizine carboxamide,
- 6-{5-chloro-2-[((3*S*)-3-(4-morpholinylmethyl)-3,4-dihydro-2(1*H*)-isoquinolinyl)-carbonyl]phenyl}-*N*-(3-fluoro-4-methylphenyl)-*N-*(4-hydroxyphenyl)-3,4-dihydro-1*H*-pyrrolo[2,1-c][1,4]oxazine-8-carboxamide,
- 3-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-*N*-(1-methyl-1*H*-pyrazol-4-yl)-5,6,7,8-tetrahydroindolizine-1-carboxamide,
their enantiomers and diastereoisomers, and addition salts thereof with a pharmaceutically acceptable acid or base.

9. Compound of formula (I) according to claim 8 which is *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-5,6,7,8-tetrahydro-1-indolizine carboxamide.

10. Compound of formula (I) according to claim 8 which is 3-{5-chloro-2-[((3*S*)-3-(4-morpholinylmethyl)-3,4-dihydro-2(1*H*)-isoquinolinyl)carbonyl]phenyl}-*N*-(4-hydroxyphenyl)-*N*-(1-methyl-1*H*-indol-5-yl)-5,6,7,8-tetrahydro-1-indolizine carboxamide.

11. Compound of formula (I) according to claim 8 which is *N*-(4-hydroxyphenyl)-*N*-(1-methyl-1*H*-indazol-5-yl)-3-{2,2-didcuterio-6-[((3*S*)-3-(4-morpholinylmethyl)-3,4-dihydro-2(1*H*)-isoquinolinyl)carbonyl]-1,3-benzodioxol-5-yl}-5,6,7,8-tetrahydro-1-indolizine carboxamide.

12. Compound of formula (I) according to claim 8 which is *N-*(4-hydroxyphenyl)-*N*-(1-methyl-1*H*-indazol-5-yl)-3-(6-{[(3*S*)-3-(morpholin-4-ylmethyl)-3,4-dihydroisoquinolin-2(1*H*)-yl]carbonyl}-1,3-benzodioxol-5-yl)-5,6,7,8-tetrahydroindolizine-1-carboxamide.

13. Compound of formula (I) according to claim 8 which is *N*-(4-hydroxyphenyl)-3-{7-[((3*S*)-3-(4-morpholinylmethyl)-3,4-dihydro-2(1*H*)-isoquinolinyl)carbonyl]-2,3-dihydro-1,4-benzodioxin-6-yl}-*N*-phenyl-5,6,7,8-tetrahydro-1-indolizine carboxamide.

14. Compound of formula (I) according to claim 8 which is *N*-(4-hydroxyphenyl)-*N*(1-methyl-1*H-*indol-5-yl)-3-{6-[((3*S*)-3-[(4-methyl-1-piperazinyl)methyl]-3,4-dihydro-2(1*H-*isoquinolinyl)carbonyl]-1,3-benzodioxol-5-yl}-1-indolizine carboxamide.

15. Compound of formula (I) according to claim 8 which is *N*-[4-(hydroxy)phenyl]-*N-*(1-methyl-1*H-*indol-5-yl)-3-{6-[((3*S*)-3-(4-morpholinylmethyl)-3,4-dihydro-2(1*H*)-isoquinolinyl)carbonyl]-1,3-benzodioxol-5-yl}-5,6,7,8-tetrahydro-1-indolizine carboxamide.

16. Compound of formula (I) according to claim 8 which is *N*-(4-hydroxyphenyl)-3-{6-[((3*S*)-3-(4-morpholinylmethyl)-3,4-dihydro-2(1*H*)-isoquinolinyl)carbonyl]-1,3-benzodioxol-5-yl}-*N*-phenyl-1-indolizine carboxamide.

17. Compound of formula (I) according to claim 8 which is 3-{5-chloro-2-[((3*S*)-3-(4-morpholinylmethyl)-3,4-dihydro-2(1*H*)-isoquinolinyl)carbonyl]phenyl}-*N*-(4-hydroxyphenyl)-*N*-(1-methyl-1*H*-indol-5-yl)-1-indolizine carboxamide.

18. Compound of formula (I) according to claim 8 which is 6-{5-chloro-2-[((3*S*)-3-(4-morpholinylmethyl)-3,4-dihydro-2(1*H*)-isoquinolinyl)carbonyl]phenyl}-*N*-(3-fluoro-4-methylphenyl)-*N*-(4-hydroxyphenyl)-3,4-dihydro-1*H*-pyrrolo[2,1-c][1,4]oxazine-8-carboxamide.

19. Compound of formula (I) according to claim 8 which is 3-(5-chloro-2-{[(3*S*)-3-(morpholin-4-ylmethyl)-3,4-dihydroisoquinolin-2(1*H*)-yl]carbonyl}phenyl)-*N*-(4-hydroxyphenyl)-*N-*(1-methyl-1*H-*pyrazol-4-yl)-5,6,7,8-tetrahydroindolizine-1-carboxamide.

20. Hydrochloride salt of a compound of formula (I) according to any one of claims 9 to 13 and 15 to 19.

21. Dihydrochloride salt of the compound of formula (I) according to claim 14.

22. Process for the preparation of a compound of formula (I) according to claim 1, **characterised in that** there is used as starting material the compound of formula (II): 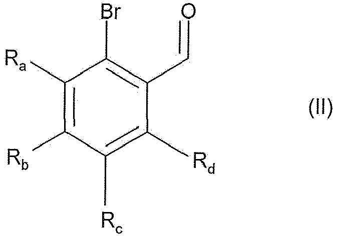 wherein Rₐ, R_{b}, R_{c} and R_{d} are as defined for formula (I),
which compound of formula (II) is subjected to a Heck reaction, in an aqueous or organic medium, in the presence of a palladium catalyst, of a base, of a phosphine and of the compound of formula (III): 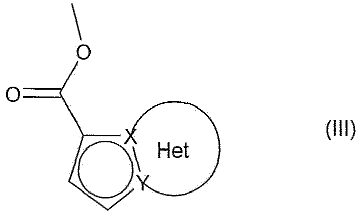 wherein the groups X, Y and Het are as defined for formula (I),
to obtain the compound of formula (IV): 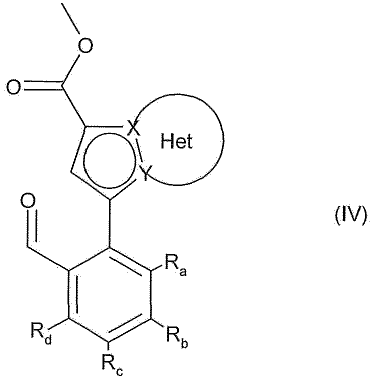 wherein Rₐ, R_{b}, R_{c}, R_{d}, X, Y and Het are as defined for formula (I),
the aldehyde function of which compound of formula (IV) is oxidised to the carboxylic acid to form the compound of formula (V): 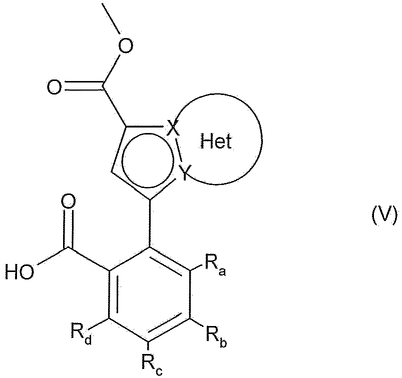 wherein Rₐ, R_{b}, R_{c}, R_{d}, X, Y and Het are as defined for formula (I),
which compound of formula (V) is then subjected to peptide coupling with a compound of formula (VI): 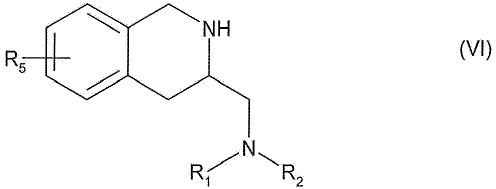 wherein R₁, R₂ and R₅ are as defined for formula (I),
to yield the compound of formula (VII): 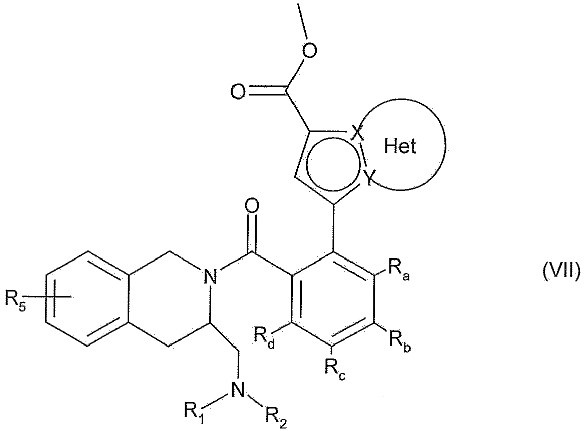 wherein Rₐ, R_{b}, R_{c}, R_{d}, R₁, R₂, R₅, X, Y and Het are as defined for formula (I),
the ester function of which compound of formula (VII) is hydrolysed to yield the carboxylic acid or the corresponding carboxylate, which may be converted into an acid derivative such as acyl chloride or the corresponding anhydride before being coupled with an amine NHR₃R₄ wherein R₃ and R₄ have the same meanings as for formula (I), to yield the compound of formula (I),
which compound of formula (I) may be purified according to a conventional separation technique, which is converted, if desired, into its addition salts with a pharmaceutically acceptable acid or base and which is optionally separated into its isomers according to a conventional separation technique,
it being understood that, at any time considered appropriate in the course of the above-described process, certain groups (hydroxy, amino...) of the reagents or intermediates of synthesis may be protected and then deprotected according to the requirements of synthesis.

23. Process according to claim 22 for the preparation of a compound of formula (I) wherein one of the groups R₃ and R₄ is substituted by a hydroxy function, **characterised in that** the amine NHR₃R₄ is subjected beforehand to a protection reaction of the hydroxy function prior to coupling with the carboxylic acid formed from the compound of formula (VII), or with a corresponding acid derivative thereof, the resulting protected compound of formula (I) subsequently undergoes a deprotection reaction and is then optionally converted into one of its addition salts with a pharmaceutically acceptable acid or base.

24. Pharmaceutical composition comprising a compound of formula (I) according to any one of claims 1 to 19 or an addition salt thereof with a pharmaceutically acceptable acid or base in combination with one or more pharmaceutically acceptable excipients.

25. Pharmaceutical composition according to claim 24 for use as a pro-apoptotic agent.

26. Pharmaceutical composition according to claim 24 for use in the treatment of cancers and of immune and auto-immune diseases.

27. Pharmaceutical composition according to claim 24 for use in the treatment of cancers of the bladder, brain, breast and uterus, chronic lymphoid leukaemias, colorectal cancer, oesophagus and liver, lymphoblastic leukaemias, follicular lymphomas, melanomas, malignant haemopathies, myelomas, ovarian cancer, non-small-cell lung cancer, prostate cancer and small-cell lung cancer.

28. Compound of formula (I) according to any one of claims 1 to 19, or an addition salt thereof with a pharmaceutically acceptable acid or base, for use in the treatment of cancers of the bladder, brain, breast and uterus, chronic lymphoid leukaemias, colorectal cancer, oesophagus and liver, lymphoblastic leukaemias, follicular lymphomas, melanomas, malignant haemopathies, myelomas, ovarian cancer, non-small-cell lung cancer, prostate cancer and small-cell lung cancer.

29. Association of a compound of formula (I) according to any one of claims 1 to 21 with an anti-cancer agent selected from genotoxic agents, mitotic poisons, anti-metabolites, proteasome inhibitors, kinase inhibitors and antibodies.

30. Pharmaceutical composition comprising an association according to claim 29 in combination with one or more pharmaceutically acceptable excipients.

31. Association according to claim 29 for use in the treatment of cancers.

32. Compound of formula (I) according to any one of claims 1 to 21 for use in association with radiotherapy in the treatment of cancers.
